# EUROPEAN PATENT SPECIFICATION

(11) **EP 3 053 918 B1**
(45) Date of publication and mention of the grant of the patent: **11.04.2018**
(21) Application number: 15154169.5
(22) Date of filing: 06.02.2015
(51) Int. Cl.: C07D 405/14, C07D 403/14, C07D 409/14, C07D 487/04, C09K 11/06, H01L 51/00

(54) **2-carbazole substituted benzimidazoles for electronic applications**
2-Carbazol substituierte Benzimidazole für Elektronische Anwendungen
Benzimidazoles substitués par un 2-carbazole pour des applications électroniques

(43) Date of publication of application: 10.08.2016
(73) Proprietor: Idemitsu Kosan Co., Ltd., Chiyoda-ku Tokyo 100-8321 (JP)
(72) Inventor: GROARKE, Michelle, 4125 Riehen (CH); SCHÄFER, Thomas, 4410 Liestal (CH); NAKANO, Yuki, 4057 Basel (CH); SHIOMI, Takushi, 4055 Basel (CH); NAGASHIMA, Hideaki, 4057 Basel (CH)
(74) Representative: Hollah, Dorothee

(56) References cited:
- EP-A1- 2 700 695
- WO-A1-2014/051232
- KR-A- 20140 083 897
- KR-A- 20140 099 082
- US-A1- 2014 225 088

## Description

The present invention relates to compounds of formula **I,** a process for their production and their use in electronic devices, especially electroluminescent devices. When used as charge transport material and/or host material for phosphorescent emitters in electroluminescent devices, the compounds of formula **I** may provide improved efficiency, stability, manufacturability and/or spectral characteristics of electroluminescent devices.

WO2011162162 a light-emitting device material which comprises a compound having a specific structure of a carbazole skeleton and can realize a light-emitting device having high light-emitting efficiency and durability. The following compounds are explicitly mentioned:

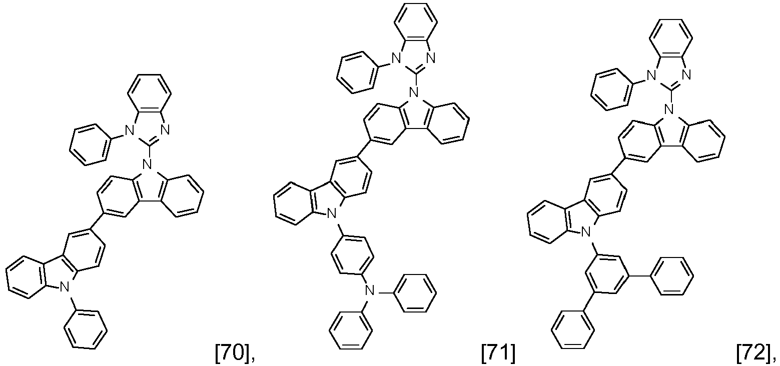

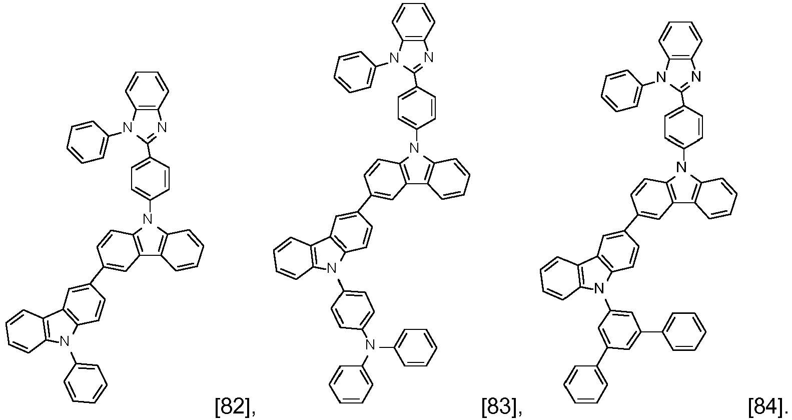

DE102012022880 relates to an electronic device comprises at least one organic layer comprising one or more substituted heteroaryl compounds 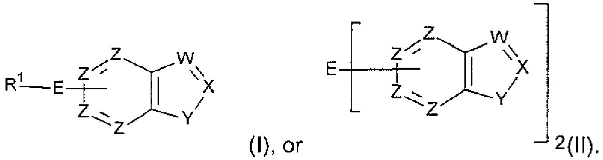 In a preferred embodiment DE102012022880 is directed to compounds of formula 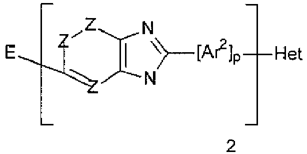 (II-2-b). E is a divalent group S=O, or S(=O)₂.

US2013001537 relates to compounds of formula 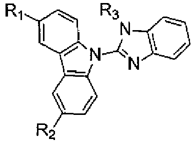 and their use as a host material for phosphorescence emission. R₁ and R₂ each independently represent an unsubstituted phenyl group or a substituted phenyl group substituted with an alkyl group having 1 to 6 carbon atoms; and R₃ represents an alkyl group having 1 to 6 carbon atoms.

US2014145149 relates to compounds of formula 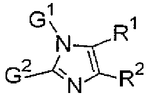 containing an imidazole core and electron donor and acceptor fragments.

G¹ is an electron donor group or an electron acceptor group.

G² is an electron donor group or an electron acceptor group.

G¹ is an electron donor group, then G² is an electron acceptor group.

G¹ is an electron acceptor group, then G² is an electron donor group.

The electron acceptor group is at least one chemical group selected from the group consisting of a six-membered aromatic ring system having at least two nitrogen atoms and a 5-membered aromatic ring system containing at least one nitrogen atom, one oxygen atom, one sulfur atom, or one selenium atom.

Preferred acceptor groups are 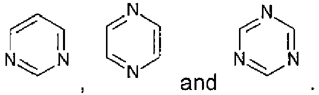 The following compound is explicitly mentioned:

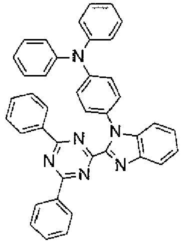

EP 2 700 695 relates to a compound for an organic optoelectronic device represented by the Chemical Formula 1

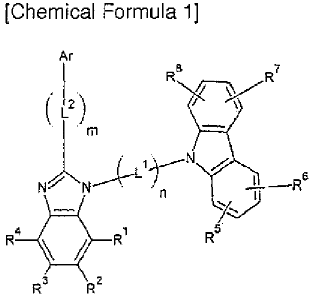

Notwithstanding these developments, there remains a need for organic light emitting devices comprising new charge transport materials to provide improved efficiency, stability, manufacturability, and/or spectral characteristics of electroluminescent devices.

Accordingly, it is an object of the present invention, with respect to the aforementioned prior art, to provide further materials suitable for use in OLEDs and further applications in organic electronics. More particularly, it should be possible to provide charge transport materials, charge/exciton blocker materials and matrix materials for use in OLEDs. The materials should be suitable especially for OLEDs which comprise at least one phosphorescence emitter, especially at least one green emitter or at least one blue emitter. Furthermore, the materials should be suitable for providing OLEDs which ensure good efficiencies, good operative lifetimes and a high stability to thermal stress, and a low use and operating voltage of the OLEDs.

Certain 2-carbazole substituted benzimidazoles are found to be suitable for use in organo-electroluminescent devices. In particular, said derivatives are suitable charge transport materials, or host materials for phosphorescent emitters with good efficiency and durability.

Said object has been solved by compounds of the formula 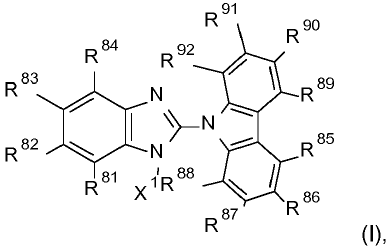 wherein
R81, R⁸², R⁸³ and R⁸⁴ are independently of each other H, a C₁-C₂₅alkyl group, which can optionally be substituted by E and or interrupted by D; a C₆-C₂₄aryl group, which can optionally be substituted by G, a C₂-C₃₀heteroaryl group, which can optionally be substituted by G;
R⁸⁵, R⁸⁶, R⁸⁷, R⁸⁸, R⁸⁹, R⁹⁰, R⁹¹ and R⁹² are independently of each other H, CN, a C₁-C₂₅alkyl group, which can optionally be substituted by E and or interrupted by D; a C₆-C₂₄aryl group, which can optionally be substituted by G, a C₂-C₃₀heteroaryl group, which can optionally be substituted by G;
with the proviso that R⁸⁵, R⁸⁶, R⁸⁷, R⁸⁸, R⁸⁹, R⁹⁰, R⁹¹ and R⁹² are different from a carbazolyl group,
X¹ is a group of formula -A¹-X², which is selected from 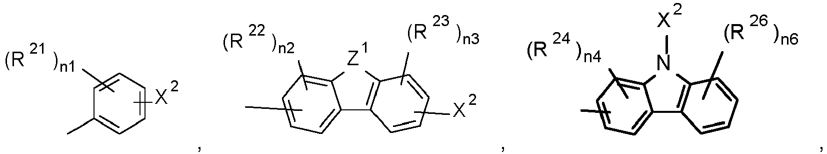 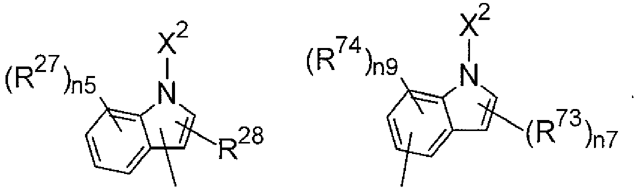 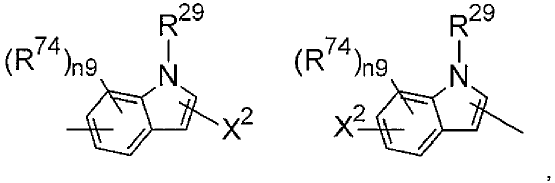 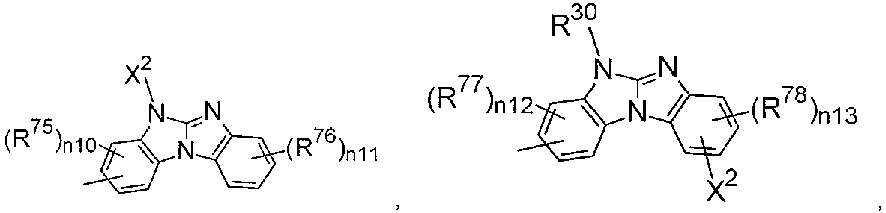 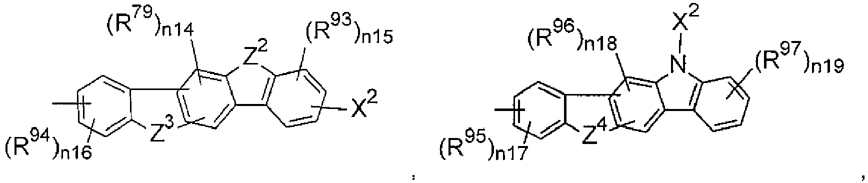 or 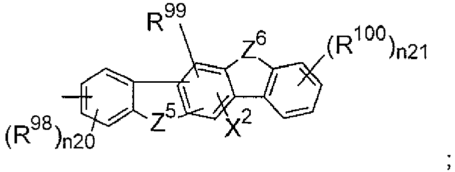 X² is a group of formula -(A²)ₚ-(A³)_{q}-(A⁴)ᵣ-R¹⁶,
p is 0, or 1, q is 0, or 1, r is 0, or 1;
R¹⁶ is a group of formula 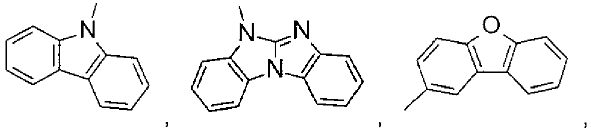 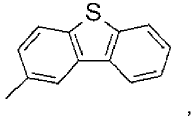 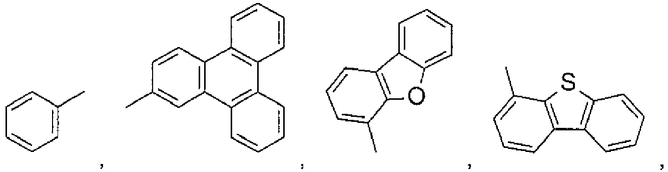 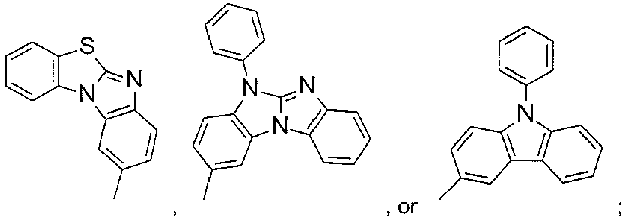
R¹⁰ and R¹¹ are independently of each other a C₆-C₂₄aryl group, which can optionally be substituted by G; or a C₂-C₃₀heteroaryl group, which can optionally be substituted by G; R¹², R¹³ and R¹⁴ are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G; n7, n14 and n18 are independently of each other 0, 1, or 2;
n2, n3, n4, n9, n10, n12, n13, n15, n16, n17 and n20 are independently of each other 0, 1, 2, or 3,
n1, n5, n6, n11, n19 and n21 are independently of each other 0, 1, 2, 3, or 4;
R²¹, R²², R²³, R²⁴, R²⁶, R²⁷, R²⁸, R⁷³, R⁷⁴, R⁷⁵, R⁷⁶, R⁷⁷, R⁷⁸, R⁷⁹, R⁹³, R⁹⁴, R⁹⁵, R⁹⁶, R⁹⁷, R⁹⁸, R⁹⁹ and R¹⁰⁰ are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G;
Z¹, Z², Z³, Z⁴, Z⁵ and Z⁶ are independently of each other O, S, or NR²⁹;
R²⁹ and R³⁰ are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G;
A², A³ and A⁴ are independently of each other a C₆-C₂₄arylen group, which can optionally be substituted by G, or a C₂-C₃₀heteroarylen group, which can optionally be substituted by G;
D is -CO-, -COO-, -S-, -O-, -NR⁶⁵-, -SiR⁷⁰R⁷¹-, -POR⁷²-, -CR⁶³=CR⁶⁴-, or -C≡C-,
E is -OR⁶⁹, -SR⁶⁹, -NR⁶⁵R⁶⁶, -COR⁶⁸, -COOR⁶⁷, -CONR⁶⁵R⁶⁶, -CN, or F,
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ together form a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl, with the proviso that
X¹ is bonded to the nitrogen atom by a carbon atom.

The combination of the electron transporting benzimidazole group with the hole transporting carbazolyl group gives rise to materials that are highly suitable in devices that emit green, or blue light. Moreover, the improved amipolar characteristics give rise to more balanced charge transport in devices resulting in lower voltages and higher external quantum efficiencies (EQE's).

The compounds of the present invention may be used for electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors, dye lasers and electroluminescent devices, such as, for example, organic light-emitting diodes (OLEDs).

Accordingly, a further subject of the present invention is directed to an electronic device, comprising a compound according to the present invention. The electronic device is preferably an electroluminescent device.

The compounds of formula I can in principal be used in any layer of an EL device, but are preferably used as host, charge transport and/or charge/exciton blocking material. Particularly, the compounds of formula I are used as host material for green, especially blue light emitting phosphorescent emitters.

Hence, a further subject of the present invention is directed to a charge transport layer, comprising a compound of formula I according to the present invention.

A further subject of the present invention is directed to an emitting layer, comprising a compound of formula I according to the present invention. In said embodiment a compound of formula I is preferably used as host material in combination with a phosphorescent emitter.

A further subject of the present invention is directed to a charge/exciton blocking layer, comprising a compound of formula I according to the present invention.

D is preferably -CO-, -COO-, -S-, -O-, -NR⁶⁵-, wherein R⁶⁵ is C₁-C₁₈alkyl, such as methyl, ethyl, n-propyl, iso-propyl, n-butyl, isobutyl, or sec-butyl, or C₆-C₁₄aryl, such as phenyl, tolyl, naphthyl, or biphenylyl, or C₂-C₃₀heteroaryl, such as, for example, benzimid-azo[1,2-a]benzimidazo-2-yl ( 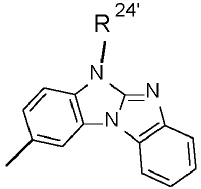 ), carbazolyl, dibenzofuranyl, which can be unsubstituted or substituted especially by C₆-C₁₀aryl, or C₆-C₁₀aryl, which is substituted by C₁-C₈alkyl; or C₂-C₁₃heteroaryl.

E is preferably -OR⁶⁹; -SR⁶⁹; -NR⁶⁵R⁶⁵; -COR⁶⁸; -COOR⁶⁷; -CONR⁶⁵R⁶⁵; or -CN; wherein R⁶⁵, R⁶⁷, R⁶⁸ and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl.

G is preferably-OR⁶⁹; -SR⁶⁹; -NR⁶⁵R⁶⁵; a C₁-C₁₈alkyl group, a C₆-C₁₄aryl group, a C₆-C₁₄aryl group, which is substituted by F, or C₁-C₁₈alkyl; a C₂-C₁₃heteroaryl group, or a C₂-C₁₃heteroaryl group, which is substituted by F, or C₁-C₁₈alkyl; or -Si(R^{12'})(R^{13'})(R^{14'}); wherein R⁶⁵, R⁶⁷, R⁶⁸ and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl; R^{12'}, R^{13'} and R^{14'} are independently of each other; a C₆-C₁₄aryl group, which can optionally be substituted by C₁-C₁₈alkyl ; or a C₂-C₁₃heteroaryl group, which can optionally be substituted by C₁-C₁₈alkyl.

A C₂-C₁₃heteroaryl group is for example, benzimidazo[1,2-a]benzimidazo-5-yl ( 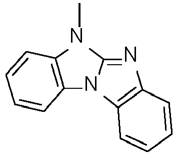 ), benzimidazo[1,2-a]benzimidazo-2-yl ( 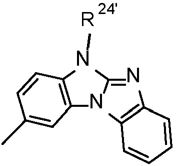 ), benzimidazolo[2,1-b][1,3]benzothiazolyl, carbazolyl, dibenzofuranyl, which can be unsubstituted or substituted, especially by C₆-C₁₀aryl, or C₆-C₁₀aryl, which is substituted by C₁-C₄alkyl; or C₂-C₁₃heteroaryl. R^{24'} is a C₆-C₂₄aryl group, or a C₂-C₃₀heteroaryl group, which can optionally be substituted by G, wherein G is defined above.

R⁸¹, R⁸², R⁸³ and R⁸⁴ are preferably H.

Among compounds of formula (I) compounds of formula 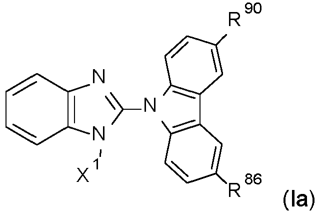 are preferred, wherein X¹, R⁸⁶ and R⁹⁰ are defined above, or below.

Preferably, R⁸⁶ and R⁹⁰ are independently of each other H, CN, or a C₆-C₂₄aryl group, which can optionally be substituted by G. A C₆-C₂₄aryl group is preferably phenyl, napthyl, anthracenyl, phenynthrenyl, triphenylene, or fluorenyl, which can optionally be substituted by G.

In addition, R⁸⁶ and R⁹⁰ might independently of each other represent a C₂-C₃₀heteroaryl group, which can optionally be substituted by G and which is different from a carbazolyl group. Preferred C₂-C₃₀heteroaryl groups, R⁸⁶ and R⁹⁰, are dibenzofuranyl, dibenzothiophenyl, benzimidazo[1,2-a]benzimidazo-5-yl ( 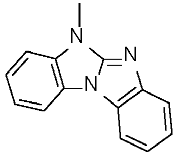 ), benzimidazo[1,2-a]benzimidazo-2-yl ( 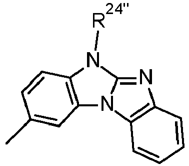 ), benzimidazolo[2,1-b][1,3]benzothiazolyl, which can be unsubstituted or substituted, especially by C₆-C₁₀aryl, or C₆-C₁₀aryl, which is substituted by C₁-C₄alkyl; or C₂-C₁₃heteroaryl. R^{24"} is a C₆-C₂₄aryl group, or a C₂-C₃₀heteroaryl group, which can optionally be substituted by G, wherein G is defined above.

Among compounds of formula (**Ia**) compounds of formula (**Ia**-**1**) are more preferred, wherein R⁹⁰ is H, 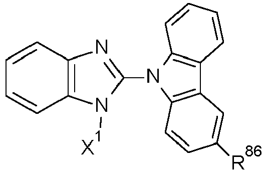 (**Ia-1**); and compounds of formula (**Ia-2**) are most preferred, wherein R⁸⁶ and R⁹⁰ are H,

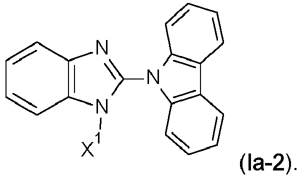

Compounds of formula (**Ia**), wherein at least one of R⁸⁵, R⁸⁶, R⁸⁷, R⁸⁸, R⁸⁹, R⁹⁰, R⁹¹ and R⁹², especially R⁸⁶ and/or R⁹⁰ is a C₁-C₂₅alkyl group; are suitable materials for solution processable OLEDs. Examples of such compounds are shown in the table below. 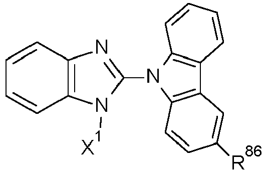 (X¹ is a group of formula --A¹-(A²)ₚ-(A³)_{q}-(A⁴)ᵣ-R¹⁶):

| **Compound** | **--A¹-(A²)ₚ-(A³)_{q}-(A⁴)ᵣ-** | **R¹⁶** | **R⁸⁶** |
|---|---|---|---|
| **A-1** | 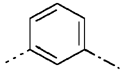 | 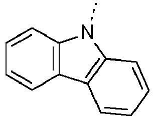 | n-C₈H₁₇ |
| **A-2** | 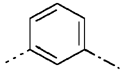 | 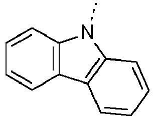 | |
| **A-3** | 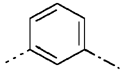 | 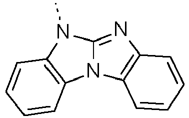 | n-C₈H₁₇ |
| **A-4** | 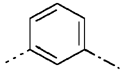 | 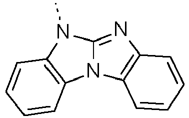 | |
| **A-5** | 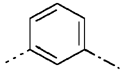 | 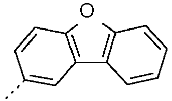 | n-C₈H₁₇ |
| **A-6** | 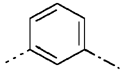 | 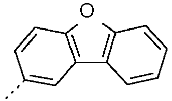 | |
| **A-7** | 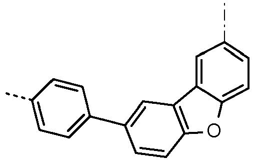 | 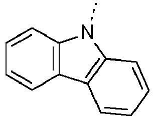 | n-C₈H₁₇ |
| **A-8** | 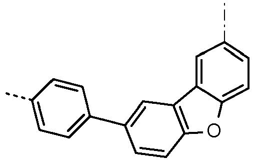 | 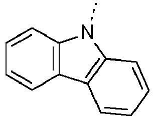 | |
| **A-9** | 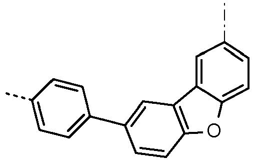 | 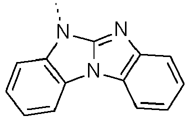 | n-C₈H₁₇ |
| **A-10** | 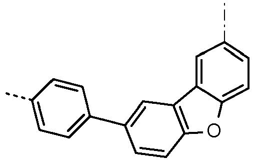 | 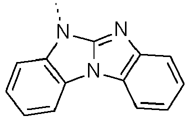 | |
| **A-11** | 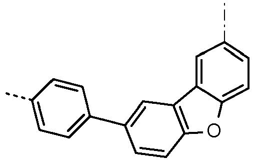 | 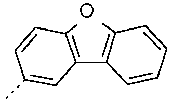 | n-C₈H₁₇ |
| **A-12** | 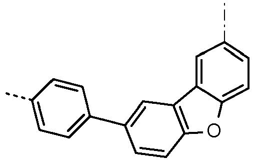 | 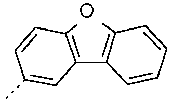 | |
| **A-13** | 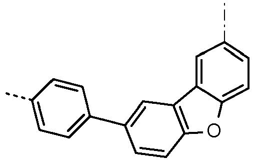 | 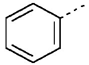 | n-C₈H₁₇ |
| **A-14** | 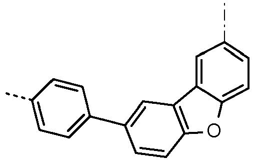 | 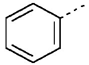 | |
| **A-15** | 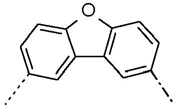 | 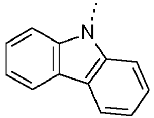 | n-C₈H₁₇ |
| **A-16** | 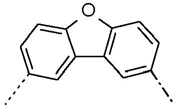 | 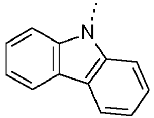 | |
| **A-17** | 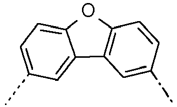 | 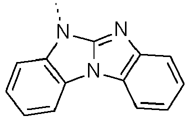 | n-C₈H₁₇ |
| **A-18** | 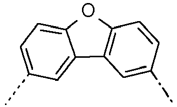 | 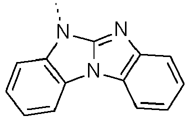 | |
| **A-19** | 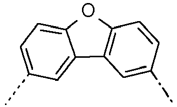 | 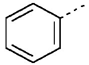 | n-C₈H₁₇ |
| **A-20** | 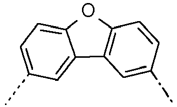 | 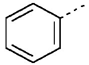 | |
| **A-21** | 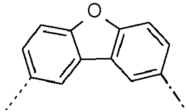 | 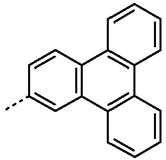 | n-C₈H₁₇ |
| **A-22** | 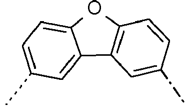 | 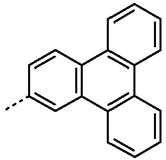 | |

| | | | |
|---|---|---|---|
| 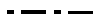 represents the bonding to R¹⁶. | | | |

X¹ is a group of formula -A¹-X²(--A¹ₒ-(A²)ₚ-(A³)_{q}-(A⁴)ᵣ-R¹⁶), which is selected from 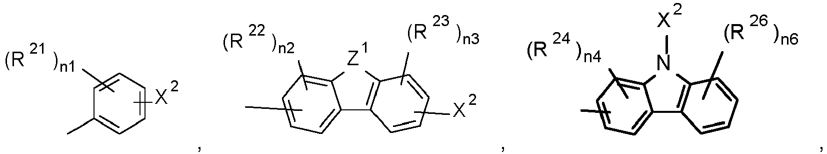 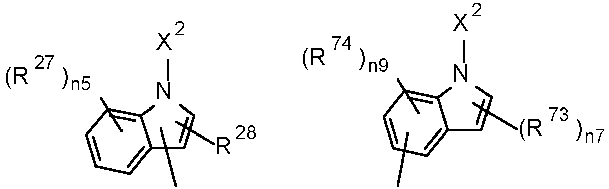 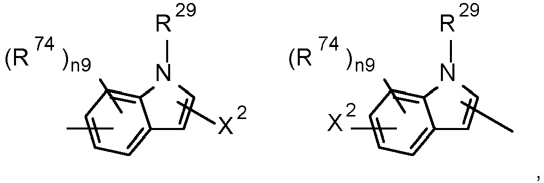 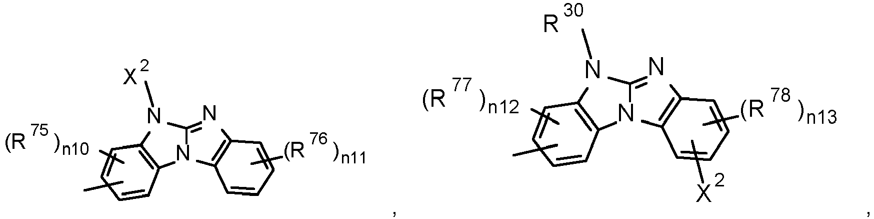 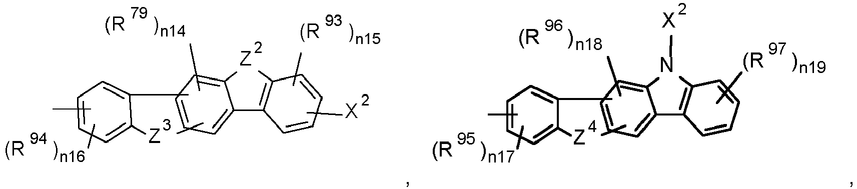 or

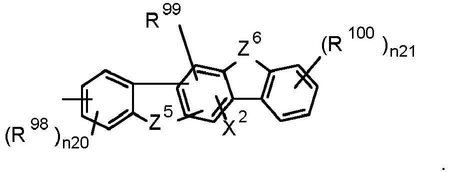

The group of formula -A¹-X² is preferably a group of formula 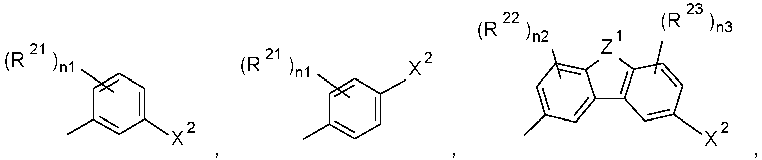 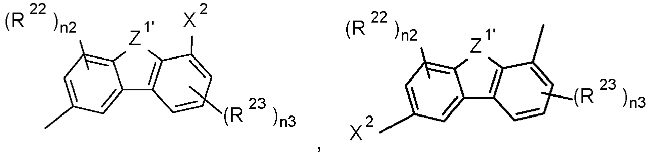 (Z^{1'} = O, or S), 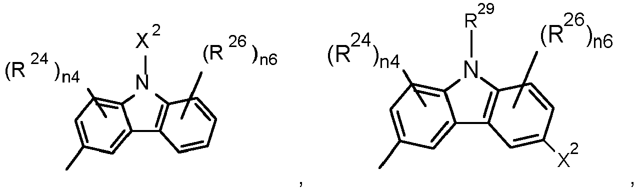 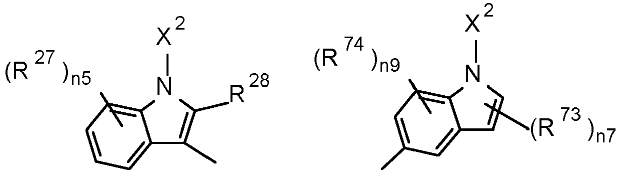 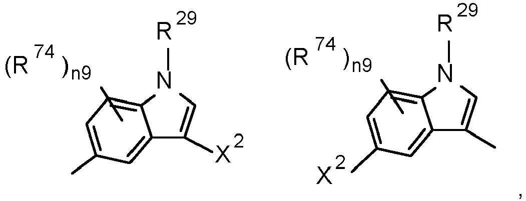 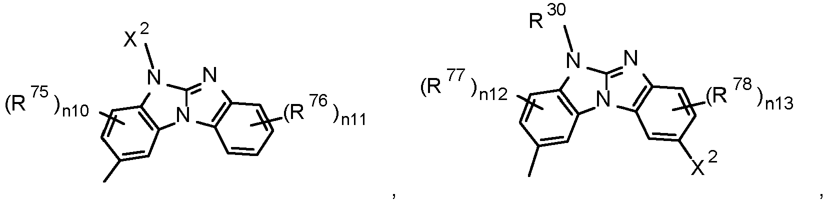 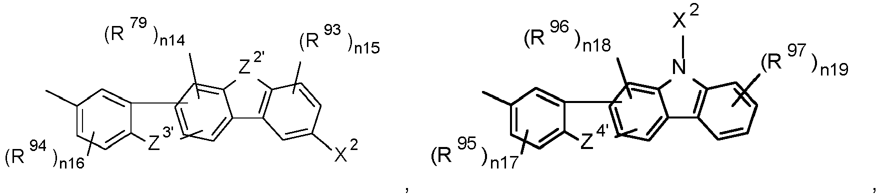 or 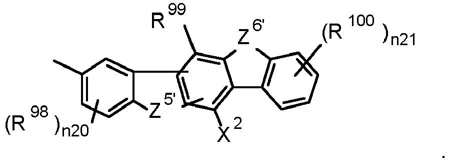 n7, n14 and n18 are independently of each other 0, 1, or 2, especially 0, or 1, very especially 0. n2, n3, n4, n9, n10, n12, n13, n15, n16, n17 and n20 are independently of each other 0, 1, 2, or 3, especially 0, or 1, very especially 0. n1, n5, n6, n11, n19 and n21 are independently of each other 0, 1, 2, 3, or 4, especially 0, or 1, very especially 0.

R²¹, R²², R²³, R²⁴, R²⁶, R²⁷, R²⁸, R⁷³, R⁷⁴, R⁷⁵, R⁷⁶, R⁷⁷, R⁷⁸, R⁷⁹, R⁹³, R⁹⁴, R⁹⁵, R⁹⁶, R⁹⁷, R⁹⁸, R⁹⁹ and R¹⁰⁰ are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G; especially a C₁-C₂₅alkyl group.

Z¹ is O, S, or NR²⁹;

Z^{1'}, Z^{2'}, Z^{3'}, Z^{4'}, Z^{5'} and Z^{6'} are independently of each other O, or S.

R²⁹ and R³⁰ are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G; especially C₆-C₁₀aryl, or C₆-C₁₀aryl, which is substituted by C₁-C₈alkyl; or C₂-C₁₃heteroaryl.

Most preferred, the group -A¹-X² is a group of formula 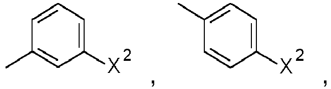 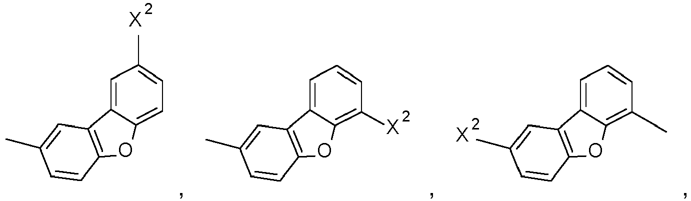 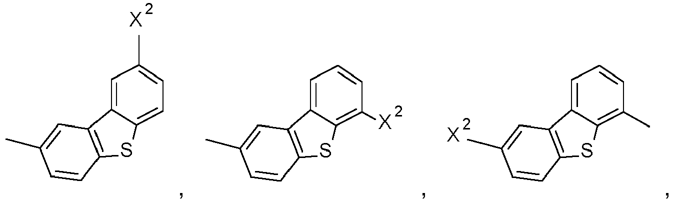 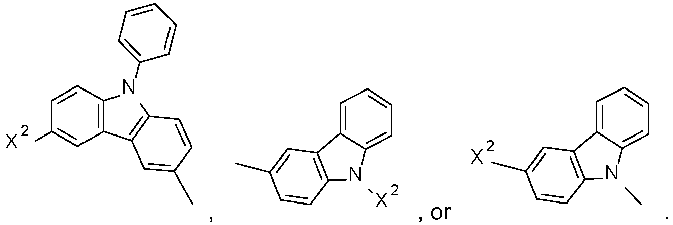 X² is a group of formula -(A²)ₚ-(A³)_{q}-(A⁴)ᵣ-R¹⁶, wherein p, q, r, R¹⁶ A², A³ and A⁴ are defined above, or below.

A², A³ and A⁴ are independently of each other a C₆-C₂₄arylen group, which can optionally be substituted by G, or a C₂-C₃₀heteroarylen group, which can optionally be substituted by G.

The C₆-C₂₄arylen groups A², A³ and A⁴ which optionally can be substituted by G, are typically phenylene, 4-methylphenylene, 4-methoxyphenylene, naphthylene, especially 1-naphthylene, or 2-naphthylene, biphenylylene, terphenylylene, pyrenylene, 2- or 9-fluorenylene, phenanthrylene, or anthrylene, which may be unsubstituted or substituted.

Preferred C₆-C₂₄arylen groups are 1,3-phenylene, 1,4-phenylene, 3,3'-biphenylylene, 3,3'-m-terphenylene, 2- or 9-fluorenylene, phenanthrylene, which may be unsubstituted or substituted, especially by C₆-C₁₀aryl, C₆-C₁₀aryl which is substituted by C₁-C₄alkyl; or C₂-C₁₄heteroaryl.

The C₂-C₃₀heteroarylen groups A², A³ and A⁴, which optionally can be substituted by G, represent a ring with five to seven ring atoms or a condensed ring system, wherein nitrogen, oxygen or sulfur are the possible hetero atoms, and is typically a heterocyclic group with five to 30 atoms having at least six conjugated-electrons such as, for example, benzofuro[2,3-b]pyridylene ( 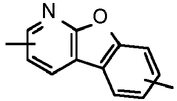 ), benzothiopheno[2,3-b]pyridylene ( 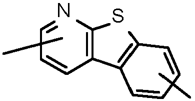 ), pyrido[2,3-b]indolylene ( 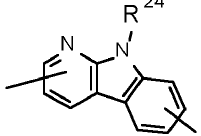 ), benzofuro[2,3-c]pyridylene ( 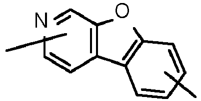 ), benzothiopheno[2,3-c]pyridylene ( 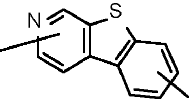 ), pyrido[2,3-c]indolylene ( 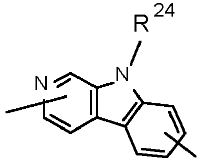 ), 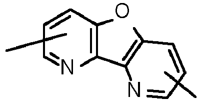 furo[3,2-b:4,5-b']dipyridylene, benzofuro[3,2-b]pyridylene ( 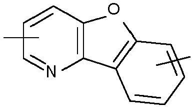 ), benzothiopheno[3,2-b]pyridylene ( 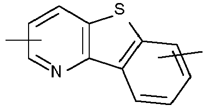 ), thieno[3,2-b:4,5-b']dipyridylene ( 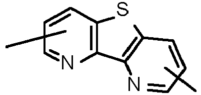 ), pyrrolo[3,2-b:4,5-b']dipyridylene ( 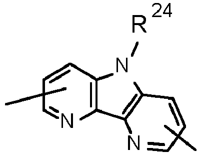 ), thienylene, benzothiophenylene, thianthrenylene, furylene, furfurylene, 2H-pyranylene, benzofuranylene, isobenzofuranylene, dibenzofuranylene ( 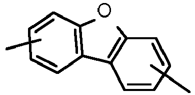 ), dibenzothiophenylene ( 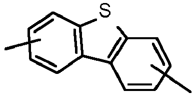 ), phenoxy-thienylene, pyrrolylene, imidazolylene, pyrazolylene, pyridylene, bipyridylene, triazinylene, pyrimidinylene, pyrazinylene, pyridazinylene, indolizinylene, isoindolylene, indolylene, indazolylene, purinylene, chinolizinylene, chinolylene, isochinolylene, phthalazinylene, naphthyridinylene, chinoxalinylene, chinazolinylene, cinnolinylene, pteridinylene, carbolinylene, benzotriazolylene, benzoxazolylene, phenanthridinylene, acridinylene, pyrimidinylene, phenanthrolinylene, phenazinylene, isothiazolylene, phenothiazinylene, isoxazolylene, furazanylene, carbazolylene ( 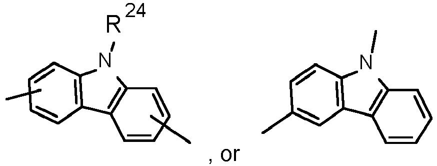 ), benzimidazo[1,2-a]benzimidazo-2,5-ylene ( 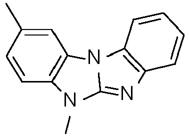 ), benzimidazo-1,2-ylene ( 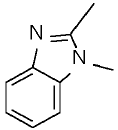 ), phenoxazinylene, indolo[2,3-b]carbazol-2,8-ylene ( 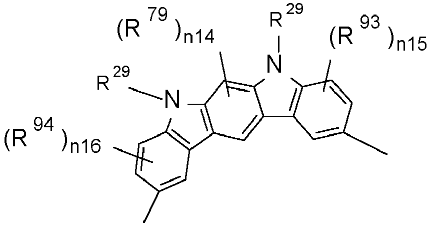 ), benzofuro[2,3-b]dibenzofuran-2,8-ylene ( 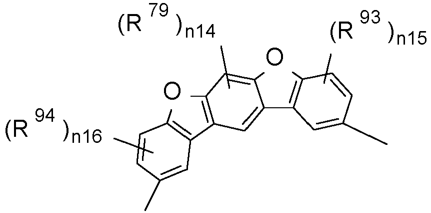 ), benzofuro[2,3-b]carbazol-2,7-ylene ( 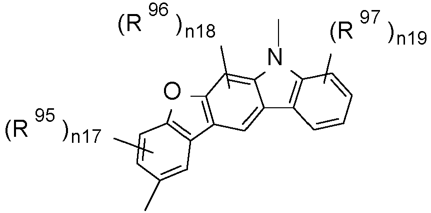 ), benzofuro[3,2-b]carbazol-2,11-ylene ( 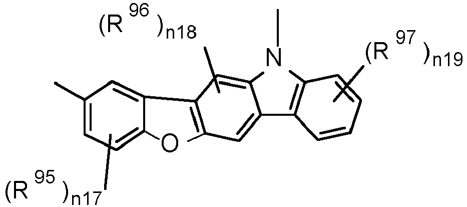 ), indolo[3,2-b]carbazol-2,11-ylene ( 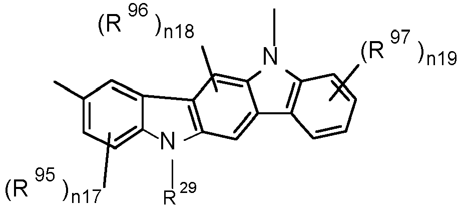 ), benzofuro[3,2-b]dibenzofuran-2,8-ylene( 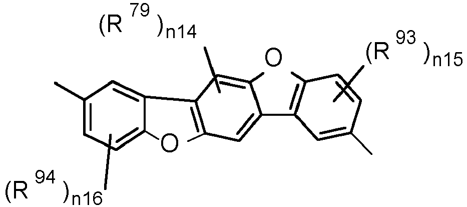 ), which can be unsubstituted or substituted. R²⁴ is a C₆-C₂₄aryl group, or a C₂-C₃₀heteroaryl group, which can optionally be substituted by G, wherein G is as defined in above.

Preferred C₂-C₃₀heteroarylen groups A², A³ and A⁴, are pyridylene, triazinylene, pyrimidinylene, such as, for example, 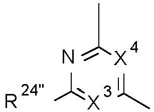 (X³ is N, or CH; X⁴ is N, or CH; R^{24"} is phenyl, or 9-carbazolyl); benzofuro[2,3-b]pyridylene, benzothiopheno[2,3-b]pyridylene , pyrido[2,3-b]indolylene , benzofuro[2,3-c]pyridylene, benzothiopheno[2,3-c]pyridylene , pyrido[2,3-c]indolylene, furo[3,2-b:4,5-b']dipyridylene, thieno[3,2-b:4,5-b']dipyridylene, pyrrolo[3,2-b:4,5-b']dipyridylene, dibenzofuranylene, dibenzothiophenylene , carbazolylene and benzimidazo[1,2-a]benzimidazo-2,5-ylene, benzofuro[3,2-b]pyridylene, benzothiopheno[3,2-b]pyridylene, or benzimidazo-1,2-ylene, which can be unsubstituted or substituted, especially by C₆-C₁₄aryl, C₆-C₁₄aryl which is substituted by C₁-C₄alkyl; or C₂-C₁₃heteroaryl.

The C₆-C₂₄arylen and C₂-C₃₀heteroarylen groups may be substituted by G.

G is preferably C₁-C₁₈alkyl, such as methyl, ethyl, n-propyl, iso-propyl, n-butyl, isobutyl, sec-butyl, hexyl, octyl, or 2-ethyl-hexyl, -CF₃, a C₆-C₁₄aryl group, a C₆-C₁₄aryl group, which is substituted by F, or C₁-C₁₈alkyl; a C₂-C₁₃heteroaryl group, or a C₂-C₁₃heteroaryl group, which is substituted by F, or C₁-C₁₈alkyl.

Benzimidazo[1,2-a]benzimidazo-5-yl, benzimidazo[1,2-a]benzimidazo-2-yl, carbazolyl and dibenzofuranyl are examples of a C₂-C₁₃heteroarylgroup. Phenyl, 1-naphthyl and 2-naphthyl are examples of a C₆-C₁₄aryl group.

More preferred, A², A³ and A⁴ are independently of each other a group of formula 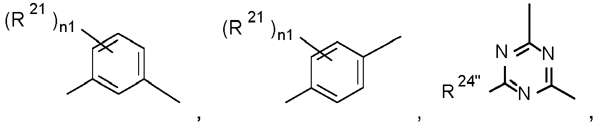 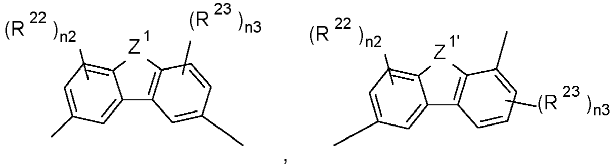 (Z^{1'} = O, or S), 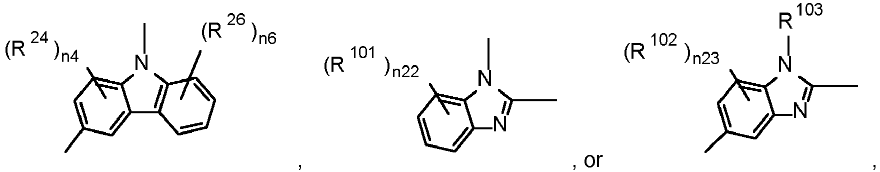 n2, n3, n4 and n23 are independently of each other 0, 1, 2, or 3, especially 0, or 1, very especially 0. n1, n6 and n22 are independently of each other 0, 1, 2, 3, or 4, especially 0, or 1, very especially 0.

R²¹, R²², R²³, R²⁴, R²⁶ R¹⁰¹ and R¹⁰² are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G; especially a C₁-C₂₅alkyl group.

Z¹ is O, S, or NR²⁹.

R²⁹ and R¹⁰³ are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G; especially C₆-C₁₀aryl, or C₆-C₁₀aryl, which is substituted by C₁-C₈alkyl; or C₂-C₁₃heteroaryl.

R^{24"} is

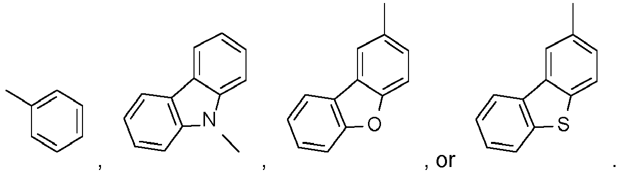

Most preferred, A², A³ and A⁴ are independently of each other a group of formula 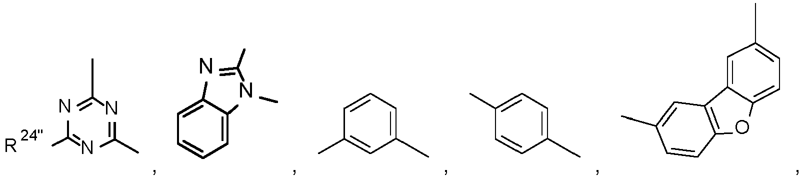 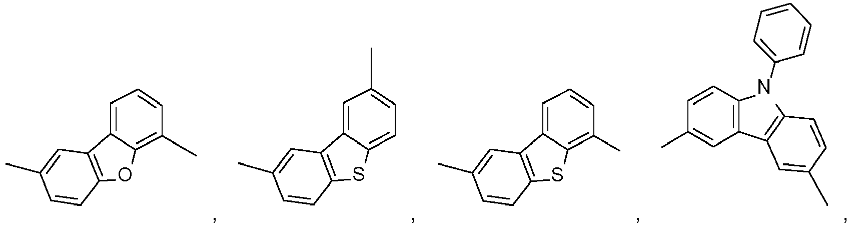 or 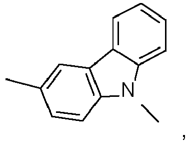 wherein R^{24"} is

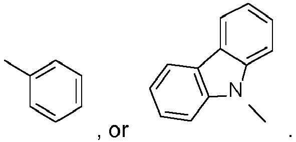

The group of formula --A¹-(A²)ₚ-(A³)_{q}-(A⁴)ᵣ- is preferably a group of formula 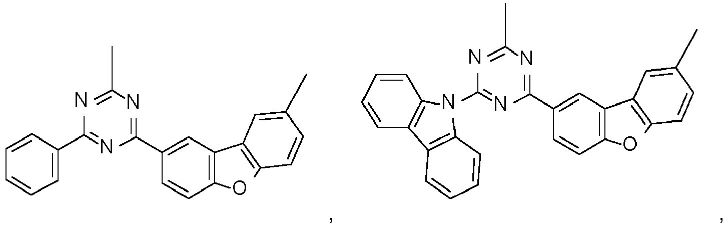 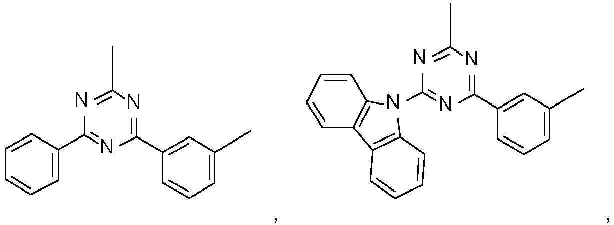 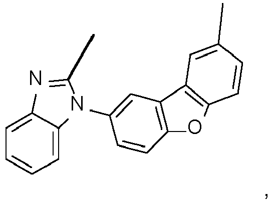 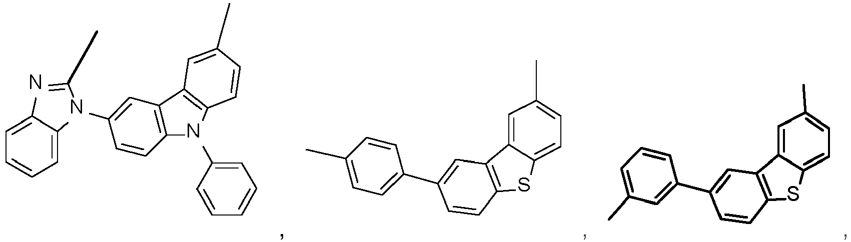 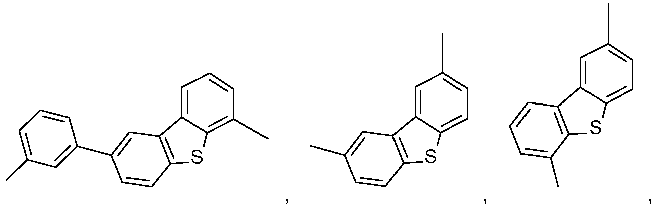 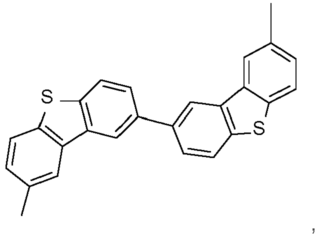 especially

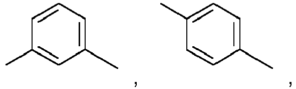

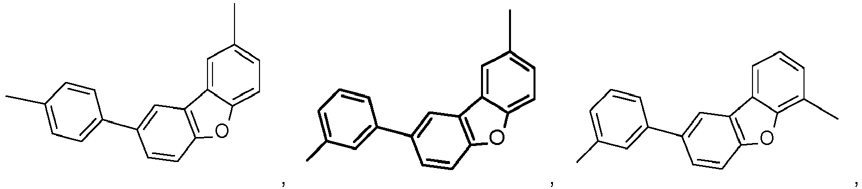

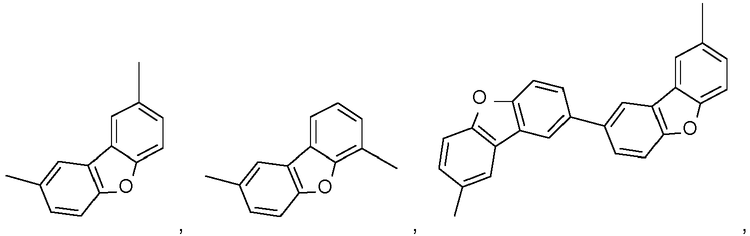

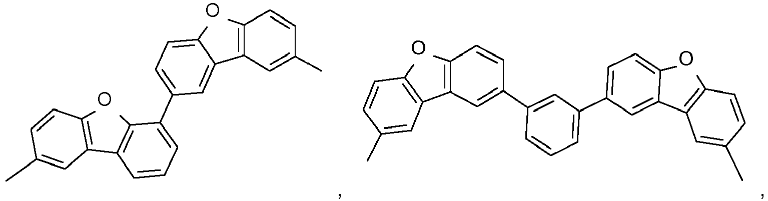

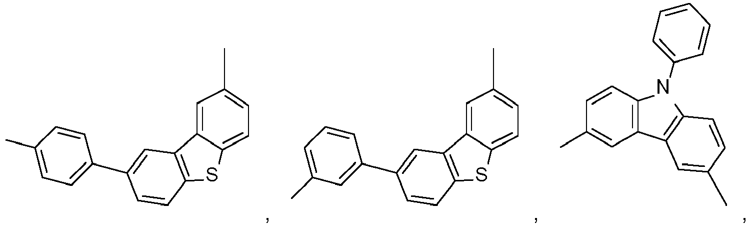

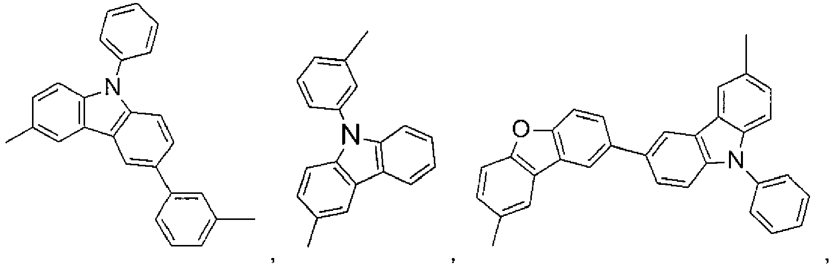

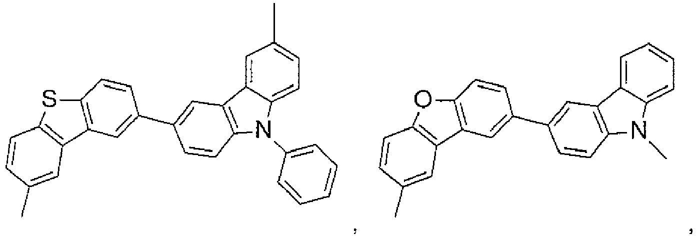

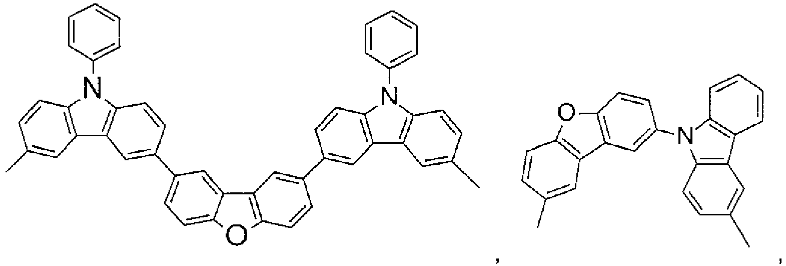

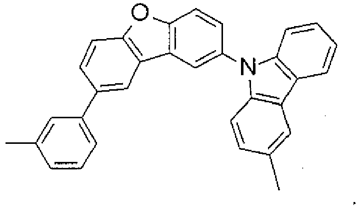

R¹⁶ is a group of formula

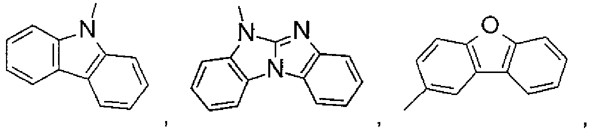

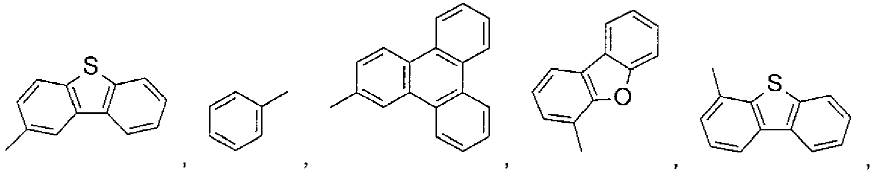

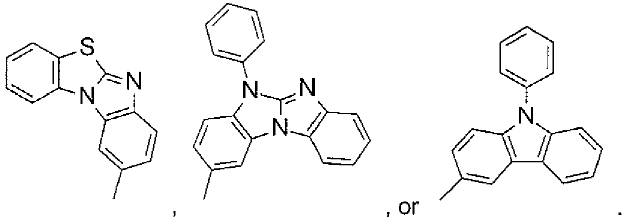

Compounds of the formula 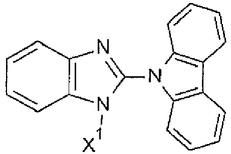 (**Ia-2**) are most preferred, wherein X¹ is a group of formula --A¹-(A²)ₚ-(A³)_{q}-(A⁴)ᵣ-R¹⁶, wherein --A¹-(A²)ₚ-(A³)_{q}-(A⁴)ᵣ- is a group of formula 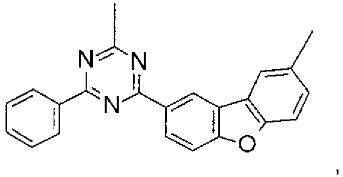 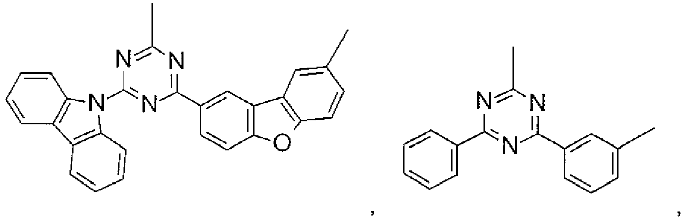 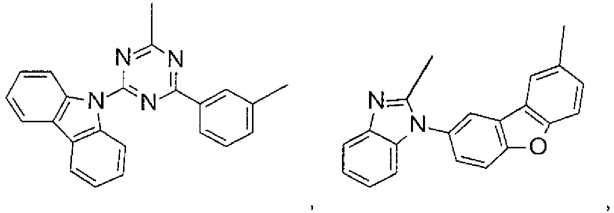 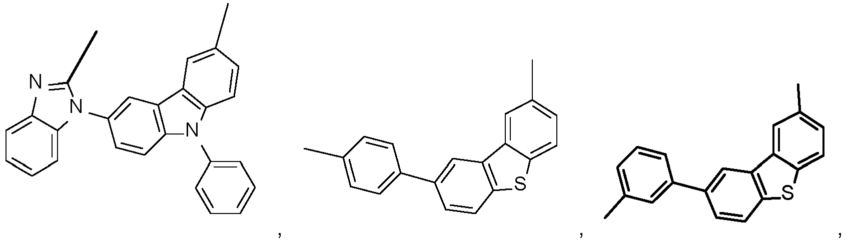 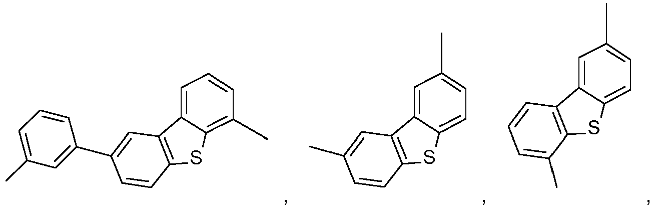 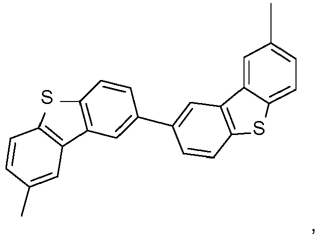 especially 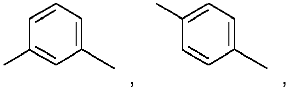 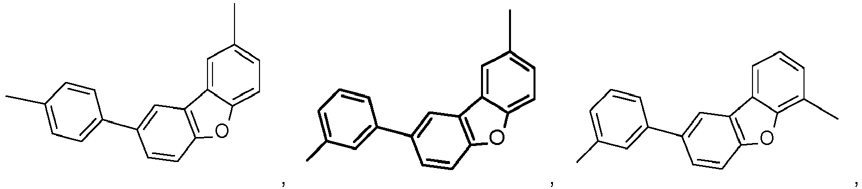 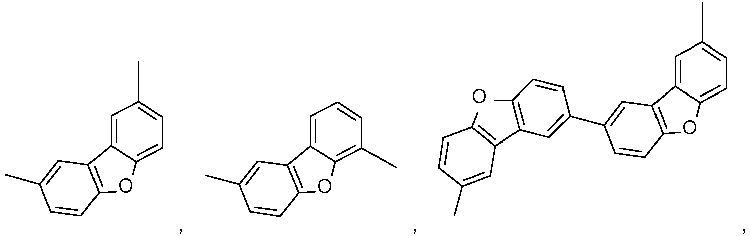 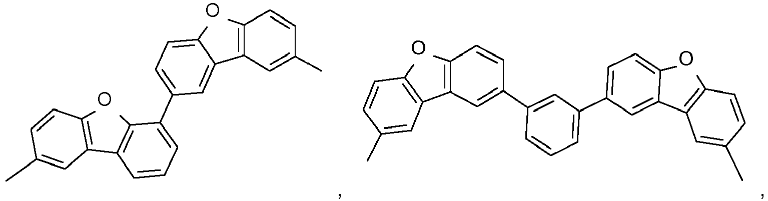 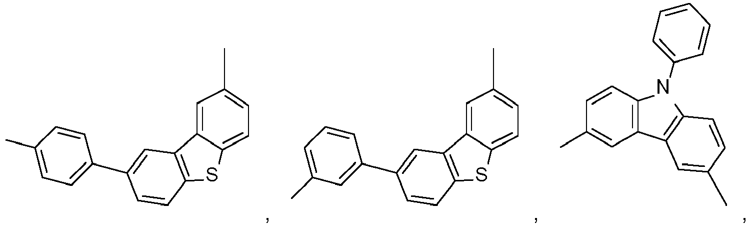 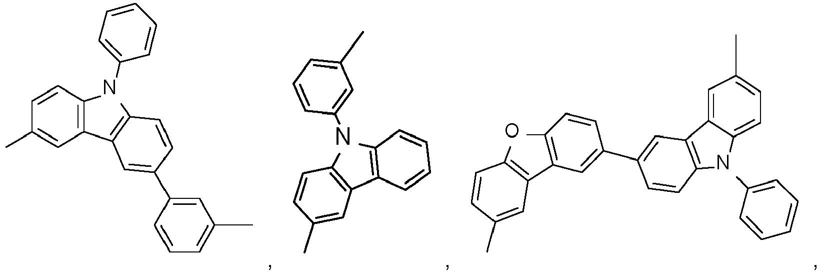 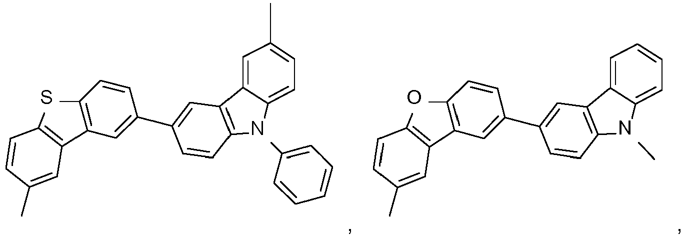 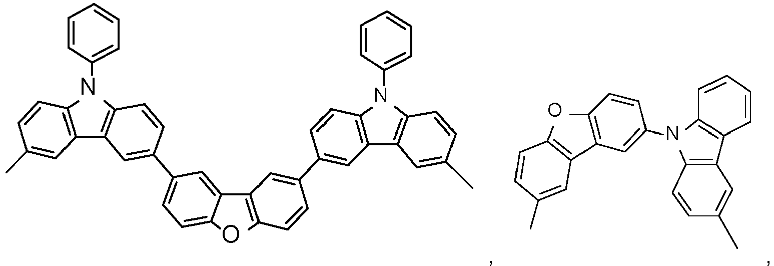 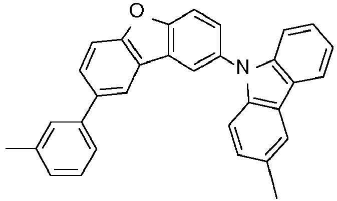 ; and R¹⁶ is a group of formula

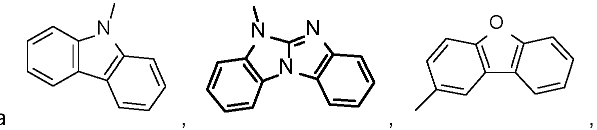

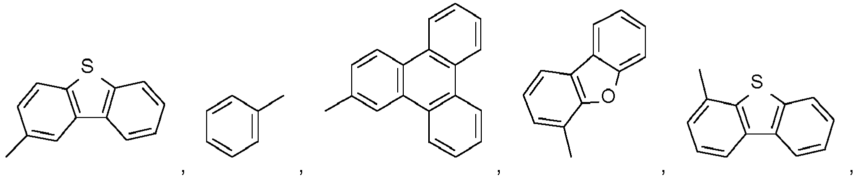

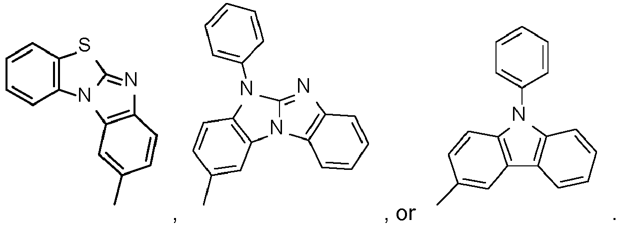

Most preferred, R¹⁶ is a group of formula

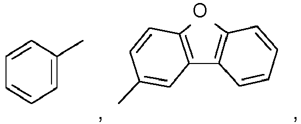

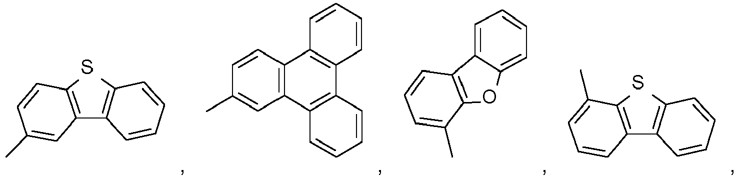

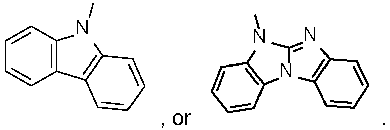

Examples of compounds of formula (I) are shown in the table below. 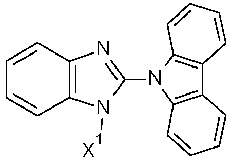 (X¹ is a group of formula --A¹-(A²)ₚ-(A³)_{q}-(A⁴)ᵣ-R¹⁶):

| Compound | --A¹-(A²)ₚ-(A³)_{q}-(A⁴)ᵣ- | R¹⁶ |
|---|---|---|
| C-1 | 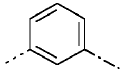 | 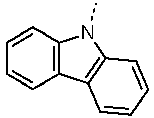 |
| C-2 | 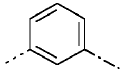 | 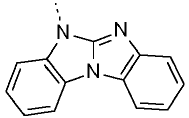 |
| C-3 | 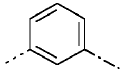 | 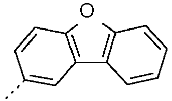 |
| C-4 | 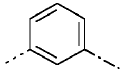 | 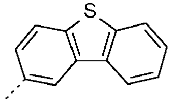 |
| C-5 | 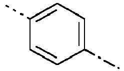 | 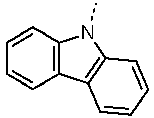 |
| C-6 | 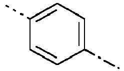 | 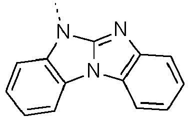 |
| C-7 | 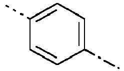 | 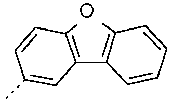 |
| C-8 | 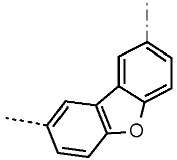 | 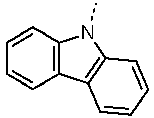 |
| C-9 | 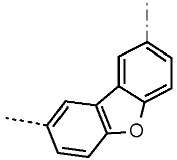 | 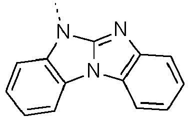 |
| C-10 | 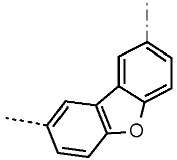 | 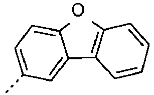 |
| C-11 | 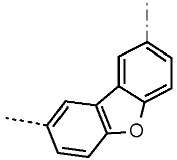 | 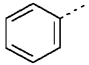 |
| C-12 | 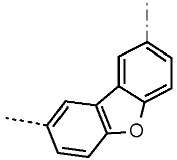 | 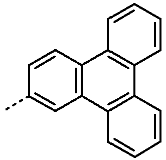 |
| C-13 | 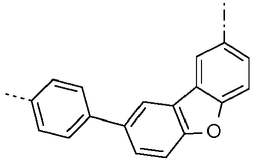 | 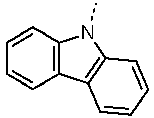 |
| C-14 | 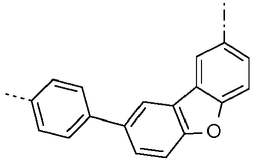 | 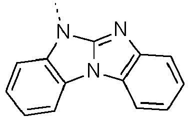 |
| C-15 | 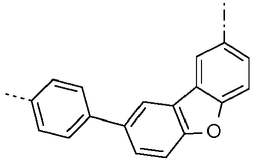 | 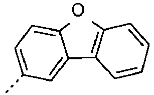 |
| C-16 | 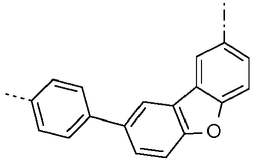 | 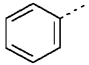 |
| C-17 | 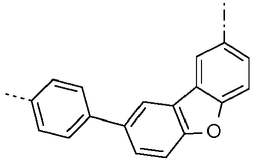 | 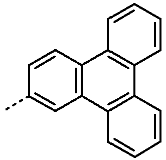 |
| C-18 | 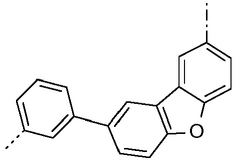 | 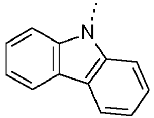 |
| C-19 | 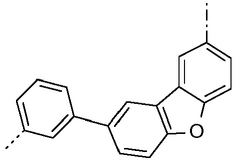 | 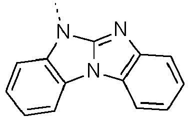 |
| C-20 | 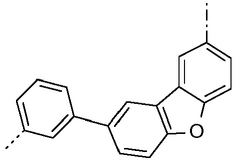 | 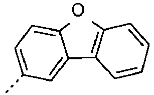 |
| C-21 | 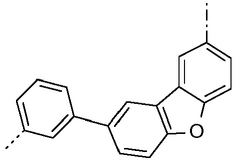 | 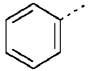 |
| C-22 | 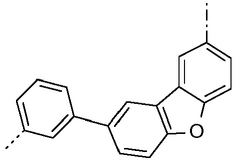 | 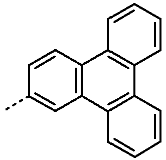 |
| C-23 | 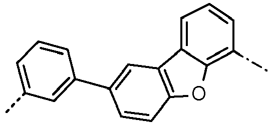 | 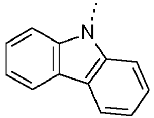 |
| C-24 | 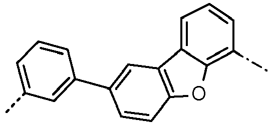 | 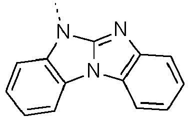 |
| C-25 | 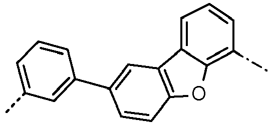 | 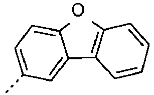 |
| C-26 | 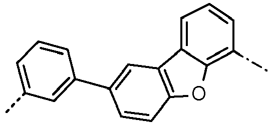 | 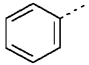 |
| C-27 | 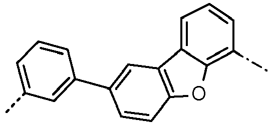 | 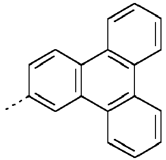 |
| C-28 | 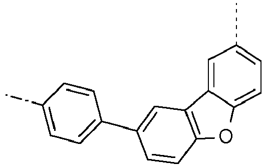 | 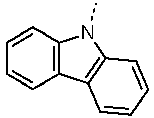 |
| C-29 | 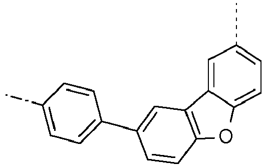 | 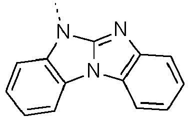 |
| C-30 | 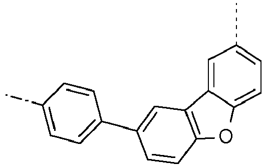 | 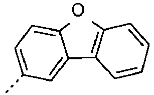 |
| C-31 | 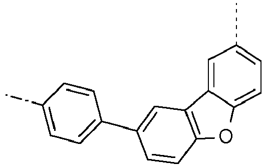 | 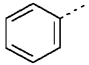 |
| C-32 | 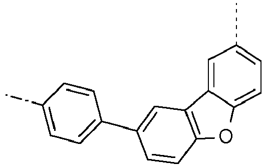 | 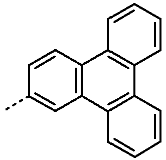 |
| C-33 | 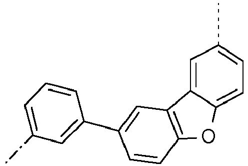 | 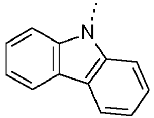 |
| C-34 | 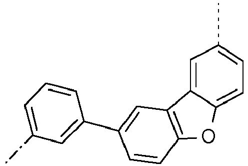 | 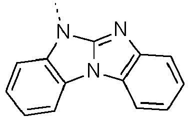 |
| C-35 | 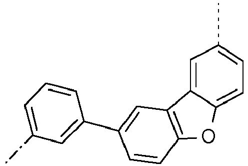 | 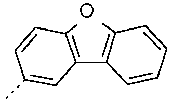 |
| C-36 | 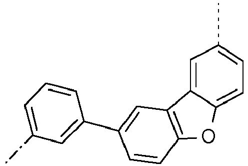 | 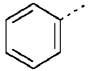 |
| C-37 | 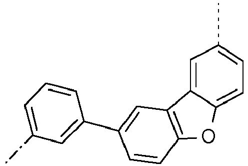 | 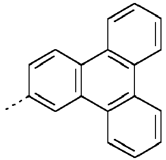 |
| C-38 | 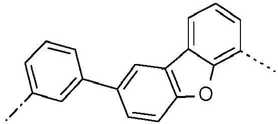 | 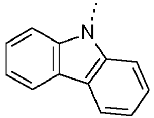 |
| C-39 | 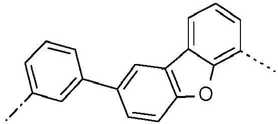 | 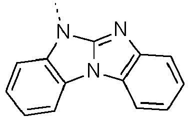 |
| C-40 | 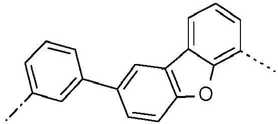 | 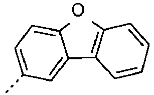 |
| C-41 | 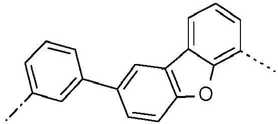 | 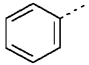 |
| C-42 | 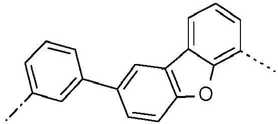 | 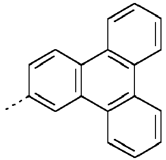 |
| C-43 | 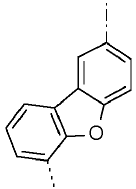 | 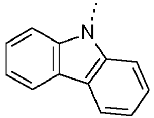 |
| C-44 | 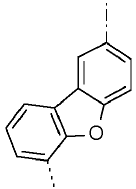 | 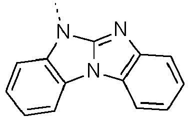 |
| C-45 | 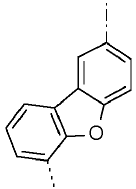 | 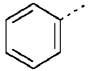 |
| C-46 | 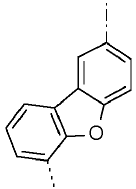 | 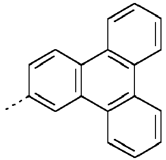 |
| C-47 | 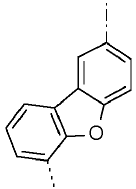 | 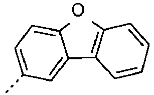 |
| C-48 | 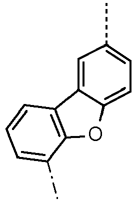 | 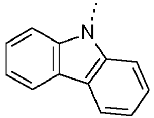 |
| C-49 | 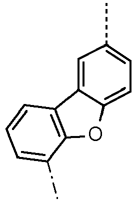 | 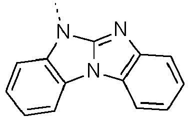 |
| C-50 | 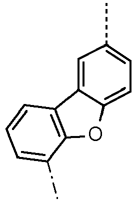 | 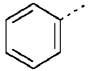 |
| C-51 | 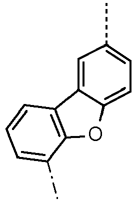 | 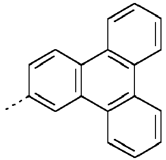 |
| C-52 | 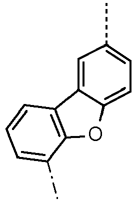 | 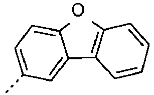 |
| C-53 | 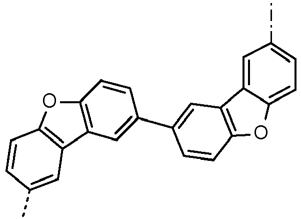 | 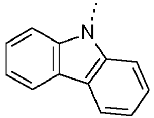 |
| C-54 | 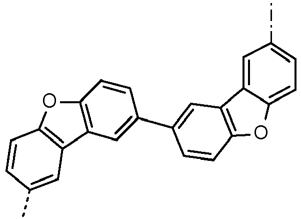 | 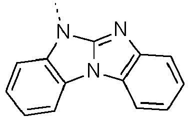 |
| C-55 | 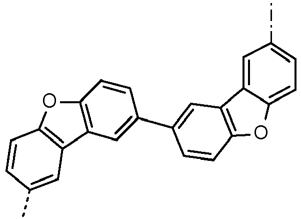 | 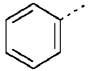 |
| C-56 | 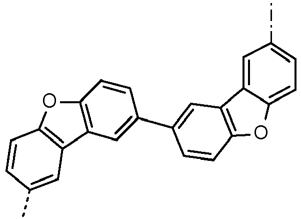 | 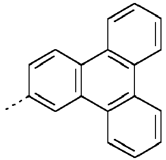 |
| C-57 | 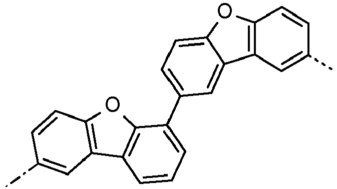 | 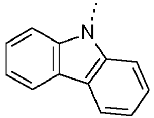 |
| C-58 | 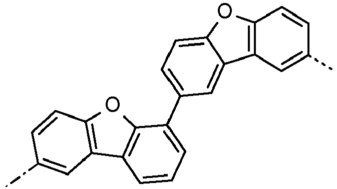 | 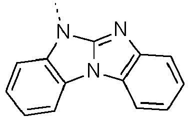 |
| C-59 | 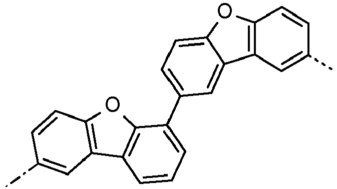 | 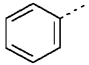 |
| C-60 | 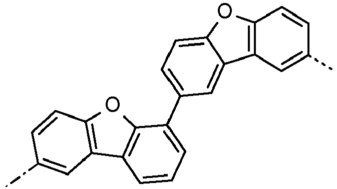 | 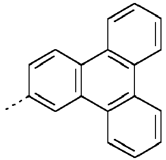 |
| C-61 | 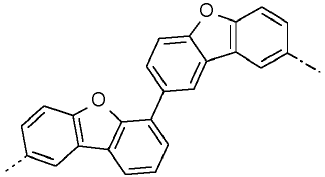 | 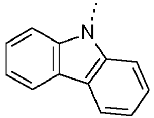 |
| C-62 | 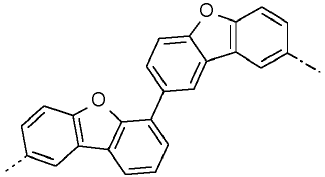 | 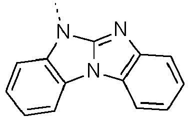 |
| C-63 | 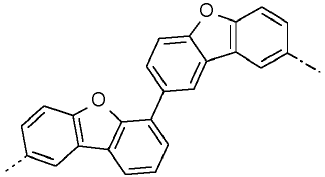 | 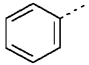 |
| C-64 | 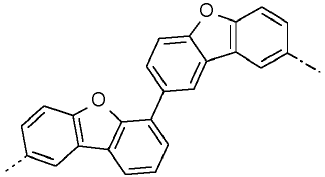 | 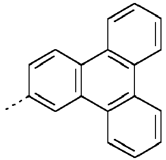 |
| C-65 | 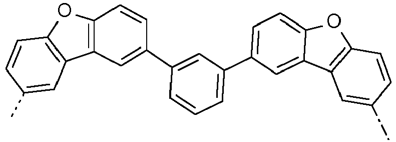 | 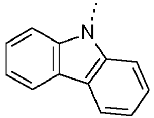 |
| C-67 | 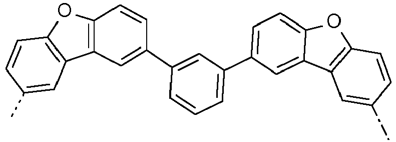 | 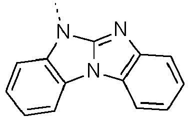 |
| C-67 | 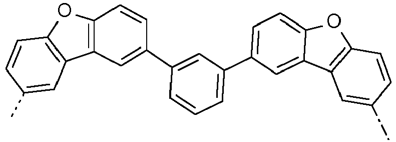 | 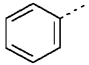 |
| C-68 | 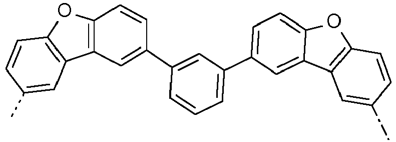 | 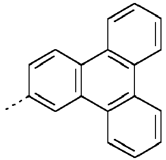 |
| C-69 | 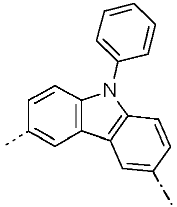 | 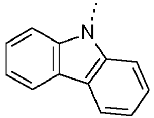 |
| C-70 | 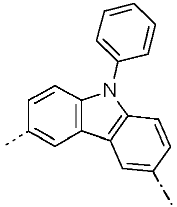 | 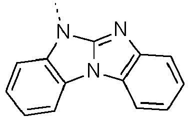 |
| C-71 | 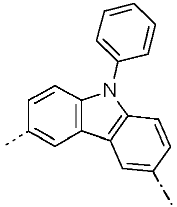 | 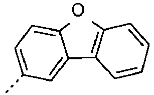 |
| C-72 | 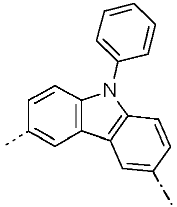 | 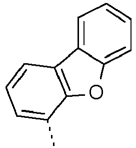 |
| C-73 | 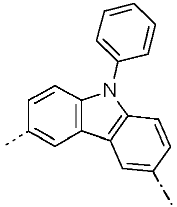 | 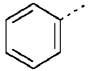 |
| C-74 | 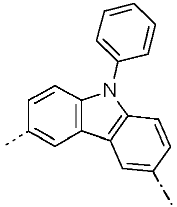 | 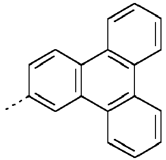 |
| C-75 | 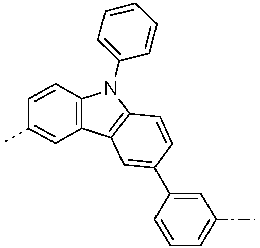 | 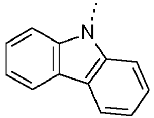 |
| C-76 | 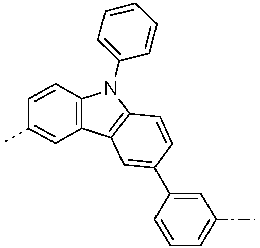 | 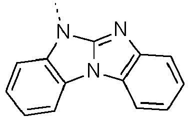 |
| C-77 | 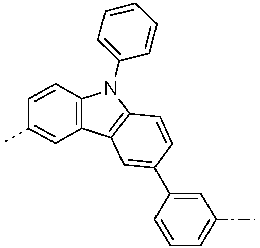 | 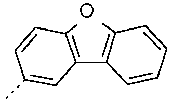 |
| C-78 | 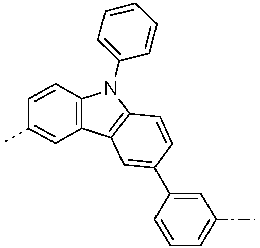 | 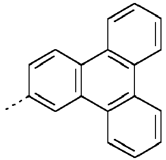 |
| C-79 | 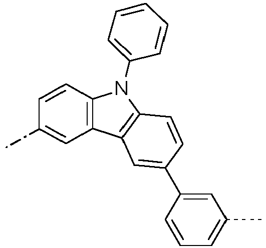 | 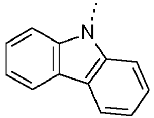 |
| C-80 | 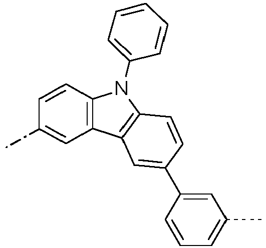 | 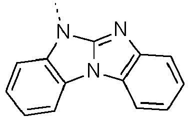 |
| C-81 | 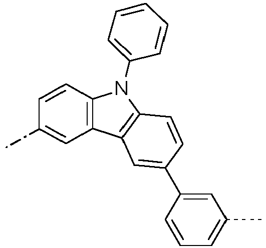 | 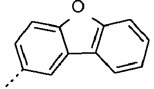 |
| C-82 | 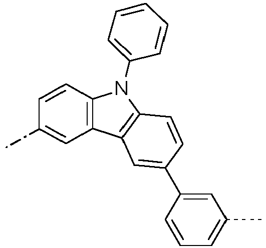 | 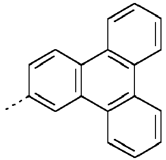 |
| C-83 | 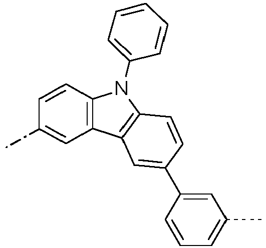 | 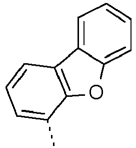 |
| C-84 | 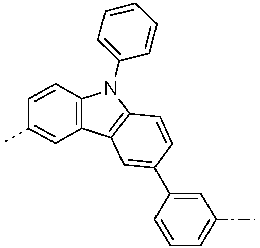 | 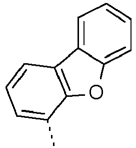 |
| C-85 | 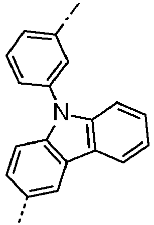 | 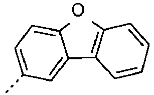 |
| C-86 | 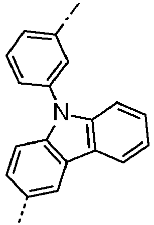 | 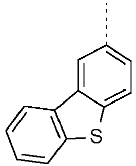 |
| C-87 | 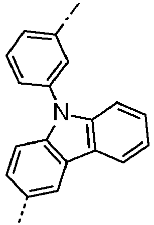 | 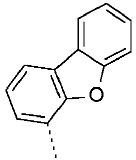 |
| C-88 | 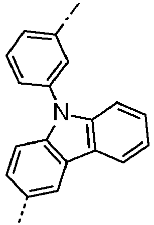 | 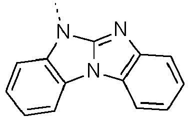 |
| C-89 | 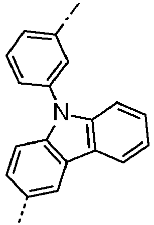 | 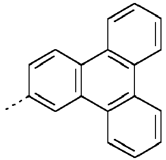 |
| C-90 | 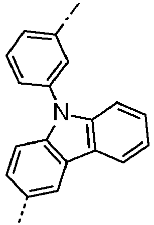 | 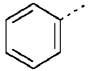 |
| C-91 | 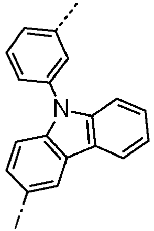 | 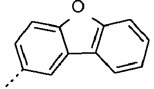 |
| C-92 | 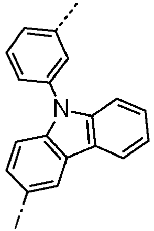 | 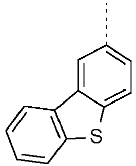 |
| C-93 | 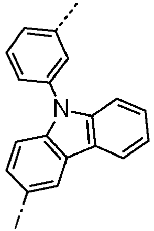 | 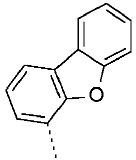 |
| C-94 | 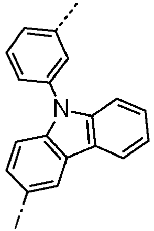 | 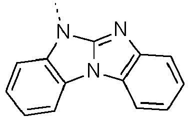 |
| C-95 | 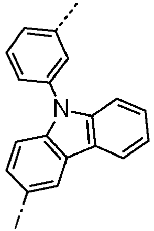 | 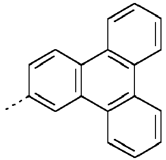 |
| C-96 | 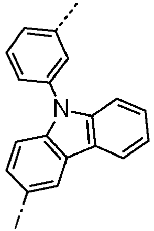 | 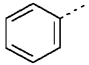 |
| C-97 | 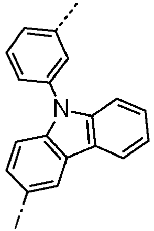 | 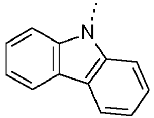 |
| C-98 | 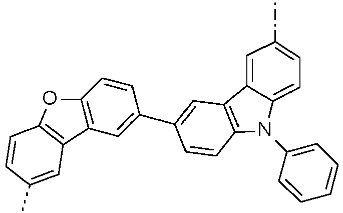 | 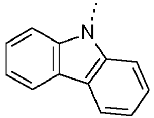 |
| C-99 | 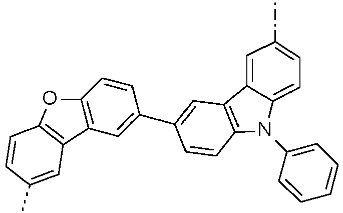 | 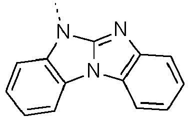 |
| C-100 | 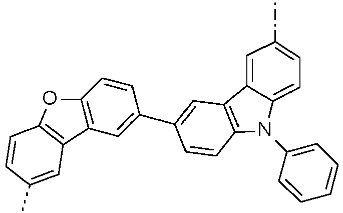 | 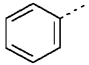 |
| C-101 | 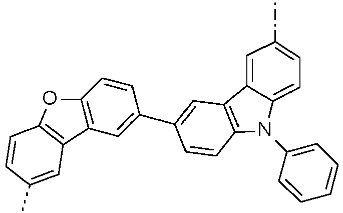 | 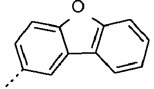 |
| C-102 | 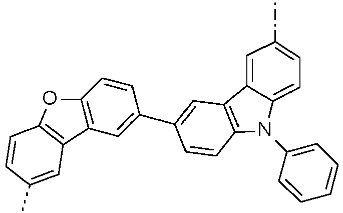 | 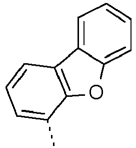 |
| C-103 | 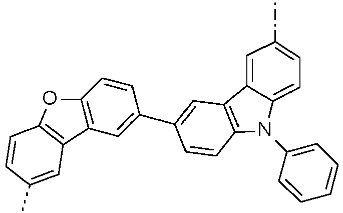 | 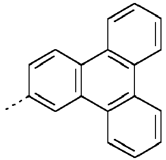 |
| C-104 | 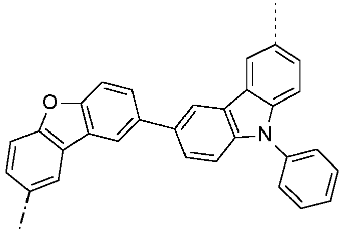 | 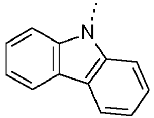 |
| C-105 | 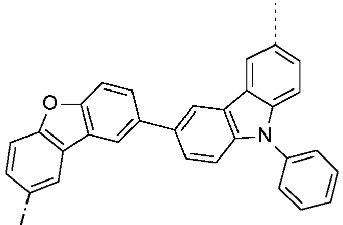 | 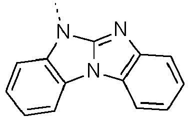 |
| C-106 | 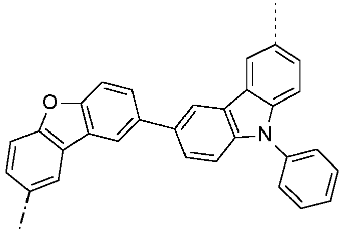 | 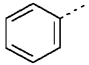 |
| C-107 | 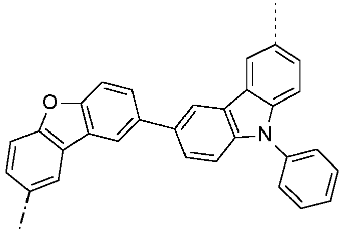 | 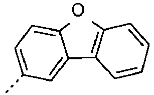 |
| C-108 | 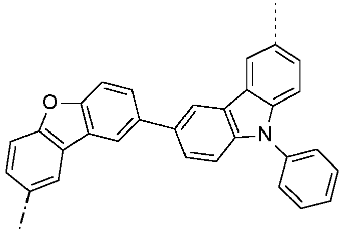 | 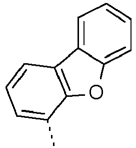 |
| C-109 | 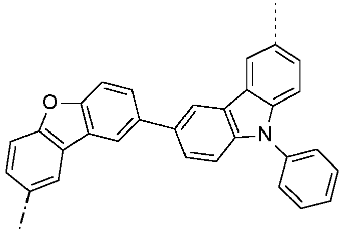 | 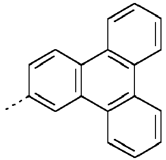 |
| C-110 | 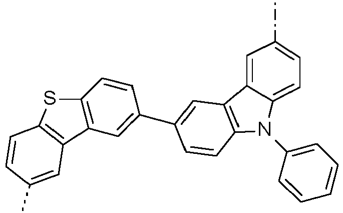 | 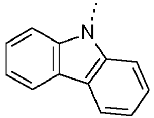 |
| C-111 | 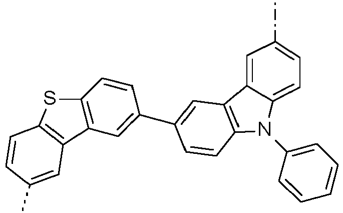 | 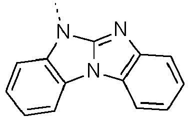 |
| C-112 | 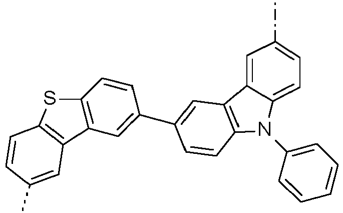 | 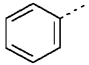 |
| C-113 | 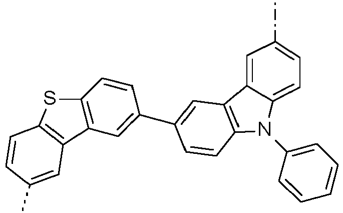 | 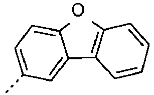 |
| C-114 | 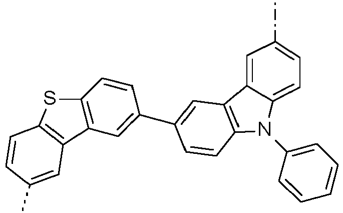 | 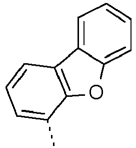 |
| C-115 | 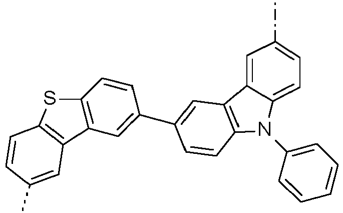 | 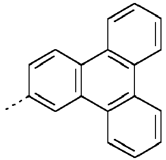 |
| C-116 | 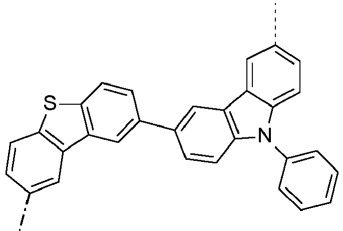 | 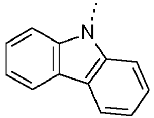 |
| C-117 | 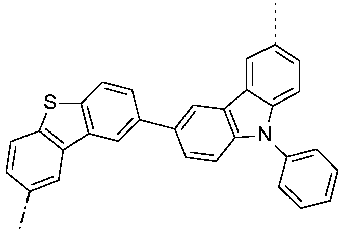 | 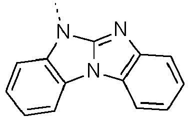 |
| C-118 | 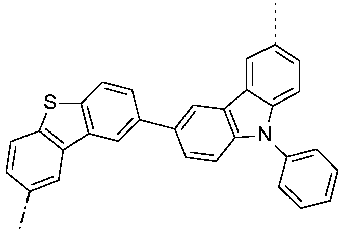 | 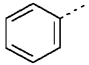 |
| C-119 | 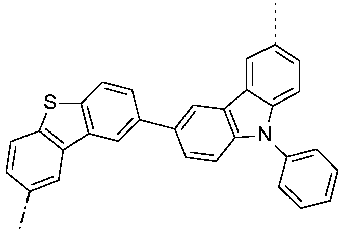 | 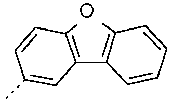 |
| C-120 | 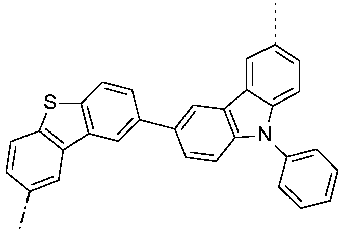 | 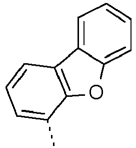 |
| C-121 | 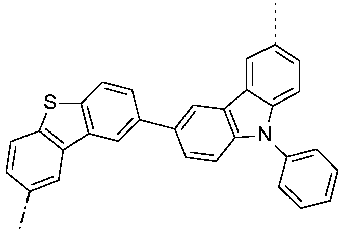 | 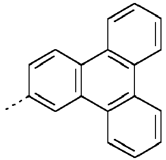 |
| C-122 | 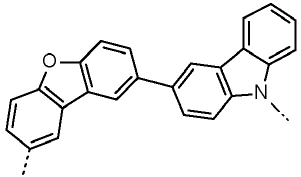 | 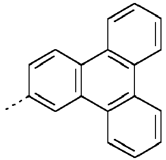 |
| C-123 | 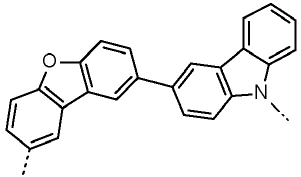 | 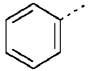 |
| C-124 | 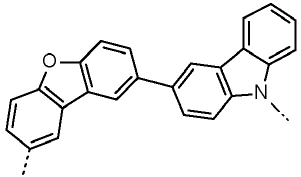 | 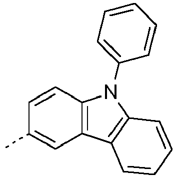 |
| C-125 | 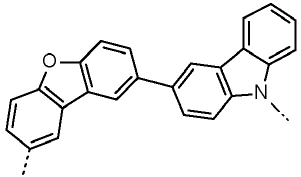 | 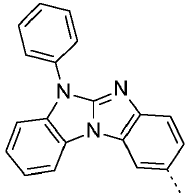 |
| C-126 | 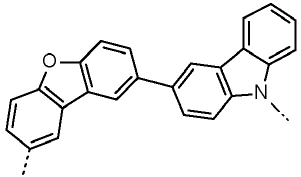 | 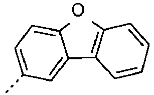 |
| C-127 | 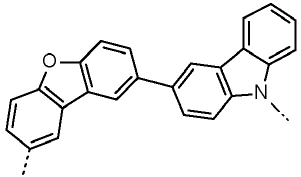 | 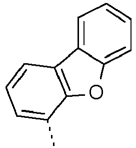 |
| C-128 | 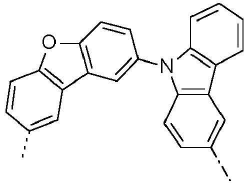 | 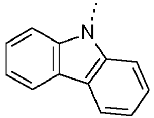 |
| C-129 | 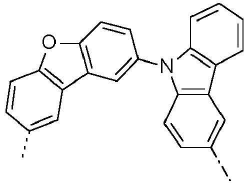 | 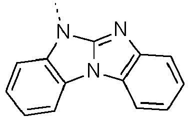 |
| C-130 | 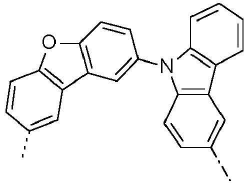 | 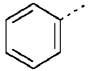 |
| C-131 | 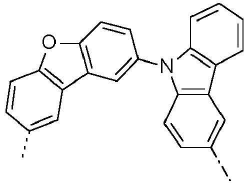 | 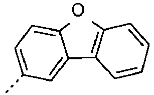 |
| C-132 | 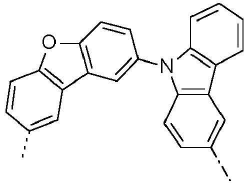 | 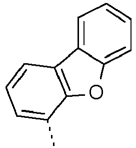 |
| C-133 | 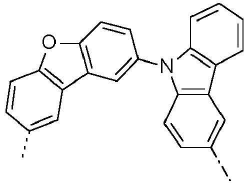 | 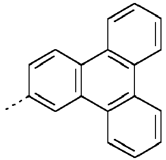 |
| C-134 | 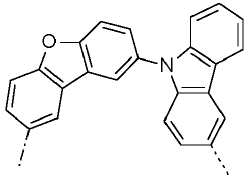 | 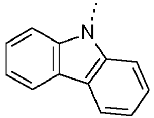 |
| C-135 | 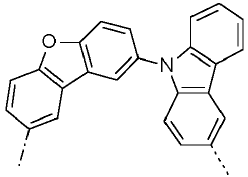 | 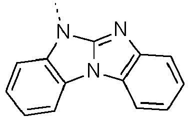 |
| C-136 | 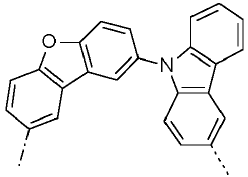 | 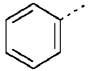 |
| C-137 | 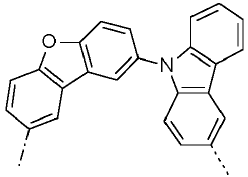 | 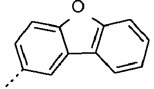 |
| C-138 | 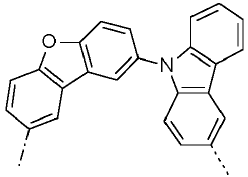 | 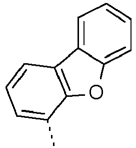 |
| C-139 | 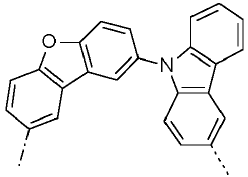 | 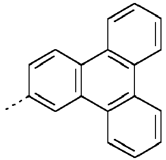 |
| C-140 | 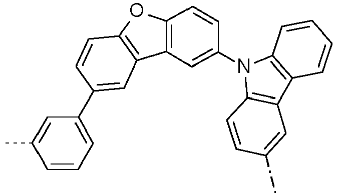 | 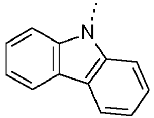 |
| C-141 | 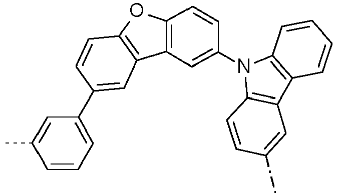 | 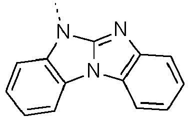 |
| C-142 | 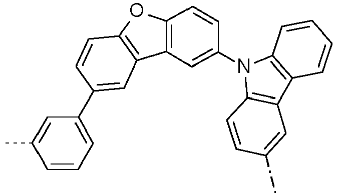 | 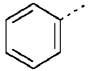 |
| C-143 | 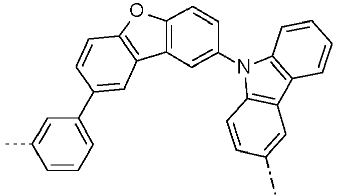 | 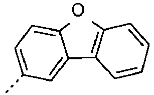 |
| C-144 | 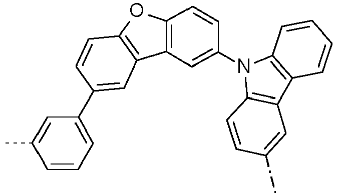 | 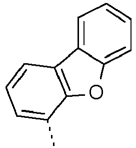 |
| C-145 | 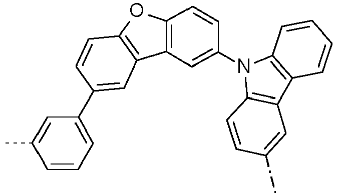 | 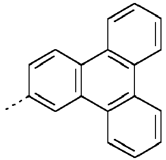 |
| C-146 | 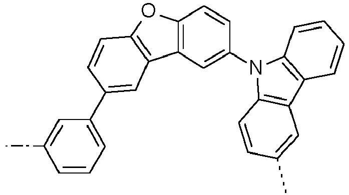 | 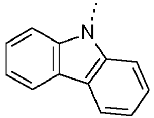 |
| C-147 | 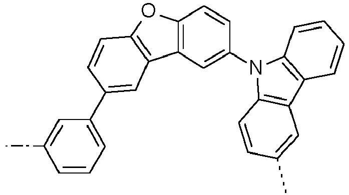 | 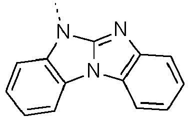 |
| C-148 | 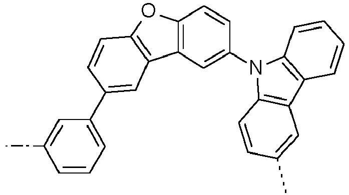 | 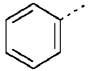 |
| C-149 | 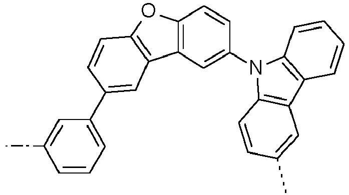 | 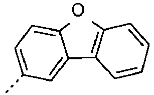 |
| C-150 | 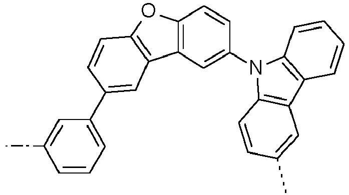 | 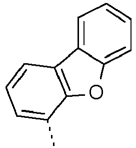 |
| C-151 | 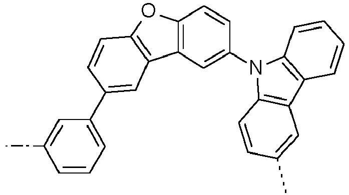 | 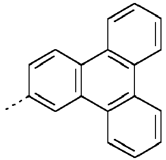 |
| C-152 | 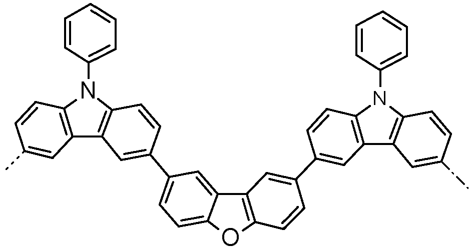 | 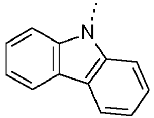 |
| C-153 | 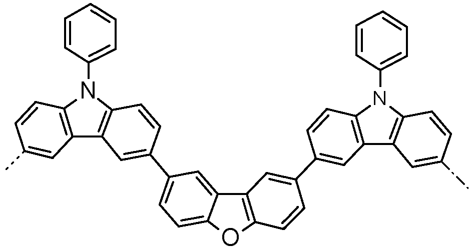 | 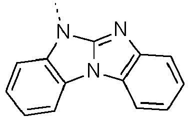 |
| C-154 | 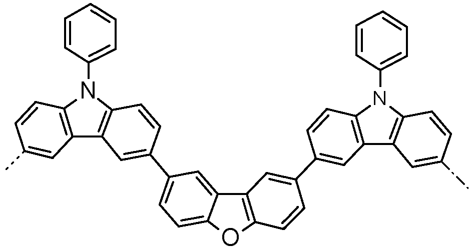 | 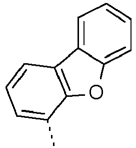 |
| C-155 | 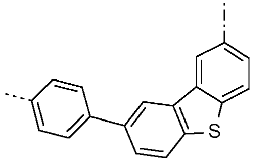 | 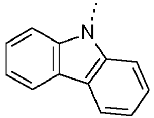 |
| C-156 | 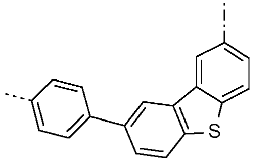 | 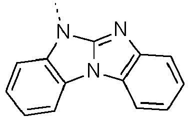 |
| C-157 | 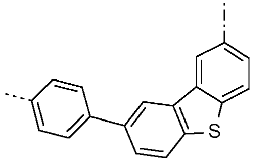 | 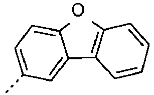 |
| C-158 | 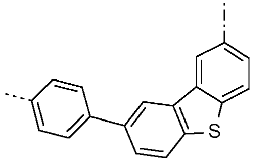 | 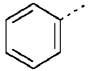 |
| C-159 | 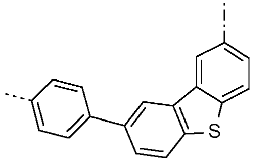 | 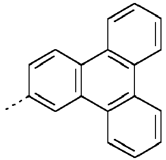 |
| C-160 | 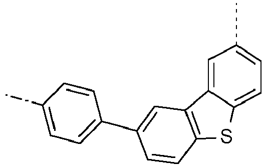 | 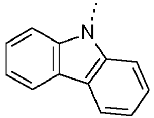 |
| C-161 | 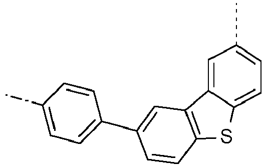 | 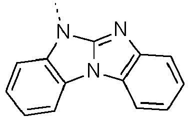 |
| C-162 | 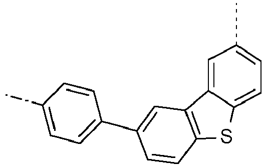 | 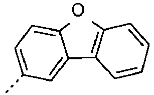 |
| C-163 | 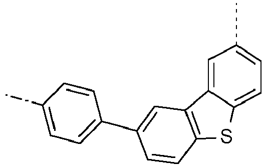 | 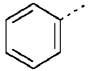 |
| C-164 | 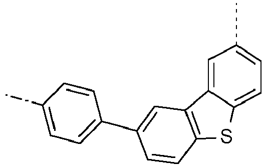 | 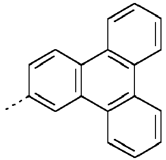 |
| C-165 | 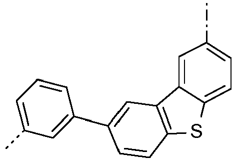 | 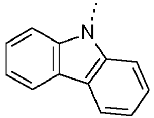 |
| C-166 | 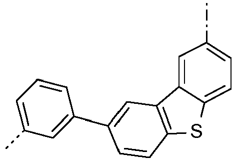 | 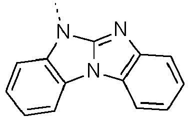 |
| C-167 | 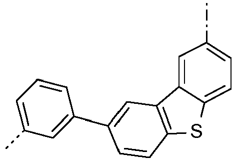 | 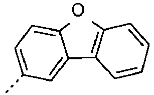 |
| C-168 | 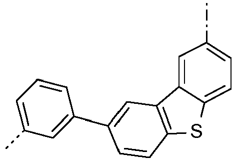 | 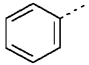 |
| C-169 | 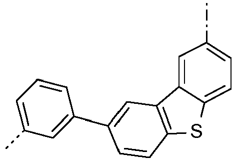 | 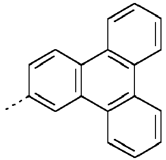 |
| C-170 | 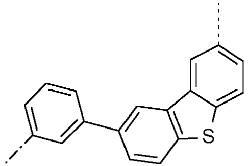 | 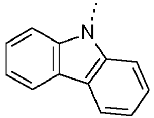 |
| C-171 | 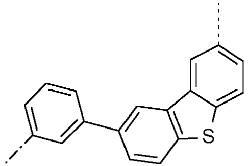 | 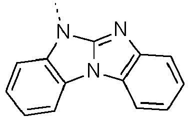 |
| C-172 | 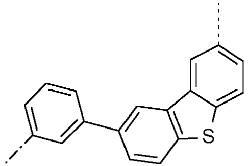 | 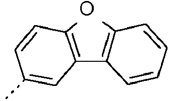 |
| C-173 | 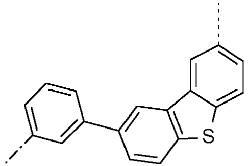 | 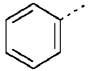 |
| C-174 | 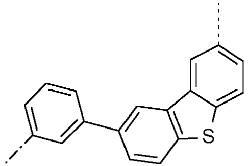 | 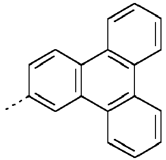 |
| C-175 | 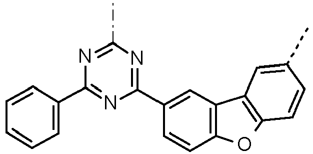 | 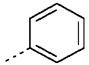 |
| C-176 | 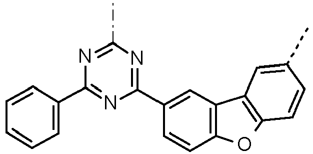 | 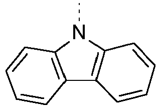 |
| C-177 | 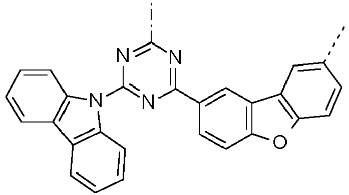 | 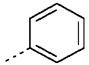 |
| C-178 | 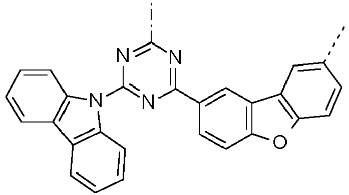 | 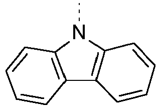 |
| C-179 | 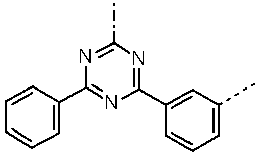 | 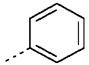 |
| C-180 | 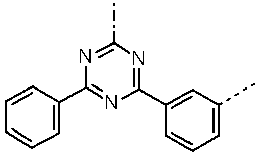 | 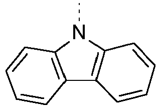 |
| C-181 | 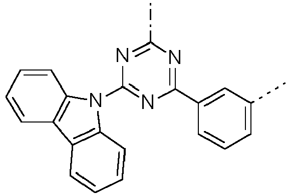 | 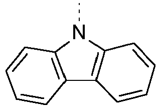 |
| C-182 | 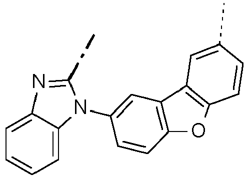 | 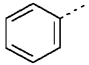 |
| C-183 | 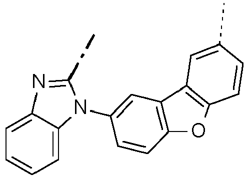 | 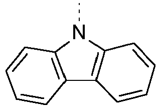 |
| C-184 | 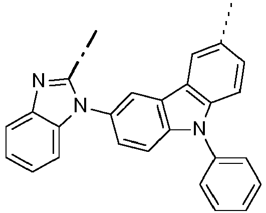 | 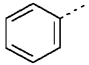 |
| C-185 | 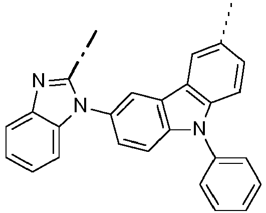 | 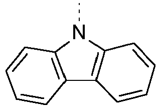 |
| C-186 | 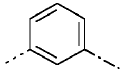 | 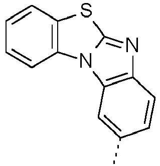 |
| C-187 | 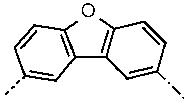 | 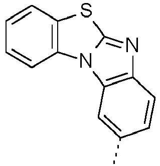 |
| C-188 | 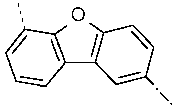 | 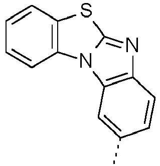 |
| C-189 | 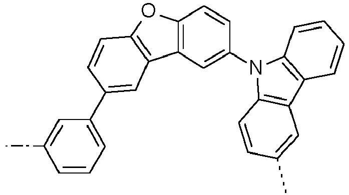 | 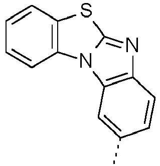 |
| C-190 | 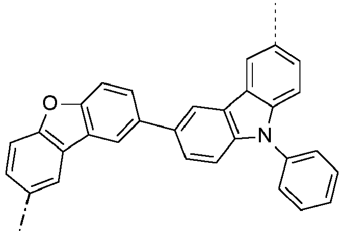 | 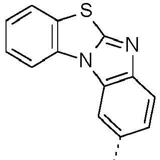 |
| C-191 | 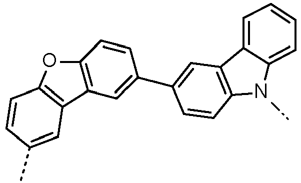 | 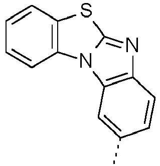 |

| | | |
|---|---|---|
| represents the bonding to R¹⁶. | | |

Compounds **C-1** to **C-191** are preferred, compounds **C-1** to **C-174** are particularly preferred.

Halogen is fluorine, chlorine, bromine and iodine.

C₁-C₂₅alkyl (C₁-C₁₈alkyl) is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

C₁-C₂₅alkoxy groups (C₁-C₁₈alkoxy groups) are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, undecyloxy, dodecyloxy, tetradecyloxy, pentadecyloxy, hexadecyloxy, heptadecyloxy and octadecyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

The term "cycloalkyl group" is typically C₅-C₁₂cycloalkyl, such as cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted.

C₆-C₂₄aryl (C₆-C₁₈aryl), which optionally can be substituted, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted. Phenyl, 1-naphthyl and 2-naphthyl are examples of a C₆-C₁₀aryl group. C₇-C₂₅aralkyl is typically benzyl, 2-benzyl-2-propyl, β-phenyt-ethyl, α,α-dimethylbenzyl, ω-phenyl-butyl, ω,ω-dimethyl-ω-phenyl-butyl, ω-phenyl-dodecyl, ω-phenyl-octadecyl, ω-phenyl-eicosyl or ω-phenyl-docosyl, preferably C₇-C₁₈aralkyl such as benzyl, 2-benzyl-2-propyl, β-phenyt-ethyl, α,α-dimethylbenzyl, ω-phenyl-butyl, ω,ω-dimethyl-ω-phenyl-butyl, ω-phenyl-dodecyl or ω-phenyl-octadecyl, and particularly preferred C₇-C₁₂aralkyl such as benzyl, 2-benzyl-2-propyl, β-phenyt-ethyl, α,α-dimethylbenzyl, ω-phenyl-butyl, or ω,ω-dimethyl-ω-phenyl-butyl, in which both the aliphatic hydrocarbon group and aromatic hydrocarbon group may be unsubstituted or substituted. Preferred examples are benzyl, 2-phenylethyl, 3-phenylpropyl, naphthylethyl, naphthylmethyl, and cumyl.

C₂-C₃₀heteroaryl represents a ring with five to seven ring atoms or a condensed ring system, wherein nitrogen, oxygen or sulfur are the possible hetero atoms, and is typically a heterocyclic group with five to 30 atoms having at least six conjugated π-etectrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, chinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, 4-imidazo[1,2-a]benzimidazoyl, 5-benzimidazo[1,2-a]benzimidazoyl, benzimidazolo[2,1-b][1,3]benzothiazolyl, carbazolyl, or phenoxazinyl, which can be unsubstituted or substituted. Benzimidazo[1,2-a]benzimidazo-5-yl, benzimidazo[1,2-a]benzimidazo-2-yl, carbazolyl and dibenzofuranyl are examples of a C₂-C₁₄heteroaryl group.

C₆-C₂₄arylen groups, which optionally can be substituted by G, are typically phenylene, 4-methylphenylene, 4-methoxyphenylene, naphthylene, especially 1-naphthylene, or 2-naphthylene, biphenylylene, terphenylylene, pyrenylene, 2- or 9-fluorenylene, phenanthrylene, or anthrylene, which may be unsubstituted or substituted. Preferred C₆-C₂₄arylen groups are 1,3-phenylene, 3,3'-biphenylylene, 3,3'-m-terphenylene, 2- or 9-fluorenylene, phenanthrylene, which may be unsubstituted or substituted.

C₂-C₃₀heteroarylen groups, which optionally can be substituted by G, represent a ring with five to seven ring atoms or a condensed ring system, wherein nitrogen, oxygen or sulfur are the possible hetero atoms, and is typically a heterocyclic group with five to 30 atoms having at least six conjugated -electrons such as thienylene, benzothiophenylene, dibenzothiophenylene, thianthrenylene, furylene, furfurylene, 2H-pyranylene, benzofuranylene, isobenzofuranylene, dibenzofuranylene, phenoxythienylene, pyrrolylene, imidazolylene, pyrazolylene, pyridylene, bipyridylene, triazinylene, pyrimidinylene, pyrazinylene, pyridazinylene, indolizinylene, isoindolylene, indolylene, indazolylene, purinylene, chinolizinylene, chinolylene, isochinolylene, phthalazinylene, naphthyridinylene, chinoxalinylene, chinazolinylene, cinnolinylene, pteridinylene, carbolinylene, benzotriazolylene, benzoxazolylene, phenanthridinylene, acridinylene, pyrimidinylene, phenanthrolinylene, phenazinylene, isothiazolylene, phenothiazinylene, isoxazolylene, furazanylene, carbazolylene, benzimidazo[1,2-a]benzimidazo-2,5-ylene, or phenoxazinylene, which can be unsubstituted or substituted. Preferred C₂-C₃₀heteroarylen groups are pyridylene, triazinylene, pyrimidinylene, carbazolylene, dibenzofuranylene and benzimidazo[1,2-a]benzimidazo-2,5-ylene 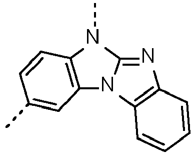 ), which can be unsubstituted or substituted, especially by C₆-C₁₀aryl, C₆-C₁₀aryl which is substituted by C₁-C₄alkyl; or C₂-C₁₄heteroaryl.

Possible substituents of the above-mentioned groups are C₁-C₈alkyl, a hydroxyl group, a mercapto group, C₁-C₈alkoxy, C₁-C₈alkylthio, halogen, halo-C₁-C₈alkyl, or a cyano group. The C₆-C₂₄aryl (C₆-C₁₈aryl) and C₂-C₃₀heteroaryl groups are preferably substituted by one, or more C₁-C₈alkyl groups.

If a substituent occurs more than one time in a group, it can be different in each occurrence.

Halo-C₁-C₈alkyl is an alkyl group where at least one of the hydrogen atoms is replaced by a halogen atom. Examples are -CF₃, -CF₂CF₃, -CF₂CF₂CF₃, -CF(CF₃)₂, -(CF₂)₃CF₃, and -C(CF₃)₃.

The wording "substituted by G" means that one, or more, especially one to three substituents G might be present.

As described above, the aforementioned groups may be substituted by E and/or, if desired, interrupted by D. Interruptions are of course possible only in the case of groups containing at least 2 carbon atoms connected to one another by single bonds; C₆-C₁₈aryl is not interrupted; interrupted arylalkyl contains the unit D in the alkyl moiety. C₁-C₁₈alkyl substituted by one or more E and/or interrupted by one or more units D is, for example, (CH₂CH₂O)₁₋₉-R^{x}, where R^{x} is H or C₁-C₁₀alkyl or C₂-C₁₀alkanoyl (e.g. CO-CH(C₂H₅)C₄H₉), CH₂-CH(OR^{y'})-CH₂-O-R^{y}, where R^{y} is C₁-C₁₈alkyl, C₅-C₁₂cycloalkyl, phenyl, C₇-C₁₅phenylalkyl, and R^{y}' embraces the same definitions as R^{y} or is H;
C₁-C₈alkylene-COO-R^{z}, e.g. CH₂COOR_{z}, CH(CH₃)COOR^{z}, C(CH₃)₂COOR^{z}, where R^{z} is H, C₁-C₁₈alkyl, (CH₂CH₂O)₁₋₉-R^{x}, and R^{x} embraces the definitions indicated above; CH₂CH₂-O-CO-CH=CH₂; CH₂CH(OH)CH₂-O-CO-C(CH₃)=CH₂.
An alkyl group substituted by E is, for example, an alkyl group where at least one of the hydrogen atoms is replaced by F. Examples are -CF₃, -CF₂CF₃, -CF₂CF₂CF₃, -CF(CF₃)₂, -(CF₂)₃CF₃, and -C(CF₃)₃.

The synthesis of 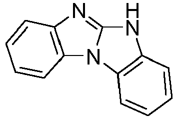 is described, for example, in Achour, Reddouane; Zniber, Rachid, Bulletin des Societes Chimiques Belges 96 (1987) 787-92.

Suitable base skeletons of the formula 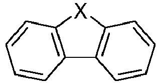 are either commercially available (especially in the cases when X is S, O, NH), or can be obtained by processes known to those skilled in the art. Reference is made to WO2010079051 and EP1885818.

The halogenation can be performed by methods known to those skilled in the art. Preference is given to brominating or iodinating in the 3 and 6 positions (dibromination) or in the 3 or 6 positions (monobromination) of the base skeleton of the formula 2,8 positions (dibenzofuran and dibenzothiophene) or 3,6 positions (carbazole).

Optionally substituted dibenzofurans, dibenzothiophenes and carbazoles can be dibrominated in the 2,8 positions (dibenzofuran and dibenzothiophene) or 3,6 positions (carbazole) with bromine or NBS in glacial acetic acid or in chloroform. For example, the bromination with Br₂ can be effected in glacial acetic acid or chloroform at low temperatures, e.g. 0°C. Suitable processes are described, for example, in M. Park, J.R. Buck, C.J. Rizzo, Tetrahedron, 54 (1998) 12707-12714 for X= NPh, and in W. Yang et al., J. Mater. Chem. 13 (2003) 1351 for X= S. In addition, 3,6-dibromocarbazole, 3,6-dibromo-9-phenylcarbazole, 2,8-dibromodibenzothiophene, 2,8-dibromodibenzofuran, 2-bromocarbazole, 3-bromodibenzothiophene, 3-bromodibenzofuran, 3-bromocarbazole, 2-bromodibenzothiophene and 2-bromodibenzofuran are commercially available.

Monobromination in the 4 position of dibenzofuran (and analogously for dibenzothiophene) is described, for example, in J. Am. Chem. Soc. 1984, 106, 7150. Dibenzofuran (dibenzothiophene) can be monobrominated in the 3 position by a sequence known to those skilled in the art, comprising a nitration, reduction and subsequent Sandmeyer reaction.

Monobromination in the 2 position of dibenzofuran or dibenzothiophene and monobromination in the 3 position of carbazole are effected analogously to the dibromination, with the exception that only one equivalent of bromine or NBS is added.

Alternatively, it is also possible to utilize iodinated dibenzofurans, dibenzothiophenes and carbazoles. The preparation is described, inter alia, in Tetrahedron. Lett. 47 (2006) 6957-6960, Eur. J. Inorg. Chem. 24 (2005) 4976-4984, J. Heterocyclic Chem. 39 (2002) 933-941, J. Am. Chem. Soc. 124 (2002) 11900-11907, J. Heterocyclic Chem, 38 (2001) 77-87.

For the nucleophilic substitution, Cl- or F-substituted dibenzofurans, dibenzothiophenes and carbazoles are required. The chlorination is described, inter alia, in J. Heterocyclic Chemistry, 34 (1997) 891-900, Org. Lett., 6 (2004) 3501-3504; J. Chem. Soc. [Section] C: Organic, 16 (1971) 2775-7, Tetrahedron Lett. 25 (1984) 5363-6, J. Org. Chem. 69 (2004) 8177-8182. The fluorination is described in J. Org. Chem. 63 (1998) 878-880 and J. Chem. Soc., Perkin Trans. 2, 5 2002 953-957.

The introduction of the group 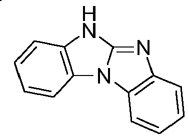 is performed in the presence of a base. Suitable bases are known to those skilled in the art and are preferably selected from the group consisting of alkali metal and alkaline earth metal hydroxides such as NaOH, KOH, Ca(OH)₂, alkali metal hydrides such as NaH, KH, alkali metal amides such as NaNH₂, alkali metal or alkaline earth metal carbonates such as K₂CO₃ or Cs₂CO₃, and alkali metal alkoxides such as NaOMe, NaOEt. In addition, mixtures of the aforementioned bases are suitable. Particular preference is given to NaOH, KOH, NaH or K₂CO₃.

Heteroarylation can be affected, for example, by copper-catalyzed coupling of 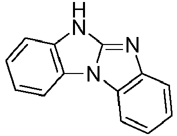 to a halogenated compound of the formula 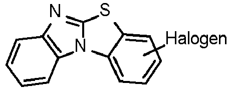 (Ullmann reaction).

The N-arylation is, for example, disclosed in H. Gilman and D. A. Shirley, J. Am. Chem. Soc. 66 (1944) 888; D. Li et al., Dyes and Pigments 49 (2001) 181 - 186 and Eur. J. Org. Chem. (2007) 2147-2151. The reaction can be performed in solvent or in a melt. Suitable solvents are, for example, (polar) aprotic solvents such as dimethyl sulfoxide, dimethylformamide, N-methyl-2-pyrrolidone (NMP), tridecane or alcohols.

The synthesis of 9-(8-bromodibenzofuran-2-yl)carbazole, 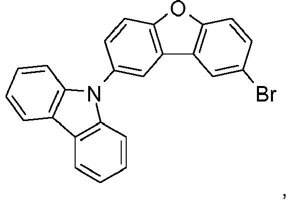 is described in WO2010079051. The synthesis of 2-bromo-8-iodo-dibenzofurane, 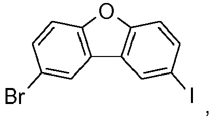 is described in EP1885818.

Diboronic acid or diboronate group containing dibenzofurans, dibenzothiophenes and carbazoles can be readily prepared by an increasing number of routes. An overview of the synthetic routes is, for example, given in Angew. Chem. Int. Ed. 48 (2009) 9240 - 9261.

By one common route diboronic acid or diboronate group containing dibenzofurans, dibenzothiophenes, and carbazoles can be obtained by reacting halogenated dibenzofurans, dibenzothiophenes and carbazoles with (Y¹O)₂B-B(OY¹)₂, 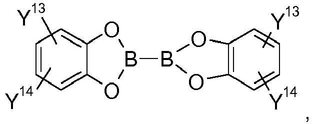 or 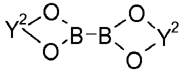 in the presence of a catalyst, such as, for example, [1,1'-bis(diphenylphosphino)ferrocene]dichloropalladium(II), complex (Pd(Cl)₂(dppf)), and a base, such as, for example, potassium acetate, in a solvent, such as, for example, dimethyl formamide, dimethyl sulfoxide, dioxane and/or toluene (cf. Prasad Appukkuttan et al., Synlett 8 (2003) 1204), wherein Y¹ is independently in each occurrence a C₁-C₁₈alkylgroup and Y² is independently in each occurrence a C₂-C₁₀alkylene group, such as -CY³Y⁴-CY⁵Y⁶-, or -CY⁷Y⁸-CY⁹Y¹⁰- CY¹¹Y¹²-, wherein Y³, Y⁴, Y⁵, Y⁶, Y⁷, Y⁸, Y⁹, Y¹⁰, Y¹¹ and Y¹² are independently of each other hydrogen, or a C₁-C₁₈alkylgroup, especially -C(CH₃)₂C(CH₃)₂-, - C(CH₃)₂CH₂C(CH₃)₂-, or -CH₂C(CH₃)₂CH₂-, and Y¹³ and Y¹⁴ are independently of each other hydrogen, or a C₁-C₁₈alkylgroup.

Diboronic acid or diboronate group containing dibenzofurans, dibenzothiophenes and carbazoles can also be prepared by reacting halogenated dibenzofurans, dibenzothiophenes and carbazoles with alkyl lithium reagents, such as, for example, n-butyl lithium, or t-buthyl lithium, followed by reaction with boronic esters, such as, for example, B(isopropoxy)₃, B(methoxy)₃, or 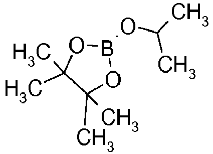 (cf. Synthesis (2000) 442-446). Diboronic acid or diboronate group containing dibenzofurans, dibenzothiophenes and carbazoles can also be prepared by reacting dibenzofurans, dibenzothiophenes and carbazoles with lithium amides, such as, for example, lithium diisopropylamide (LDA) followed by reaction with boronic esters such as, for example, B(isopropoxy)₃, B(methoxy)₃, or 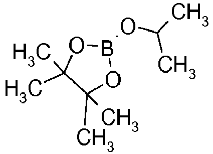 (J. Org. Chem. 73 (2008) 2176-2181).

The synthesis of of the compounds of formula (I) can be done in analogy to methods known in the literature.

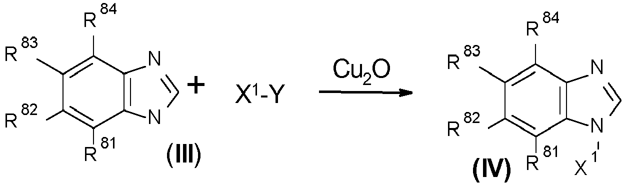

A compound of formula (III) is reacted with a diboronic acid or diboronate group containing compound, X¹-Y, in the presence of a copper compound, such as, for example, Cu₂O, or Cu(OAc)₂, and optionally a base, such as, for example, pyridine, in a solvent or solvent mixture, such as, for example, dimethyl formamide, toluene/methanol, to obtain a compound of formula (IV). Arylation at the N of benzimidazoles is known in the literature (Adv. Synth. Catal. 346 2004 1679.

Y is -B(OH)₂, -B(OY¹)₂, 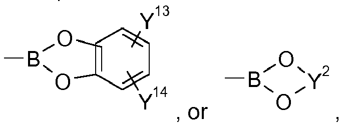 wherein Y¹ is independently in each occurrence a C₁-C₁₀alkyl group and Y² is independently in each occurrence a C₂-C₁₀alkylene group, such as -CY³Y⁴-CY⁵Y⁶-, or -CY⁷Y⁸-CY⁹Y¹⁰- CY¹¹Y¹²-, wherein Y³, Y⁴, Y⁵, Y⁶, Y⁷, Y⁸, Y⁹, Y¹⁰, Y¹¹ and Y¹² are independently of each other hydrogen, or a C₁-C₁₀alkyl group, especially -C(CH₃)₂C(CH₃)₂-, -CH₂C(CH₃)₂CH₂-, or -C(CH₃)₂CH₂C(CH₃)₂-, and Y¹³ and Y¹⁴ are independently of each other hydrogen, or a C₁-C₁₀alkyl group. lodinationion of the 2-position of the imidazole is readily carried out by deprotonation followed by suitable quenching (for example Tetrahedron, 53 (1997) 12755).

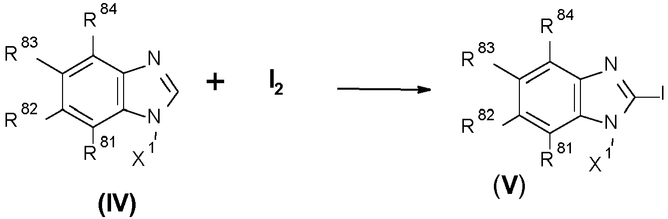

The compound of formula (IV) is reacted with a base, such as, for example, n-butyl lithium, or lithium diisopropylamide, and iodine in a solvent, or solvent mixture, such as, for example, tetrahydrofuran (THF) and THF/hexane to obtain a compound of formula (V).

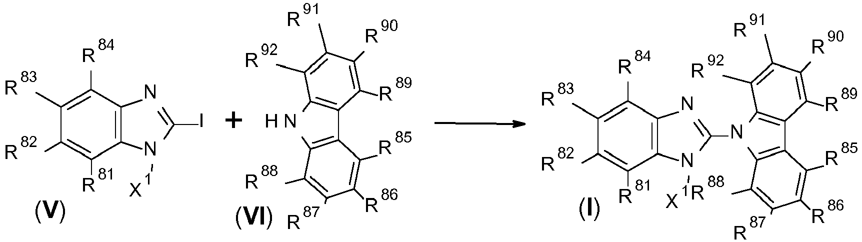

The compound of formula (V) is reacted with the carbazole (VI) in the presence of a copper compound, such as, for example, CuI, and a base, such as, for example, K₃PO₄/1,2-diaminocyclohexane in a solvent, or solvent mixture, such as, for example, dioxan, to obtain a compound of formula (I) (Ullmann Reaction). The N-arylation is, for example, disclosed in H. Gilman and D. A. Shirley, J. Am. Chem. Soc. 66 (1944) 888; D. Li et al., Dyes and Pigments 49 (2001) 181 - 186 and Eur. J. Org. Chem. (2007) 2147-2151. An alternative reaction between compound of formula (V) and the carbazole (VI) is in the presence of a base such as, for example, NaH in a suitable solvent for example DMF (US2013001537).

The synthesis of benzimidazolo[2,1-b][1,3]benzothiazole is, for example, described by Z. Wu et al., Eur. J. Org. Chem. (2011) 5242-5245:

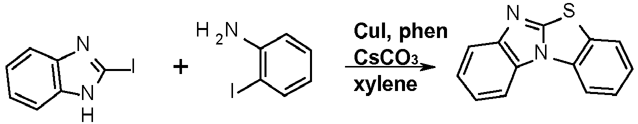

The halogenation can be performed by methods known to those skilled in the art.

4-lodobenzimidazolo[2,1-b][1,3]benzothiazole can be obtained by reacting enzimidazolo[2,1-b][1,3]benzothiazole with butyl lithium and l₂ in tetrahydrofurane. Heteroarylation can be effected, for example, by copper-catalyzed coupling of 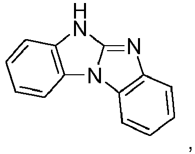 or 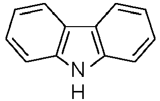 to 4-iodobenzimidazolo[2,1-b][1,3]benzothiazole (Ullmann reaction).

4-Chlorobenzimidazolo[2,1-b][1,3]benzothiazole can be prepared as described in Organic & Biomolecular Chemisty 10 (2012) 7944:
2-lodobenzimidazolo[2,1-b][1,3]benzothiazole can be obtained by reacting benzimidazolo[2,1-b][1,3]benzothiazole in CH₃COOH and CF₃COOH in the presence of N-iodosuccinimide (NIS).

It has been found that the compounds of the formula I are particularly suitable for use in applications in which charge carrier conductivity is required, especially for use in organic electronics applications, for example selected from switching elements such as organic transistors, e.g. organic FETs and organic TFTs, organic solar cells and organic light-emitting diodes (OLEDs), the compounds of the formula I being particularly suitable in OLEDs for use as matrix material in a light-emitting layer and/or as electron and/or exciton blocker material and/or as hole and/or exciton blocker material, especially in combination with a phosphorescence emitter. In the case of use of the inventive compounds of the formula I in OLEDs, OLEDs which have good efficiencies and a long lifetime and which can be operated especially at a low use and operating voltage are obtained. The inventive compounds of the formula I are suitable especially for use as matrix and/or charge/exciton blocker materials for blue and green emitters, for example light blue or deep blue emitters, these being especially phosphorescence emitters. Furthermore, the compounds of the formula I can be used as conductor/complementary materials in organic electronics applications selected from switching elements and organic solar cells.

The compounds of the formula I can be used as matrix material and/or charge/exciton blocker material and/or charge transport material (charge conductor material). The inventive compounds of the formula I are preferably used as matrix materials in organic electronics applications, especially in OLEDs.

In the emission layer or one of the emission layers of an OLED, it is also possible to combine an emitter material with a matrix material of the compound of the formula I and a further matrix material which has, for example, a good hole transport property. This achieves a high quantum efficiency of this emission layer.

When a compound of the formula I is used as matrix (host) material in an emission layer and additionally as charge/exciton blocker material, owing to the chemical identity or similarity of the materials, an improved interface between the emission layer and the adjacent charge/exciton blocker material, which can lead to a decrease in the voltage with equal luminance and to an extension of the lifetime of the OLED. Moreover, the use of the same material for charge/exciton blocker material and for the matrix of an emission layer allows the production process of an OLED to be simplified, since the same source can be used for the vapor deposition process of the material of one of the compounds of the formula I.

Suitable structures of organic electronic devices are known to those skilled in the art and are specified below.

The organic transistor generally includes a semiconductor layer formed from an organic layer with charge transport capacity; a gate electrode formed from a conductive layer; and an insulating layer introduced between the semiconductor layer and the conductive layer. A source electrode and a drain electrode are mounted on this arrangement in order thus to produce the transistor element. In addition, further layers known to those skilled in the art may be present in the organic transistor.

The organic solar cell (photoelectric conversion element) generally comprises an organic layer present between two plate-type electrodes arranged in parallel. The organic layer may be configured on a comb-type electrode. There is no particular restriction regarding the site of the organic layer and there is no particular restriction regarding the material of the electrodes. When, however, plate-type electrodes arranged in parallel are used, at least one electrode is preferably formed from a transparent electrode, for example an ITO electrode or a fluorine-doped tin oxide electrode. The organic layer is formed from two sublayers, i.e. a layer with p-type semiconductor properties or hole transport capacity, and a layer formed with n-type semiconductor properties or charge transport capacity. In addition, it is possible for further layers known to those skilled in the art to be present in the organic solar cell. The layers with charge transport capacity may comprise the compounds of formula I.

It is likewise possible that the compounds of the formula I are present both in the light-emitting layer (preferably as matrix material) and in the blocking layers (as charge/exciton blockers).

The present invention further provides an organic light-emitting diode comprising an anode (a) and a cathode (i) and a light-emitting layer (e) arranged between the anode (a) and the cathode (i), and if appropriate at least one further layer selected from the group consisting of at least one blocking layer for holes/excitons, at least one blocking layer for electrons/excitons, at least one hole injection layer, at least one hole transport layer, at least one electron injection layer and at least one electron transport layer, wherein the at least one compound of the formula I is present in the light-emitting layer (e) and/or in at least one of the further layers. The at least one compound of the formula **I** is preferably present in the light-emitting layer and/or the charge/exciton blocking layers.

In a preferred embodiment of the present invention, at least one compound of the formula **I**, especially a compound of the formula (**Ia**), very especially a compound of the formula (**Ia-2**), is used as charge transport material. Examples of preferred compounds of formula I are compounds **C-**1 to **C-191** shown above. Compounds **C-1** to **C-174** are particularly preferred.

In another preferred embodiment of the present invention, at least one compound of the formula **I**, especially a compound of the formula (**Ia**), very especially a compound of the formula (**Ia-2**), is used as charge/exciton blocker material. Examples of preferred compounds of formula **I** are compounds **C-1** to **C-191** shown above. Compounds **C-1** to **C-174** are particularly preferred.

The present application further relates to a light-emitting layer comprising at least one compound of the formula **I**.

### Structure of the inventive OLED

The inventive organic light-emitting diode (OLED) thus generally has the following structure:
an anode (a) and a cathode (i) and a light-emitting layer (e) arranged between the anode (a) and the cathode (i).

The inventive OLED may, for example - in a preferred embodiment - be formed from the following layers:
1. Anode (a)
2. Hole transport layer (c)
3. Light-emitting layer (e)
4. Blocking layer for holes/excitons (f)
5. Electron transport layer (g)
6. Cathode (i)

Layer sequences different than the aforementioned structure are also possible, and are known to those skilled in the art. For example, it is possible that the OLED does not have all of the layers mentioned; for example, an OLED with layers (a) (anode), (e) (light-emitting layer) and (i) (cathode) is likewise suitable, in which case the functions of the layers (c) (hole transport layer) and (f) (blocking layer for holes/excitons) and (g) (electron transport layer) are assumed by the adjacent layers. OLEDs which have layers (a), (c), (e) and (i), or layers (a), (e), (f), (g) and (i), are likewise suitable. In addition, the OLEDs may have a blocking layer for electrons/excitons (d) between the hole transport layer (c) and the Light-emitting layer (e).

It is additionally possible that a plurality of the aforementioned functions (electron/exciton blocker, hole/exciton blocker, hole injection, hole conduction, electron injection, electron conduction) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron conduction layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used in accordance with the invention.

In a preferred embodiment the OLED according to the present invention comprises in this order:
(a) an anode,
(b) optionally a hole injection layer,
(c) optionally a hole transport layer,
(d) optionally an exciton blocking layer
(e) an emitting layer,
(f) optionally a hole/ exciton blocking layer
(g) optionally an electron transport layer,
(h) optionally an electron injection layer, and
(i) a cathode.

In a particularly preferred embodiment the OLED according to the present invention comprises in this order:
(a) an anode,
(b) optionally a hole injection layer,
(c) a hole transport layer,
(d) an exciton blocking layer
(e) an emitting layer,
(f) a hole/ exciton blocking layer
(g) an electron transport layer, and
(h) optionally an electron injection layer, and
(i) a cathode.

The properties and functions of these various layers, as well as example materials are known from the prior art and are described in more detail below on basis of preferred embodiments.

### Anode (a):

The anode is an electrode which provides positive charge carriers. It may be composed, for example, of materials which comprise a metal, a mixture of different metals, a metal alloy, a metal oxide or a mixture of different metal oxides. Alternatively, the anode may be a conductive polymer. Suitable metals comprise the metals of groups 11, 4, 5 and 6 of the Periodic Table of the Elements, and also the transition metals of groups 8 to 10. When the anode is to be transparent, mixed metal oxides of groups 12, 13 and 14 of the Periodic Table of the Elements are generally used, for example indium tin oxide (ITO). It is likewise possible that the anode (a) comprises an organic material, for example polyaniline, as described, for example, in Nature, Vol. 357, pages 477 to 479 (June 11, 1992). Preferred anode materials include conductive metal oxides, such as indium tin oxide (ITO) and indium zinc oxide (IZO), aluminum zinc oxide (AlZnO), and metals. Anode (and substrate) may be sufficiently transparent to create a bottom-emitting device. A preferred transparent substrate and anode combination is commercially available ITO (anode) deposited on glass or plastic (substrate). A reflective anode may be preferred for some top-emitting devices, to increase the amount of light emitted from the top of the device. At least either the anode or the cathode should be at least partly transparent in order to be able to emit the light formed. Other anode materials and structures may be used.

### Hole injection layer (b):

Generally, injection layers are comprised of a material that may improve the injection of charge carriers from one layer, such as an electrode or a charge generating layer, into an adjacent organic layer. Injection layers may also perform a charge transport function. The hole injection layer may be any layer that improves the injection of holes from anode into an adjacent organic layer. A hole injection layer may comprise a solution deposited material, such as a spin-coated polymer, or it may be a vapor deposited small molecule material, such as, for example, CuPc or MTDATA. Polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS.

### Hole transport layer (c):

Either hole-transporting molecules or polymers may be used as the hole transport material. Suitable hole transport materials for layer (c) of the inventive OLED are disclosed, for example, in Kirk-Othmer Encyclopedia of Chemical Technology, 4th Edition, Vol. 18, pages 837 to 860, 1996, US20070278938, US2008/0106190, US2011/0163302 (triarylamines with (di)benzothiophen/(di)benzofuran; Nan-Xing Hu et al. Synth. Met. 111 (2000) 421 (indolocarbazoles), WO2010002850 (substituted phenylamine compounds) and WO2012/16601 (in particular the hole transport materials mentioned on pages 16 and 17 of WO2012/16601). Combination of different hole transport material may be used. Reference is made, for example, to WO2013/022419, wherein 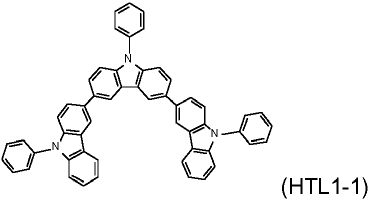 and 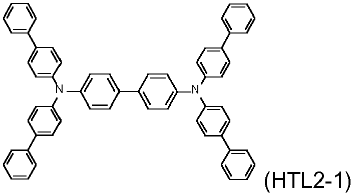 constitute the hole transport layer. Customarily used hole-transporting molecules are selected from the group consisting of 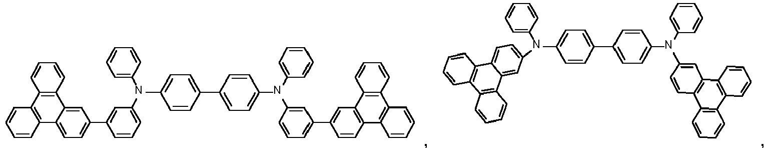 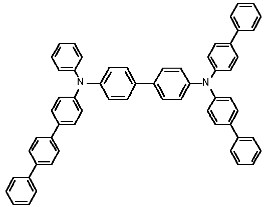 (4-phenyl-N-(4-phenylphenyl)-N-[4-[4-(N-[4-(4-phenyl-phenyl)phenyl]anilino)phenyl]phenyl]aniline), 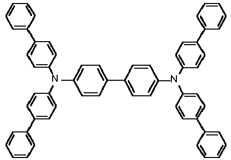 (4-phenyl-N-(4-phenylphenyl)-N-[4-[4-(4-phenyl-N-(4-phenylphenyl)anilino)phenyl]phenyl]aniline), 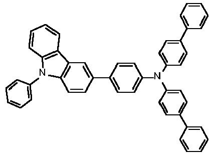 (4-phenyl-N-[4-(9-phenylcarbazol-3-yl) phenyl]-N-(4-phenylphenyl)aniline), 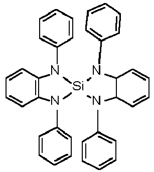 (1,1',3,3'-tetraphenylspiro[1,3,2-benzodiazasilole-2,2'-3a,7a-dihydro-1,3,2-benzodiazasilole]), 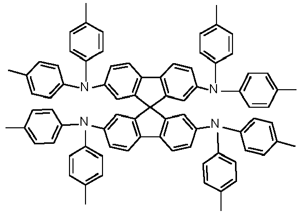 (N2,N2,N2',N2',N7,N7,N7',N7'-octakis(p-tolyl)-9,9'-spirobi[fluorene]-2,2',7,7'-tetramine),4,4'-bis[N-(1-naphthyl)-N-phenylamino]biphenyl (α-NPD), N,N'-diphenyl-N,N'-bis(3-methylphenyl)-[1,1'-biphenyl]-4,4'-diamine (TPD), 1,1-bis[(di-4-tolylamino)phenyl]-cyclohexane (TAPC), N,N'-bis(4-methylphenyl)-N,N'-bis(4-ethylphenyl)-[1,1'-(3,3'-dimethyl)-biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)2-methylphenyl](4-methylphenyl)methane (MPMP), 1-phenyl-3-[p-(diethylamino)styryl]5-[p-(diethylamino)phenyl]pyrazoline (PPR or DEASP), 1,2-trans-bis(9H-carbazol9-yl)-cyclobutane (DCZB), N,N,N',N'-tetrakis(4-methylphenyl)-(1,1'-biphenyl)-4,4'-diamine (TTB), fluorine compounds such as 2,2',7,7'-tetra(N,N-di-tolyl)amino9,9-spirobifluorene (spiro-TTB), N,N'-bis(naphthalen-1-yl)-N,N'-bis(phenyl)9,9-spirobifluorene (spiro-NPB) and 9,9-bis(4-(N,N-bis-biphenyl-4-yl-amino)phenyl-9Hfluorene, benzidine compounds such as N,N'-bis(naphthalen-1-yl)-N,N'-bis(phenyl)benzidine and porphyrin compounds such as copper phthalocyanines. In addition, polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PE-DOT/PSS. Preferred examples of a material of the hole injecting layer are a porphyrin compound, an aromatic tertiary amine compound, or a styrylamine compound. Particularly preferable examples include an aromatic tertiary amine compound such as hexacyanohexaazatriphenylene (HAT).

In a preferred embodiment it is possible to use metal carbene complexes as hole transport materials. Suitable carbene complexes are, for example, carbene complexes as described in WO2005/019373A2, WO2006/056418 A2, WO2005/113704, WO2007/115970, WO2007/115981, WO2008/000727 and PCT/EP2014/055520. One example of a suitable carbene complex is Ir(DPBIC)₃ with the formula: 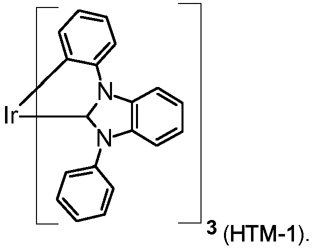 Another example of a suitable carbene complex is Ir(ABIC)₃ with the la:

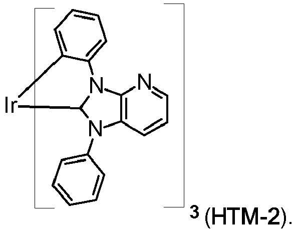

The hole-transporting layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, 2003, 359 (p-doped organic layers); A. G. Werner, F. Li, K. Harada, M. Pfeiffer, T. Fritz, K. Leo, Appl. Phys. Lett., Vol. 82, No. 25, 2003, 4495 and Pfeiffer et al., Organic Electronics 2003, 4, 89 - 103 and K. Walzer, B. Maennig, M. Pfeiffer, K. Leo, Chem. Soc. Rev. 2007, 107, 1233. For example it is possible to use mixtures in the hole-transporting layer, in particular mixtures which lead to electrical p-doping of the hole-transporting layer. p-Doping is achieved by the addition of oxidizing materials. These mixtures may, for example, be the following mixtures: mixtures of the abovementioned hole transport materials with at least one metal oxide, for example MoO₂, MoO₃, WOₓ, ReO₃ and/or V₂O₅, preferably MoO₃ and/or ReO₃, more preferably MoO₃, or mixtures comprising the aforementioned hole transport materials and one or more compounds selected from 7,7,8,8-tetracyanoquinodimethane (TCNQ), 2,3,5,6-tetrafluoro-7,7,8,8-tetracyanoquinodimethane (F₄-TCNQ), 2,5-bis(2-hydroxyethoxy)-7,7,8,8-tetracyanoquinodimethane, bis(tetra-n-butylammonium)tetracyanodiphenoquinodimethane, 2,5-dimethyl-7,7,8,8-tetracyanoquinodimethane, tetracyanoethylene, 11,11,12,12-tetracyanonaphtho2,6-quinodimethane, 2-fluoro-7,7,8,8-tetracyanoquino-dimethane, 2,5-difluoro-7,7,8,8etracyanoquinodimethane, dicyanomethylene-1,3,4,5,7,8-hexafluoro-6H-naphthalen-2-ylidene)malononitrile (F₆-TNAP), Mo(tfd)₃ (from Kahn et al., J. Am. Chem. Soc. 2009, 131 (35), 12530-12531), compounds as described in EP1988587, US2008265216, EP2180029, US20100102709, WO2010132236, EP2180029 and quinone compounds as mentioned in EP2401254. Preferred mixtures comprise the aforementioned carbene complexes, such as, for example, the carbene complexes **HTM-1** and **HTM-2,** and MoO₃ and/or ReO₃, especially MoO₃. In a particularly preferred embodiment the hole transport layer comprises from 0.1 to 10 wt % of MoO₃ and 90 to 99.9 wt % carbene complex, especially of the carbene complex **HTM-1** and **HTM-2**, wherein the total amount of the MoO₃ and the carbene complex is 100 wt %.

### Exciton blocking layer (d):

Blocking layers may be used to reduce the number of charge carriers (electrons or holes) and/or excitons that leave the emissive layer. An electron/exciton blocking layer (d) may be disposed between the first emitting layer (e) and the hole transport layer (c), to block electrons from emitting layer (e) in the direction of hole transport layer (c). Blocking layers may also be used to block excitons from diffusing out of the emissive layer. Suitable metal complexes for use as electron/exciton blocker material are, for example, carbene complexes as described in WO2005/019373A2, WO2006/056418A2, WO2005/113704, WO2007/115970, WO2007/115981, WO2008/000727 and PCT/EP2014/055520. Explicit reference is made here to the disclosure of the WO applications cited, and these disclosures shall be considered to be incorporated into the content of the present application. One example of a suitable carbene complex is compound **HTM-1** and **HTM-2**.

### Emitting layer (e)

The light-emitting layer (e) comprises at least one emitter material. In principle, it may be a fluorescence or phosphorescence emitter, suitable emitter materials being known to those skilled in the art. The at least one emitter material is preferably a phosphorescence emitter. The phosphorescence emitter compounds used with preference are based on metal complexes, and especially the complexes of the metals Ru, Rh, Ir, Pd and Pt, in particular the complexes of Ir, have gained significance. The compounds of the formula I can be used as the matrix in the light-emitting layer.

Suitable metal complexes for use in the inventive OLEDs are described, for example, in documents WO 02/60910 A1, US 2001/0015432 A1, US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1, WO 2006/056418, WO 2006121811 A1, WO 2007095118 A2, WO 2007/115970, WO 2007/115981, WO 2008/000727, WO2010129323, WO2010056669, WO10086089, US2011/0057559, WO2011/106344, US2011/0233528, WO2012/048266 and WO2012/172482.

Further suitable metal complexes are the commercially available metal complexes tris(2-phenylpyridine)iridium(III), iridium(III) tris(2-(4-tolyl)pyridinato-N,C²'), bis(2-phenylpyridine)(acetylacetonato)iridium(III), iridium(III) tris(1-phenylisoquinoline), iridium(III) bis(2,2'-benzothienyl)pyridinato-N,C³')(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldibenzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetylacetonato)iridium(III), bis(2-phenylbenzothiazolato)(acetylacetonato)iridium(III), bis(2-(9,9-dihexylfluorenyl)-1-pyridine)(acetylacetonato)iridium(III), bis(2-benzo[b]thiophen-2-yl-pyridine)(acetylacetonato)iridium(III).

In addition, the following commercially available materials are suitable: tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)-mono(phenanthroline)europium(III), tris(dibenzoylmethane)mono(4,7-diphenyl-phenanthroline)europium(III), tris(dibenzoylmethane)mono(4,7-di-methyl-phenanthroline)europium(III), tris(dibenzoylmethane)mono(4,7-dimethylphenanthrolinedisulfonic acid)europium(III) disodium salt, tris[di(4-(2-(2-ethoxyethoxy)ethoxy)-benzoylmethane)]mono(phenanthroline)europium(III) and tris[di[4-(2-(2-ethoxy-ethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)-pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Preferred phosphorescence emitters are carbene complexes. Suitable phosphorescent blue emitters are specified in the following publications: WO2006/056418A2, WO2005/113704, WO2007/115970, WO2007/115981, WO2008/000727, WO2009050281, WO2009050290, WO2011051404, US2011/057559 WO2011/073149, WO2012/121936A2, US2012/0305894A1, WO2012/170571, WO2012/170461, WO2012/170463, WO2006/121811, WO2007/095118, WO2008/156879, WO2008/156879, WO2010/068876, US2011/0057559, WO2011/106344, US2011/0233528, WO2012/048266, WO2012/172482, PCT/EP2014/064054 and PCT/EP2014/066272.

Preferably, the light emitting layer (e) comprises at least one carbine complex as phosphorescence emitter. Suitable carbine complexes are, for example, compounds of the formula 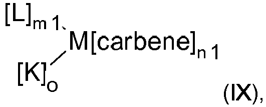 which are described in WO 2005/019373 A2, wherein the symbols have the following meanings:
M is a metal atom selected from the group consisting of Co, Rh, Ir, Nb, Pd, Pt, Fe, Ru, Os, Cr, Mo, W, Mn, Tc, Re, Cu, Ag and Au in any oxidation state possible for the respective metal atom;
Carbene is a carbene ligand which may be uncharged or monoanionic and monodentate, bidentate or tridentate, with the carbene ligand also being able to be a biscarbene or triscarbene ligand;
L is a monoanionic or dianionic ligand, which may be monodentate or bidentate;
K is an uncharged monodentate or bidentate ligand selected from the group consisting of phosphines; phosphonates and derivatives thereof, arsenates and derivatives thereof; phosphites; CO; pyridines; nitriles and conjugated dienes which form a π complex with M¹; n1 is the number of carbene ligands, where n1 is at least 1 and when n1 > 1 the carbene ligands in the complex of the formula I can be identical or different;
m1 is the number of ligands L, where m1 can be 0 or ≥ 1 and when m1 > 1 the ligands L can be identical or different;
o is the number of ligands K, where o can be 0 or ≥ 1 and when o > 1 the ligands K can be identical or different;
where the sum n1 + m1 + o is dependent on the oxidation state and coordination number of the metal atom and on the denticity of the ligands carbene, L and K and also on the charge on the ligands, carbene and L, with the proviso that n1 is at least 1.

More preferred are metal-carbene complexes of the general formula 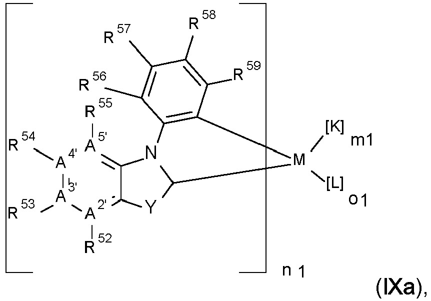 which are described in WO2011/073149, where M, n1, Y, A^{2'}, A^{3'}, A^{4'}, A^{5'}, R⁵¹, R⁵², R⁵³, R⁵⁴, R⁵⁵, R⁵⁶, R⁵⁷, R⁵⁸, R⁵⁹, K, L, m1 and o1 are each defined as follows:
M is Ir, or Pt,
n1 is an integer selected from 1, 2 and 3,
Y is NR⁵¹, O, S or C(R²⁵)₂,
A^{2'}, A^{3'}, A^{4'}, and A^{5'} are each independently N or C, where 2 A' = nitrogen atoms and at least one carbon atom is present between two nitrogen atoms in the ring,
R⁵¹ is a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
R⁵², R⁵³, R⁵⁴ and R⁵⁵ are each, if A^{2'}, A^{3'}, A^{4'} and/or A^{5'} is N, a free electron pair, or, if A^{2'}, A^{3'}, A^{4'} and/or A^{5'} is C, each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R⁵³ and R⁵⁴ together with A^{3'} and A^{4'} form an optionally substituted, unsaturated ring optionally interrupted by at least one further heteroatom and having a total of 5 to 18 carbon atoms and/or heteroatoms,
R⁵⁶, R⁵⁷, R⁵⁸ and R⁵⁹ are each independently hydrogen, linear or branched alkyl radical
optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R⁵⁶ and R⁵⁷, R⁵⁷ and R⁵⁸ or R⁵⁸ and R⁵⁹, together with the carbon atoms to which they are bonded, form a saturated, unsaturated or aromatic, optionally substituted ring optionally interrupted by at least one heteroatom and having a total of 5 to 18 carbon atoms and/or heteroatoms, and/or
if A^{5'} is C, R⁵⁵ and R⁵⁶ together form a saturated or unsaturated, linear or branched bridge optionally comprising heteroatoms, an aromatic unit, heteroaromatic unit and/or functional groups and having a total of 1 to 30 carbon atoms and/or heteroatoms, to which is optionally fused a substituted or unsubstituted, five- to eight-membered ring comprising carbon atoms and/or heteroatoms,
R²⁵ is independently a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
K is an uncharged mono- or bidentate ligand,
L is a mono- or dianionic ligand, preferably monoanionic ligand, which may be mono- or bidentate,
m1 is 0, 1 or 2, where, when m1 is 2, the K ligands may be the same or different,
o1 is 0, 1 or 2, where, when o1 is 2, the L ligands may be the same or different.

The compound of formula IX is preferably a compound of the formula:

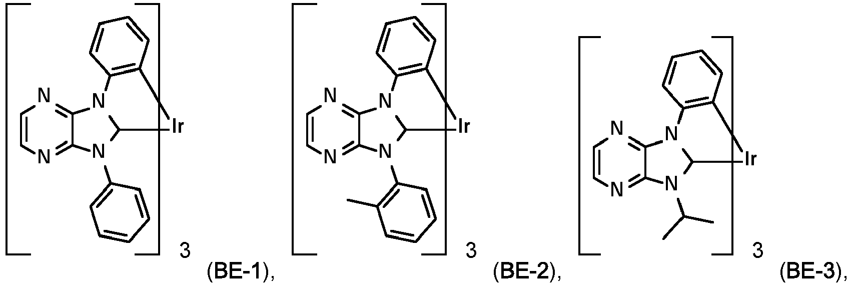

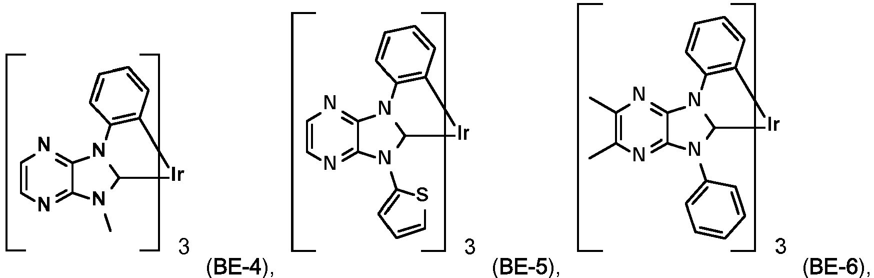

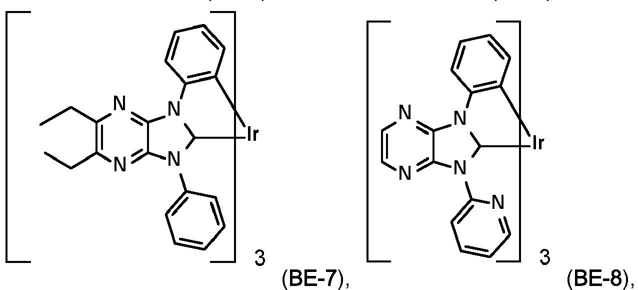

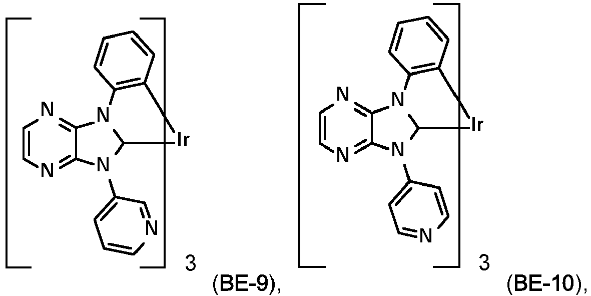

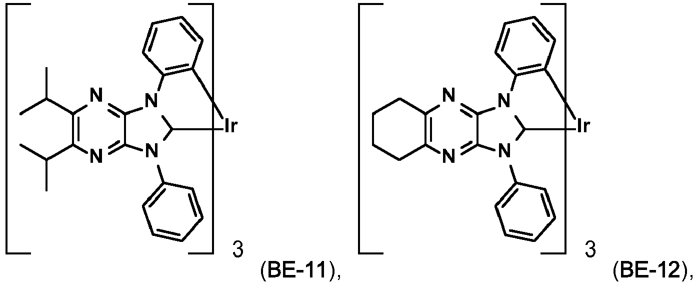

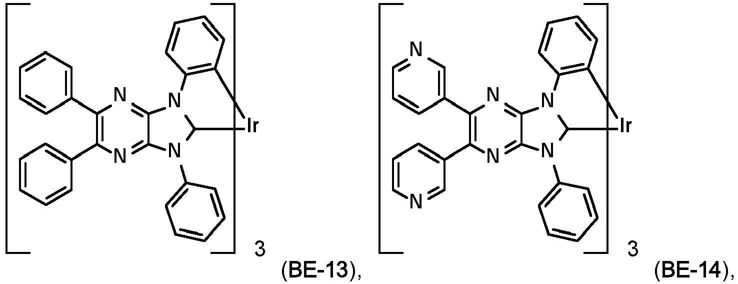

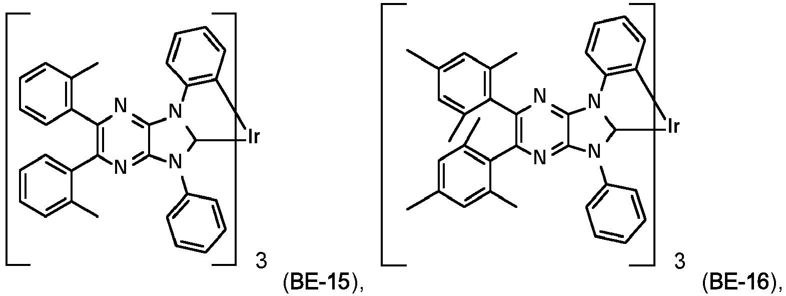

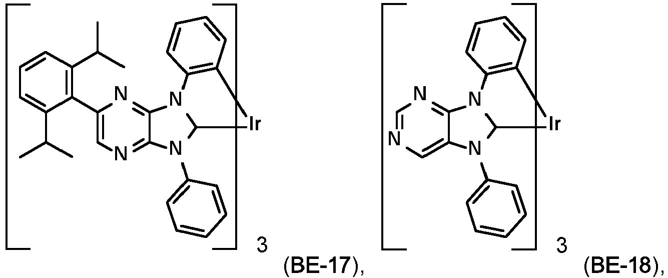

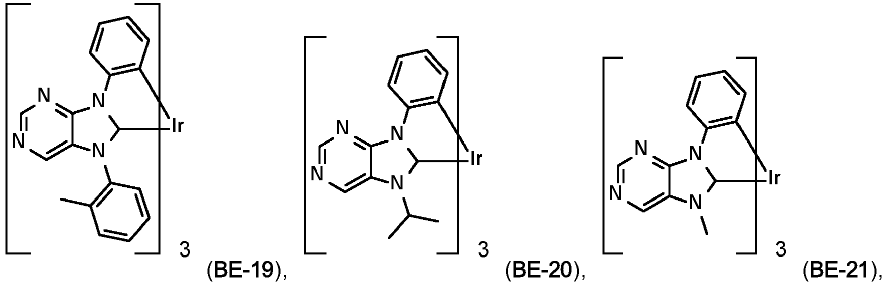

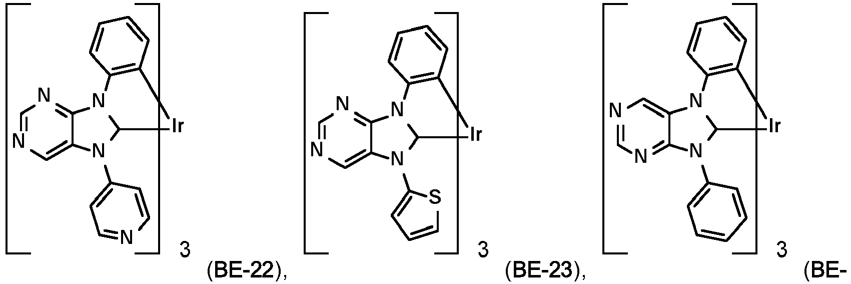

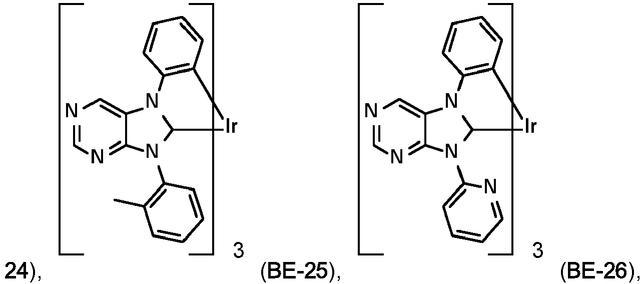

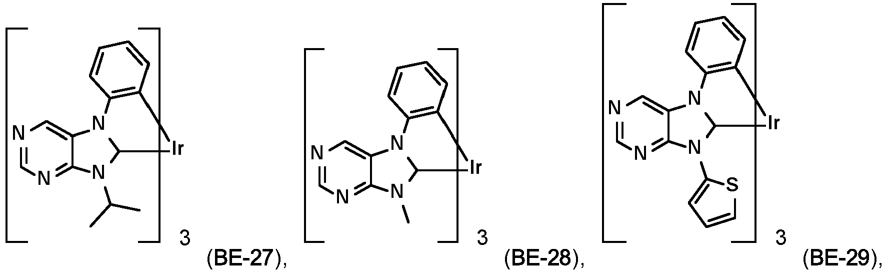

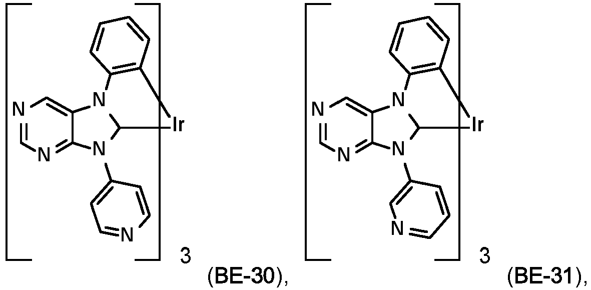

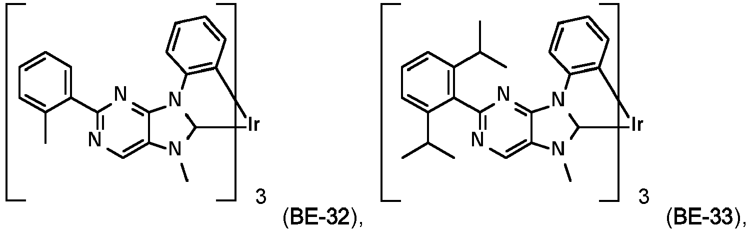

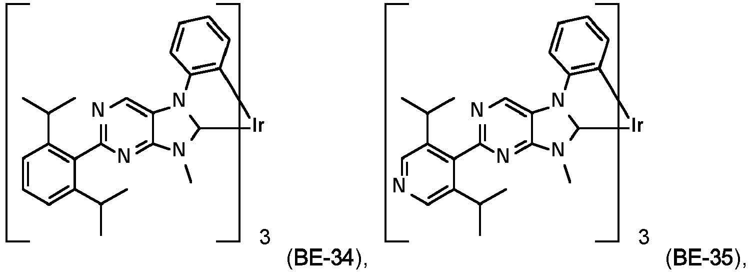

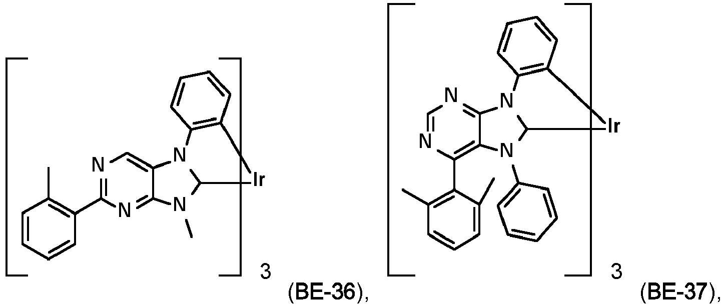

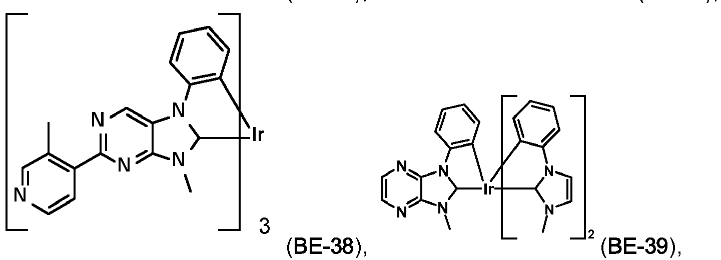

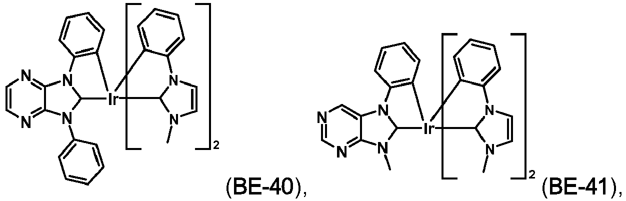

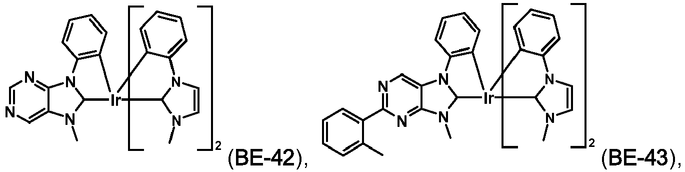

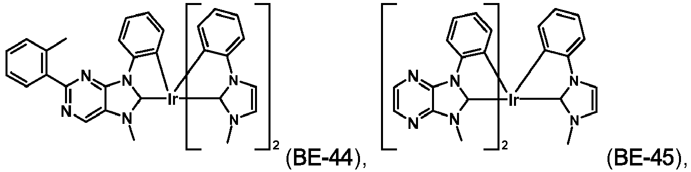

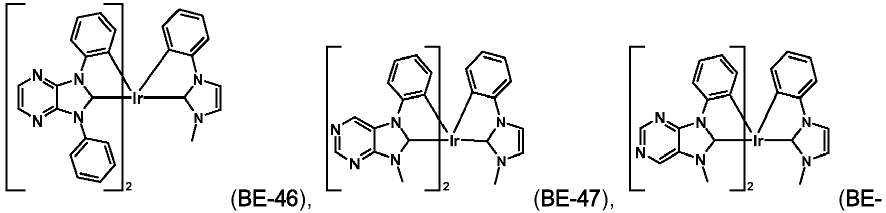

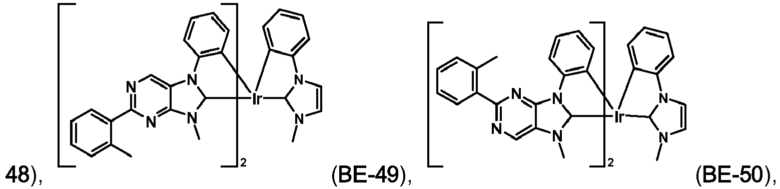

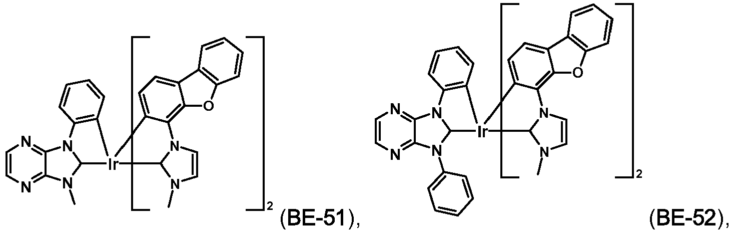

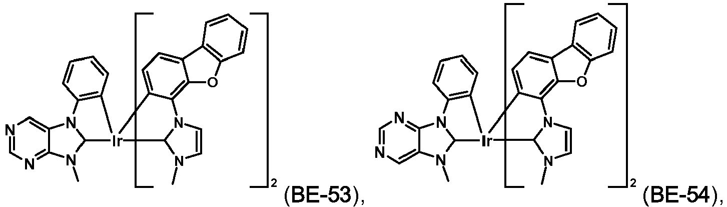

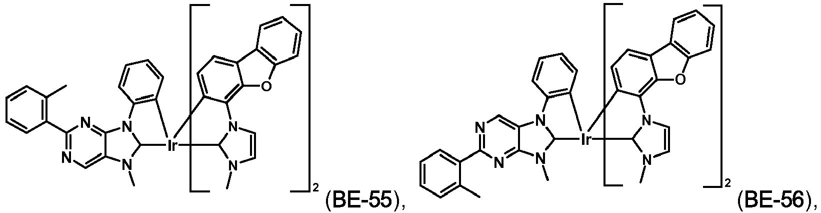

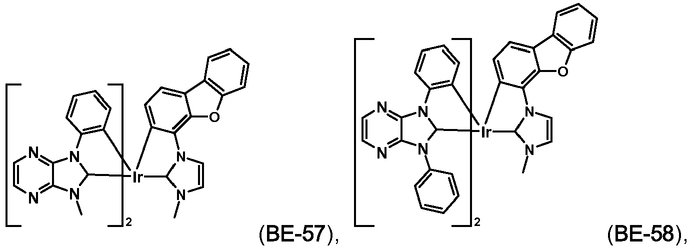

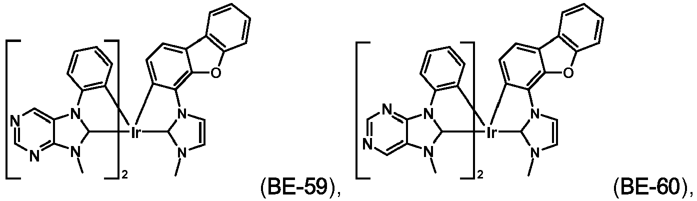

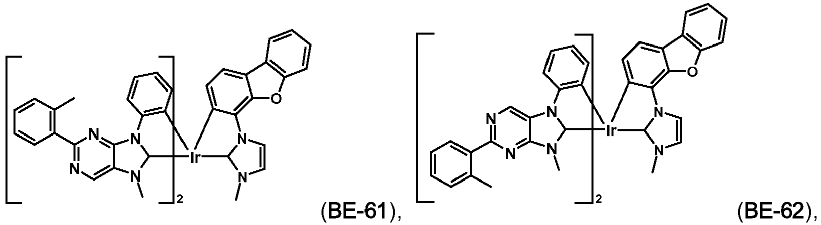

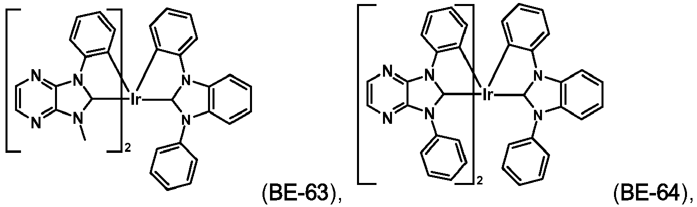

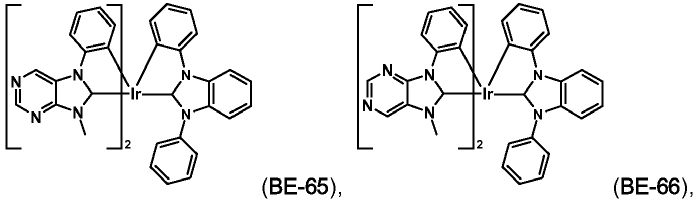

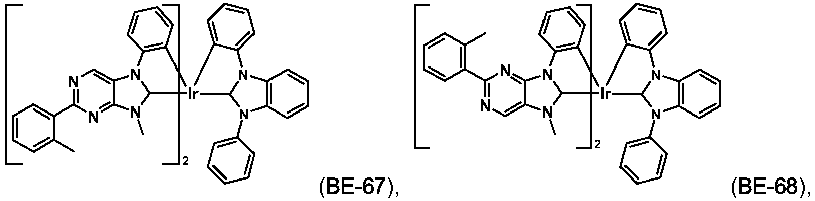

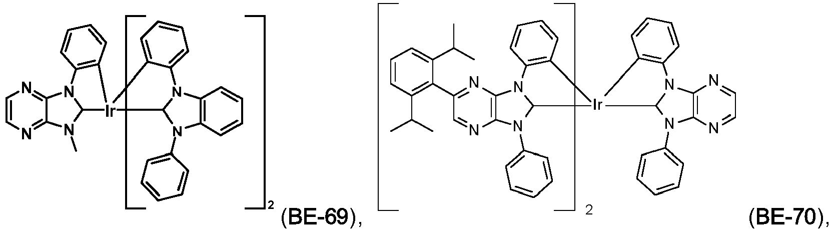

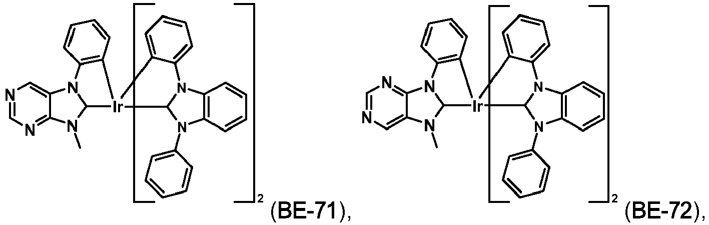

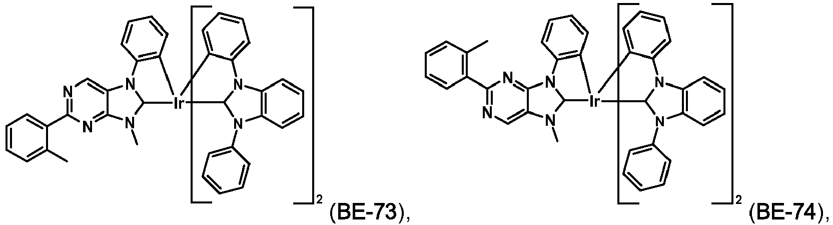

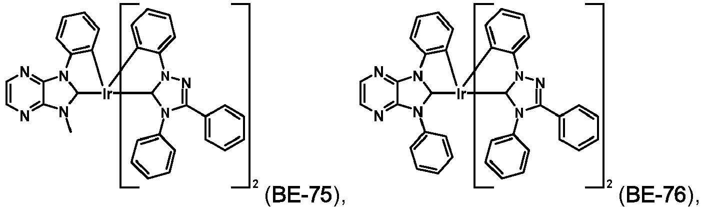

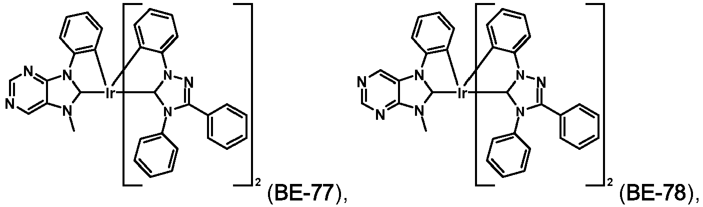

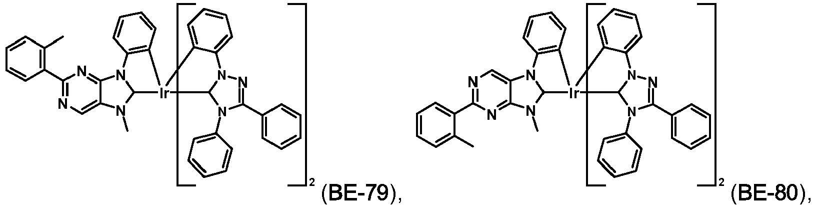

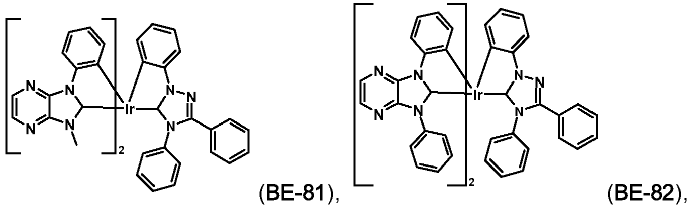

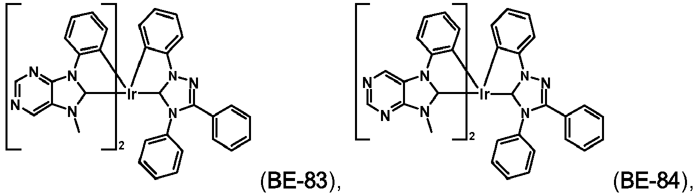

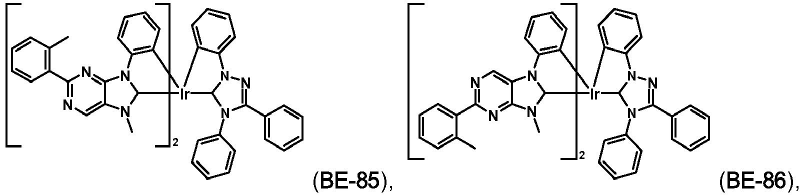

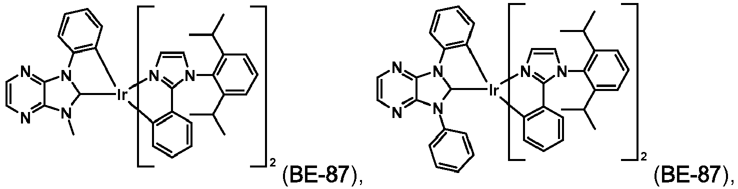

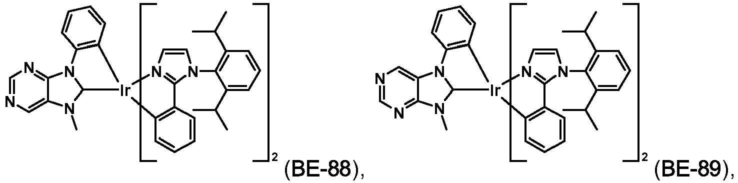

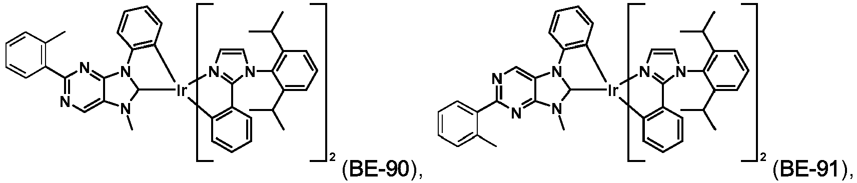

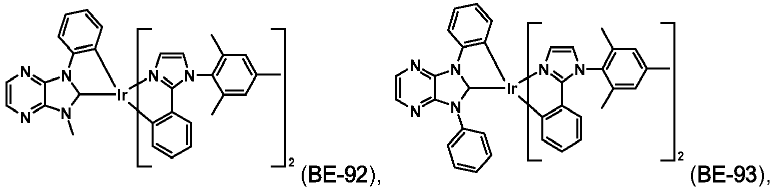

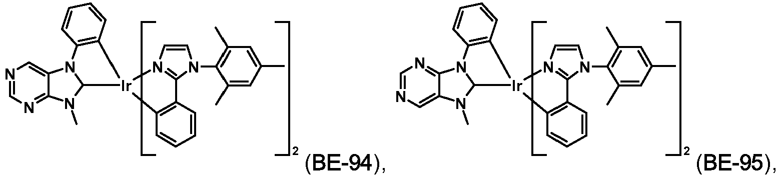

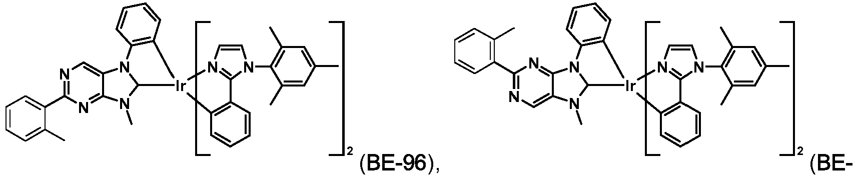

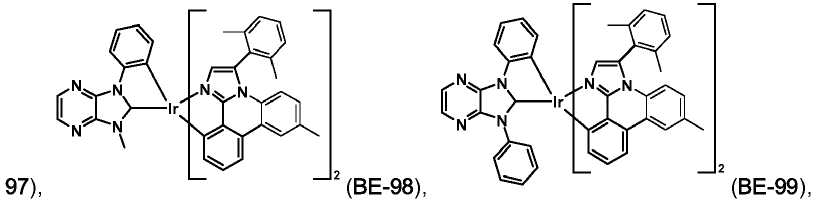

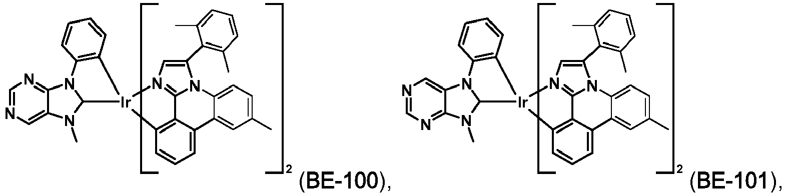

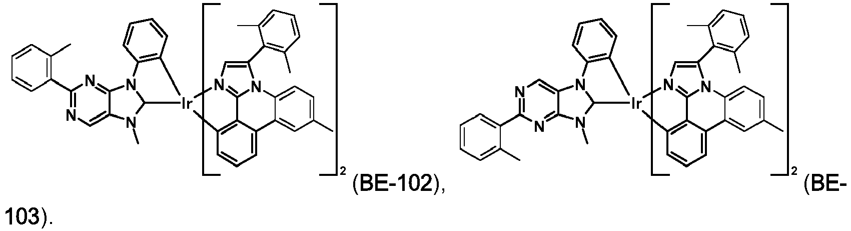

Further suitable non-carbene emitter materials are mentioned below:

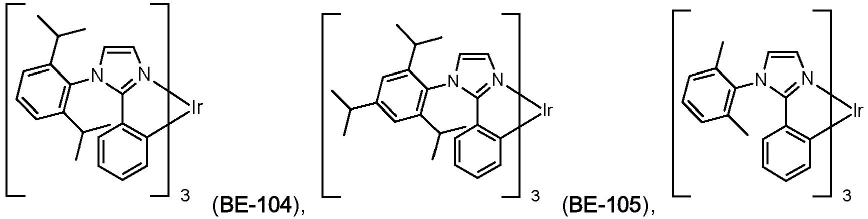

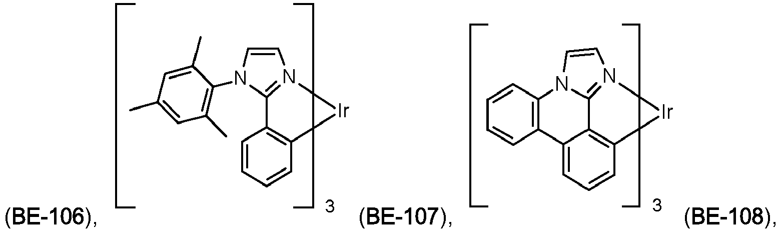

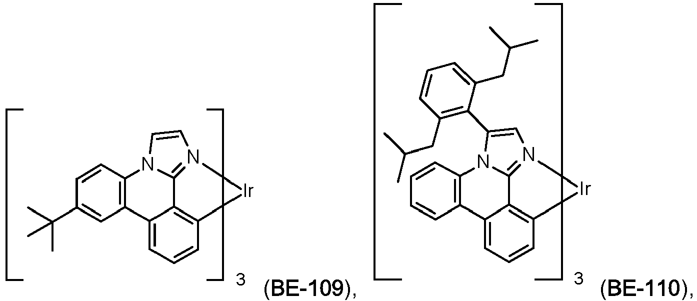

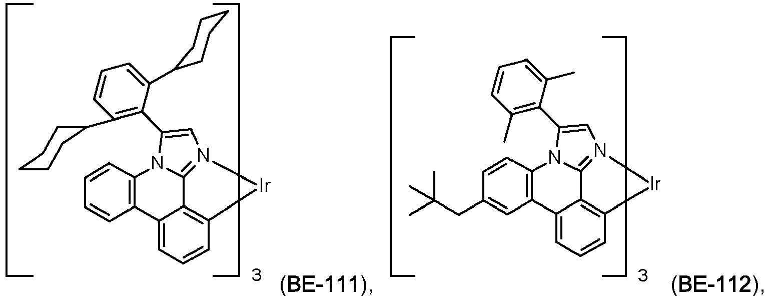

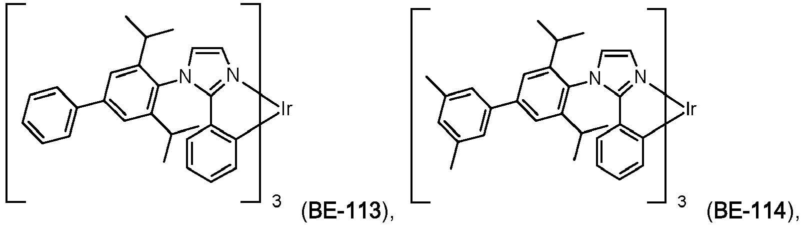

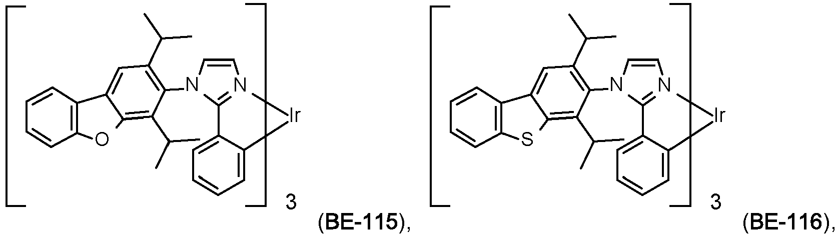

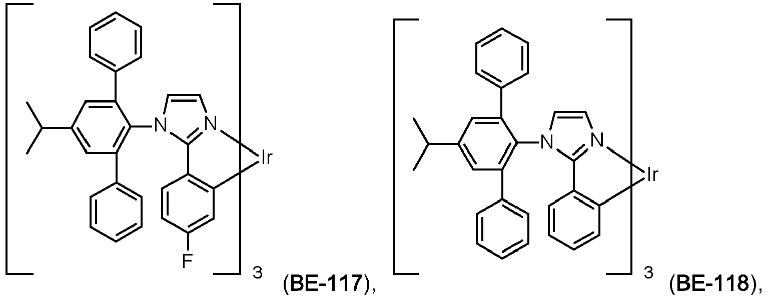

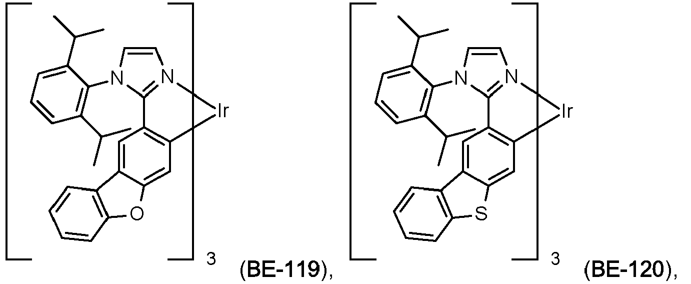

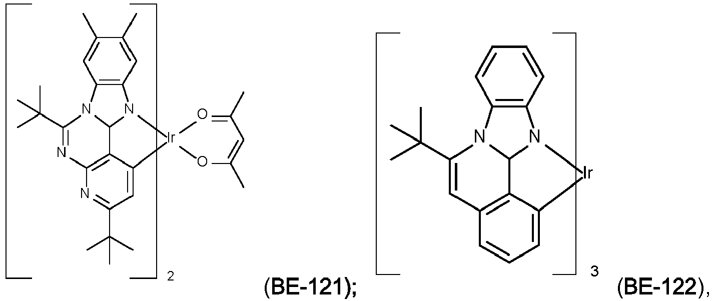

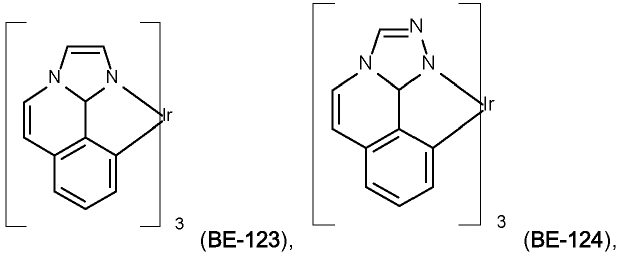

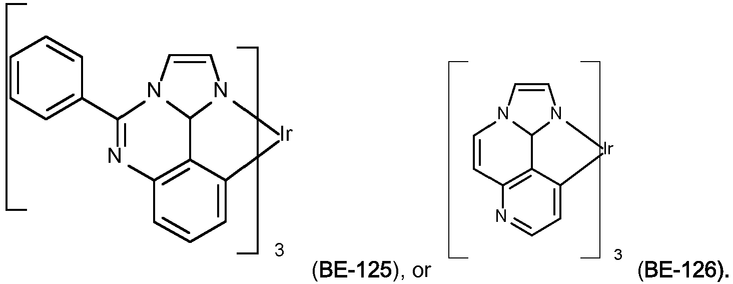

The compound of formula **IX** is more preferably a compound (**BE-1**), (**BE-2**), (**BE-7**), (**BE-12**), (**BE-16**), (**BE-64**), or (**BE-70**). The most preferred phosphorescent blue emitters are compounds (**BE-1**) and (**BE-12**).

The homoleptic metal-carbene complexes may be present in the form of facial or meridional isomers, preference being given to the facial isomers.

Suitable carbene complexes of formula (**IX**) and their preparation process are, for example, described in WO2011/073149.

The compounds of the present invention can also be used as host for phosphorescent green emitters. Suitable phosphorescent green emitters are, for example, specified in the following publications: WO2006014599, WO20080220265, WO2009073245, WO2010027583, WO2010028151, US20110227049, WO2011090535, WO2012/08881, WO20100056669, WO20100118029, WO20100244004, WO2011109042, WO2012166608, US20120292600, EP2551933A1; US6687266, US20070190359, US20070190359, US20060008670; WO2006098460, US20110210316, WO2012053627; US6921915, US20090039776; and JP2007123392.

Examples of suitable phoshorescent green emitters are shown below:

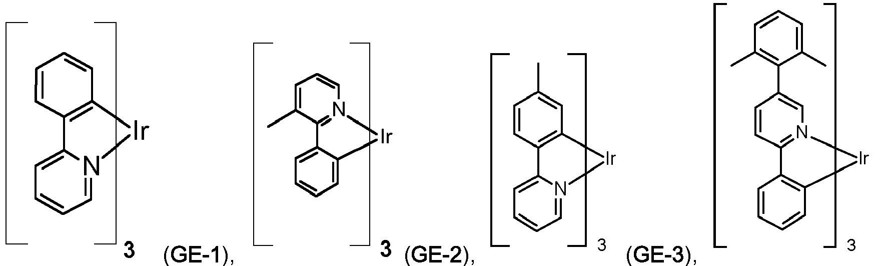

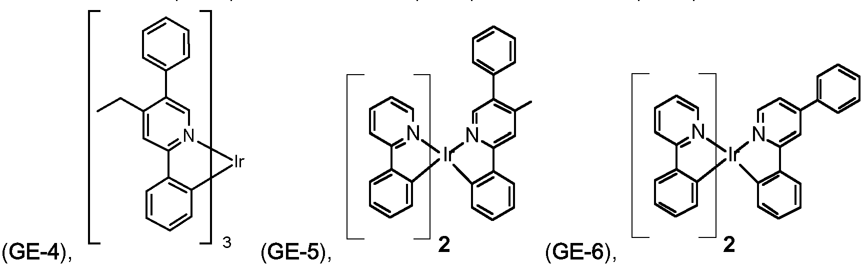

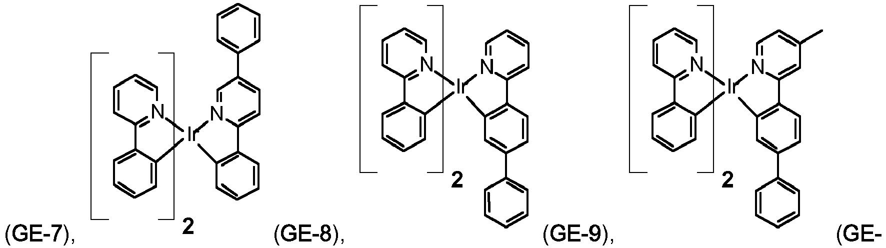

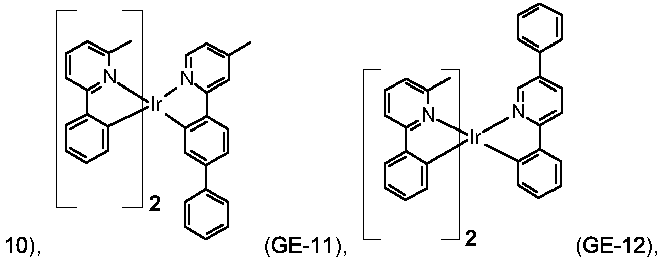

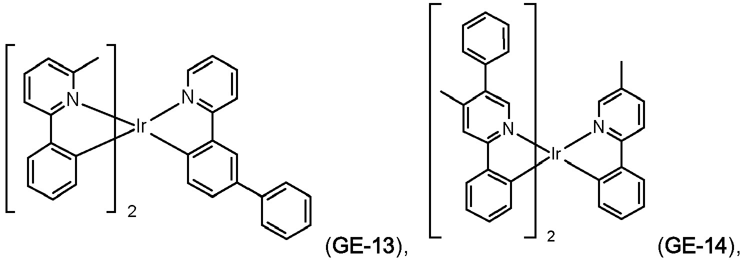

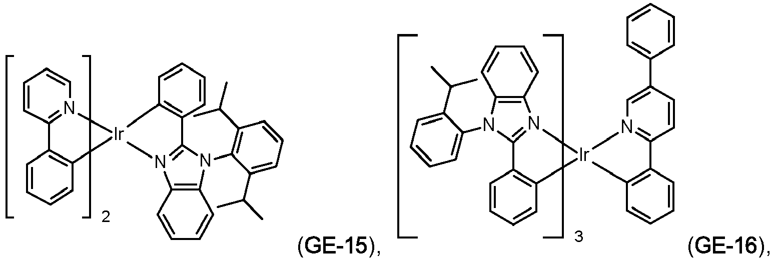

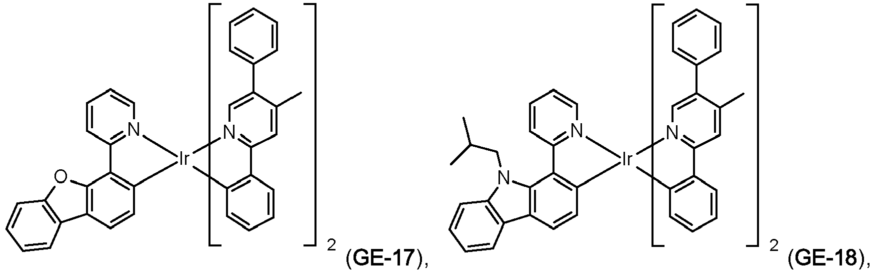

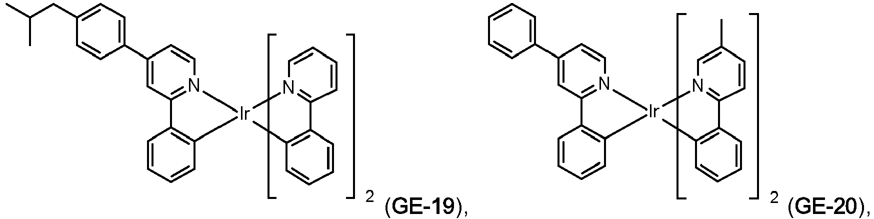

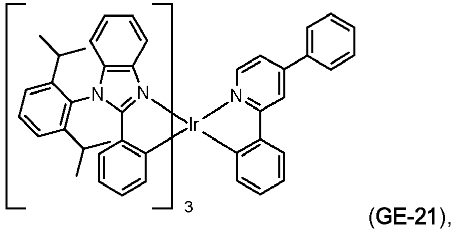

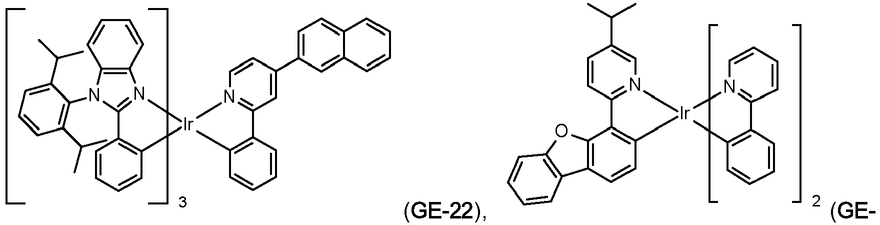

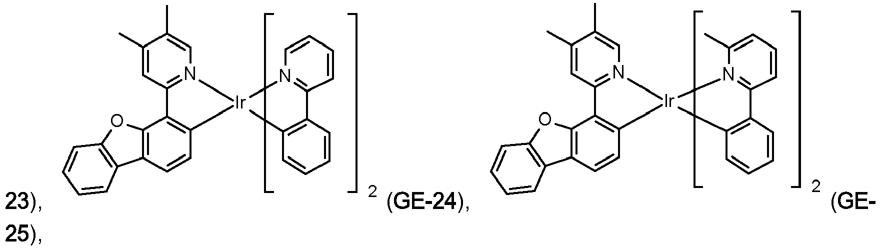

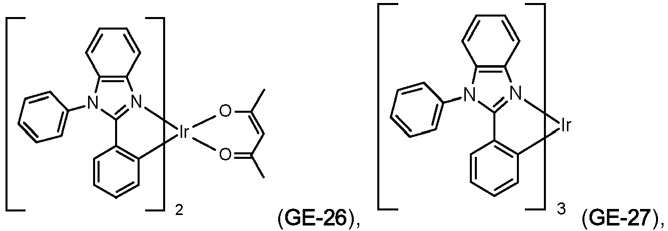

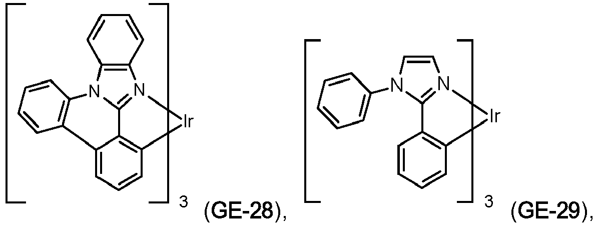

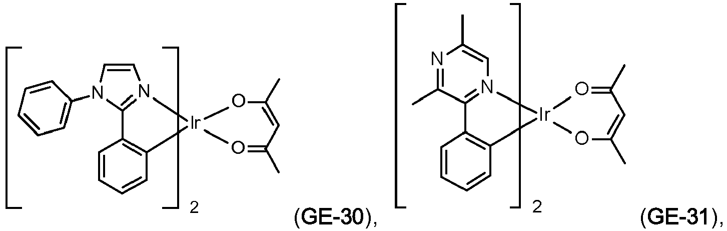

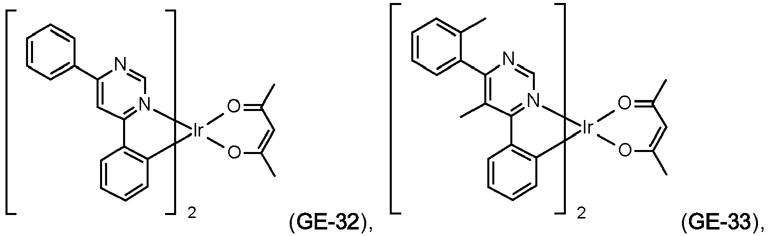

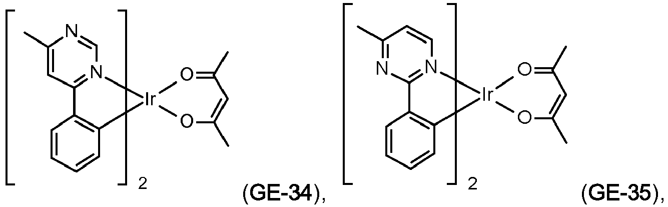

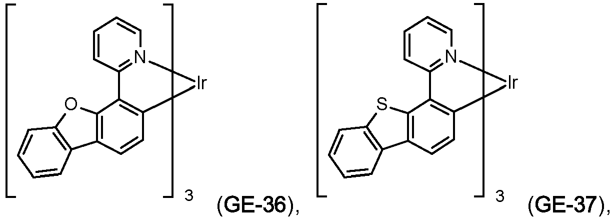

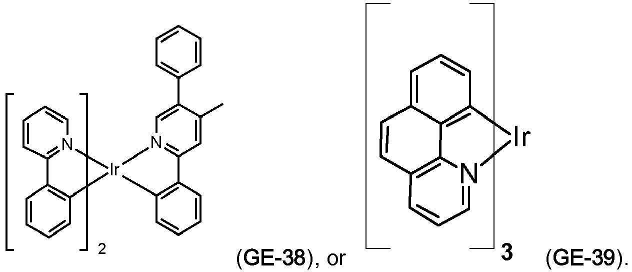

### Host (matrix) materials

The light-emitting layer may comprise further components in addition to the emitter material. For example, a fluroescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA.

In another preferred embodiment of the present invention, at least one compound of the formula **I**, especially a compound of the formula (**Ia**), very especially a compound of the formula (**Ia-2**), is used as matrix material. Examples of preferred compounds of formula **I** are compounds **C-1** to **C-191** shown above. Compounds **C-1** to **C-174** are particularly preferred.
In a preferred embodiment, the light-emitting layer is formed from 2 to 40% by weight, preferably 5 to 35% by weight, of at least one of the aforementioned emitter materials and 60 to 98% by weight, preferably 75 to 95% by weight, of at least one of the aforementioned matrix materials - in one embodiment at least one compound of the formula I, such as, for example, compound **C-1**, or **C-5** - where the sum total of the emitter material and of the matrix material adds up to 100% by weight.

Suitable metal complexes for use together with the compounds of the formula I as matrix material in OLEDs are, for example, also carbene complexes as described in WO 2005/019373 A2, WO 2006/056418 A2, WO 2005/113704, WO 2007/115970, WO 2007/115981 and WO 2008/000727.

Further suitable host materials, which may be small molecules or (co)polymers of the small molecules mentioned, are specified in the following publications: WO2007108459 (H-1 to H-37), preferably H-20 to H-22 and H-32 to H-37, most preferably H-20, H-32, H-36, H-37, WO2008035571 A1 (Host 1 to Host 6), JP2010135467 (compounds 1 to 46 and Host-1 to Host-39 and Host-43), WO2009008100 compounds No.1 to No.67, preferably No.3, No.4, No.7 to No. 12, No.55, No.59, No. 63 to No.67, more preferably No. 4, No. 8 to No. 12, No. 55, No. 59, No.64, No.65, and No. 67, WO2009008099 compounds No. 1 to No. 110, WO2008140114 compounds 1-1 to 1-50, WO2008090912 compounds OC-7 to OC-36 and the polymers of Mo-42 to Mo-51, JP2008084913 H-1 to H-70, WO2007077810 compounds 1 to 44, preferably 1, 2, 4-6, 8, 19-22, 26, 28-30, 32, 36, 39-44, WO201001830 the polymers of monomers 1-1 to 1-9, preferably of 1-3, 1-7, and 1-9, WO2008029729 the (polymers of) compounds 1-1 to 1-36, WO20100443342 HS-1 to HS-101 and BH-1 to BH-17, preferably BH-1 to BH-17, JP2009182298 the (co)polymers based on the monomers 1 to 75, JP2009170764, JP2009135183 the (co)polymers based on the monomers 1-14, WO2009063757 preferably the (co)polymers based on the monomers 1-1 to 1-26, WO2008146838 the compounds a-1 to a-43 and 1-1 to 1-46, JP2008207520 the (co)polymers based on the monomers 1-1 to 1-26, JP2008066569 the (co)polymers based on the monomers 1-1 to 1-16, WO2008029652 the (co)polymers based on the monomers 1-1 to 1-52, WO2007114244 the (co)polymers based on the monomers 1-1 to 1-18, JP2010040830 the compounds HA-1 to HA-20, HB-1 to HB-16, HC-1 to HC-23 and the (co)polymers based on the monomers HD-1 to HD-12, JP2009021336, WO2010090077 the compounds 1 to 55, WO2010079678 the compounds H1 to H42, WO2010067746, WO2010044342 the compounds HS-1 to HS-101 and Poly-1 to Poly-4, JP2010114180 the compounds PH-1 to PH-36, US2009284138 the compounds 1 to 111 and H1 to H71, WO2008072596 the compounds 1 to 45, JP2010021336 the compounds H-1 to H-38, preferably H-1, WO2010004877 the compounds H-1 to H-60, JP2009267255 the compounds 1-1 to 1-105, WO2009104488 the compounds 1-1 to 1-38, WO2009086028, US2009153034, US2009134784, WO2009084413 the compounds 2-1 to 2-56, JP2009114369 the compounds 2-1 to 2-40, JP2009114370 the compounds 1 to 67, WO2009060742 the compounds 2-1 to 2-56, WO2009060757 the compounds 1-1 to 1-76, WO2009060780 the compounds 1-1 to 1-70, WO2009060779 the compounds 1-1 to 1-42, WO2008156105 the compounds 1 to 54, JP2009059767 the compounds 1 to 20, JP2008074939 the compounds 1 to 256, JP2008021687 the compounds 1 to 50, WO2007119816 the compounds 1 to 37, WO2010087222 the compounds H-1 to H-31, WO2010095564 the compounds HOST-1 to HOST-61, WO2007108362, WO2009003898, WO2009003919, WO2010040777, US2007224446, WO06128800, WO2012014621, WO2012105310, WO2012/130709 and European patent applications EP12175635.7 and EP12185230.5. and EP12191408.9 (in particular page 25 to 29 of EP12191408.9).

The above-mentioned small molecules are more preferred than the above-mentioned (co)polymers of the small molecules.

Further suitable second host materials, are described in WO2011137072 (for example, 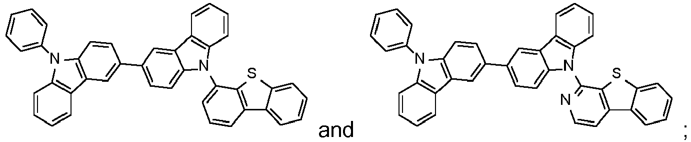 best results are achieved if said compounds are combined with 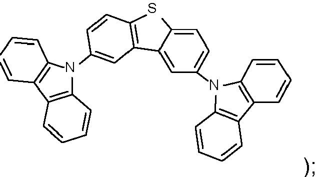 WO2012048266 (for example, 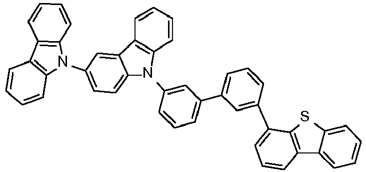 and 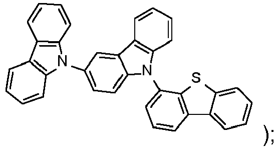 WO2012162325 (for example, 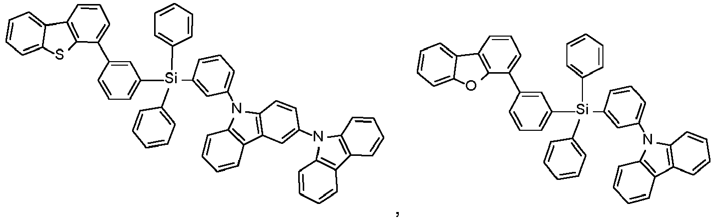 and 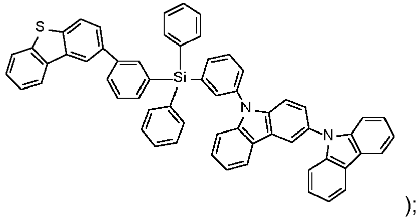 and EP2551932 (for example,

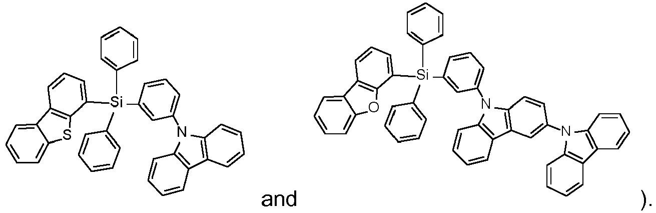

In a particularly preferred embodiment, one or more compounds of the general formula (X) specified hereinafter are used as second host material. 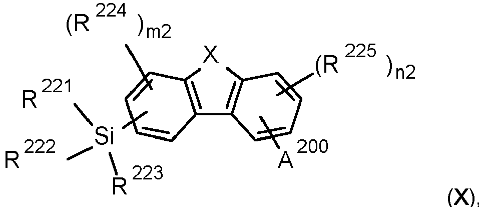 wherein
X is NR, S, O or PR;
R is aryl, heteroaryl, alkyl, cycloalkyl, or heterocycloalkyl;
A²⁰⁰ is -NR²⁰⁶R²⁰⁷, -P(O)R²⁰⁸R²⁰⁹, -PR²¹⁰R²¹¹, -S(O)₂R²¹², -S(O)R²¹³, -SR²¹⁴, or -OR²¹⁵; R²²¹, R²²² and R²²³ are independently of each other aryl, heteroaryl, alkyl, cycloalkyl, or heterocycloalkyl, wherein at least on of the groups R²²¹, R²²², or R²²³ is aryl, or heteroaryl; R²²⁴ and R²²⁵ are independently of each other alkyl, cycloalkyl, heterocycloalkyl, aryl, heteroaryl, a group A²⁰⁰, or a group having donor, or acceptor characteristics;
n2 and m2 are independently of each other 0, 1, 2, or 3;
R²⁰⁶ and R²⁰⁷ form together with the nitrogen atom a cyclic residue having 3 to 10 ring atoms, which can be unsubstituted, or which can be substituted with one, or more substituents selected from alkyl, cycloalkyl, heterocycloalkyl, aryl, heteroaryl and a group having donor, or acceptor characteristics; and/or which can be annulated with one, or more further cyclic residues having 3 to 10 ring atoms, wherein the annulated residues can be unsubstituted, or can be substituted with one, or more substituents selected from alkyl, cycloalkyl, heterocycloalkyl, aryl, heteroaryl and a group having donor, or acceptor characteristics; and R²⁰⁸, R²⁰⁹, R²¹⁰, R²¹¹, R²¹², R²¹³ R²¹⁴ und R²¹⁵ are independently of each other aryl, heteroaryl, alkyl, cycloalkyl, or heterocycloalkyl. Compounds of formula X, such as, for example, 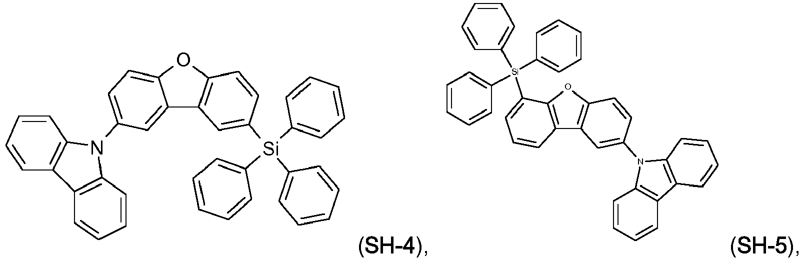 or 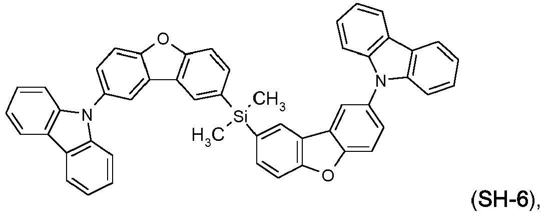 are described in WO2010079051 (in particular pages on 19 to 26 and in tables on pages 27 to 34, pages 35 to 37 and pages 42 to 43).

Additional host materials on basis of dibenzofurane are, for example, described in US2009066226, EP1885818B1, EP1970976, EP1998388, EP2034538 and European patent application no. 14160197.1. Examples of particularly preferred host materials are shown below:

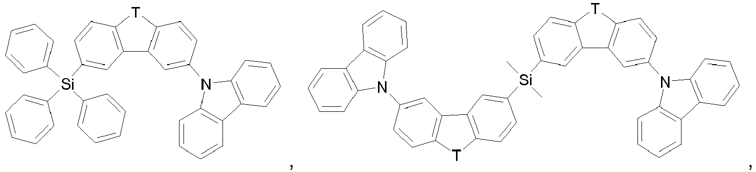

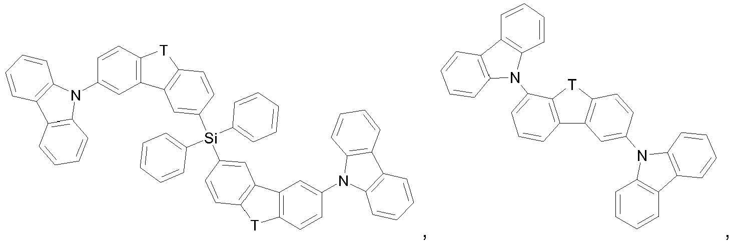

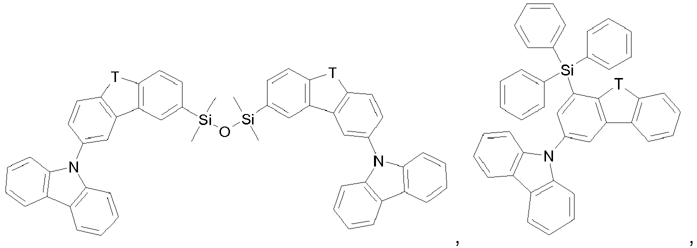

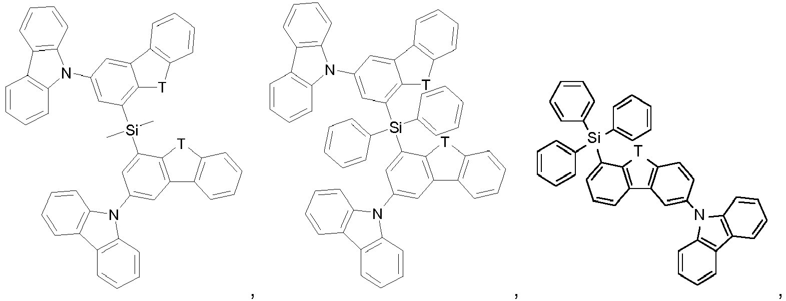

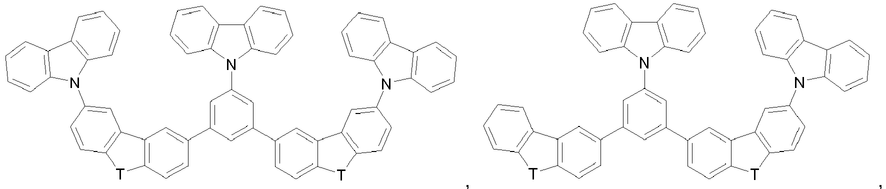

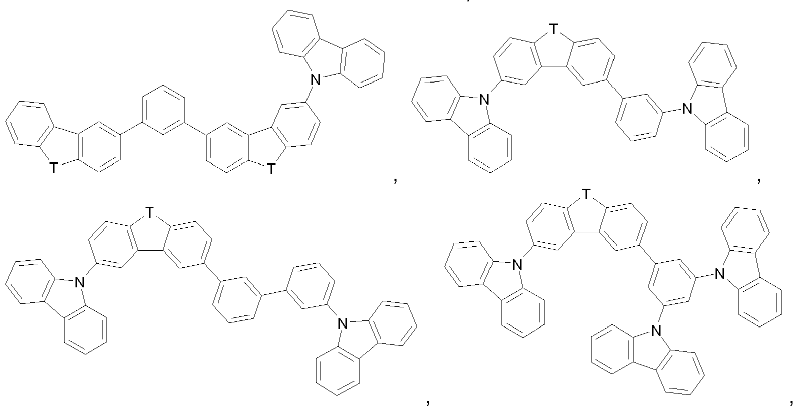

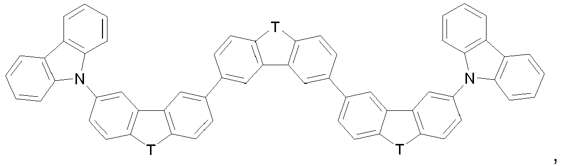

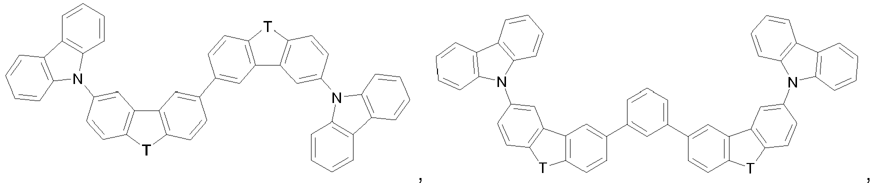

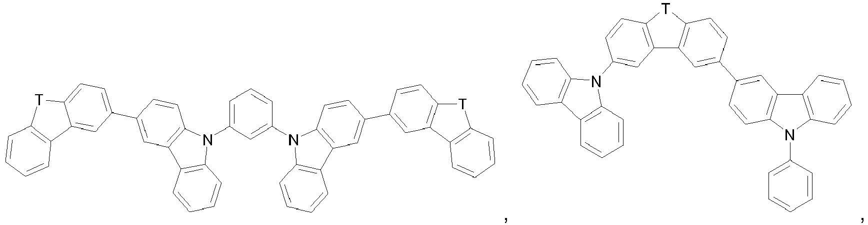

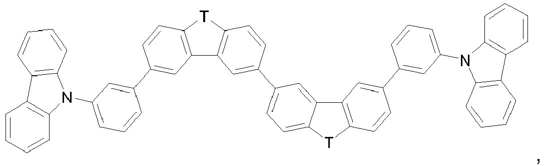

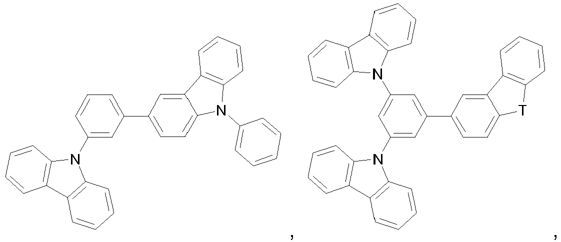

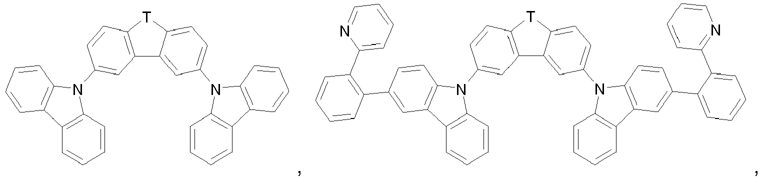

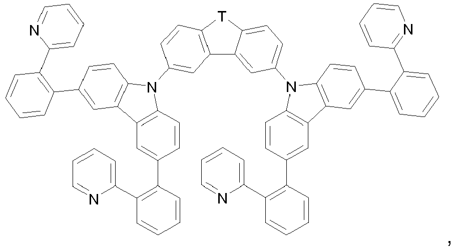

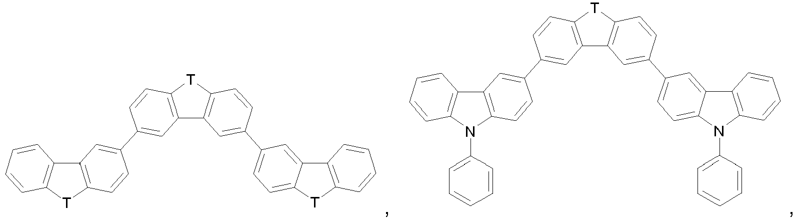

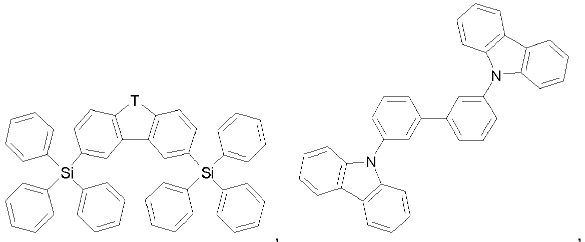

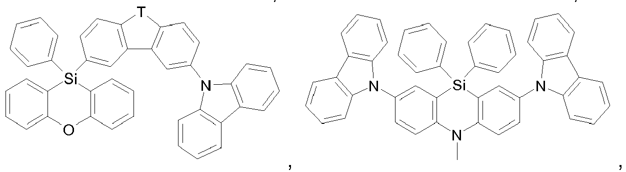

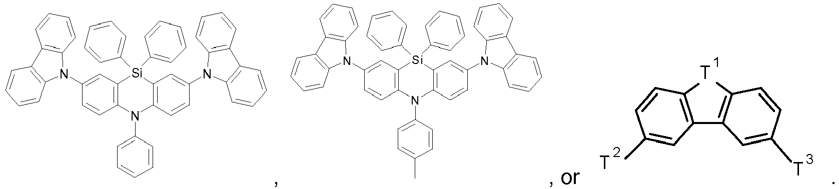

In the above-mentioned compounds T is O, or S, preferably O. If T occurs more than one time in a molecule, all groups T have the same meaning. T¹ is O, or S, preferably O. T¹ and T² are independently of each other 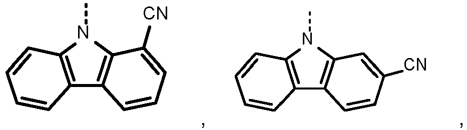 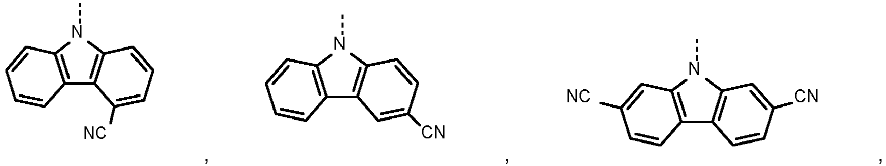 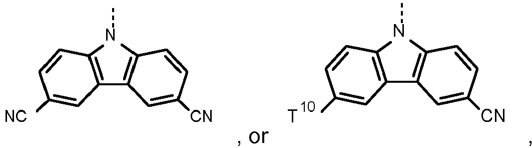 wherein T¹⁰ is a C₁-C₂₅alkyl group. 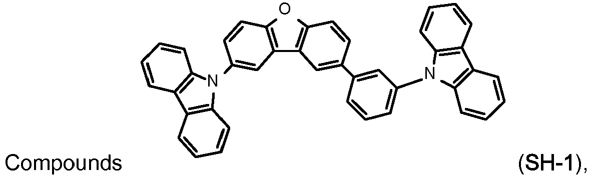 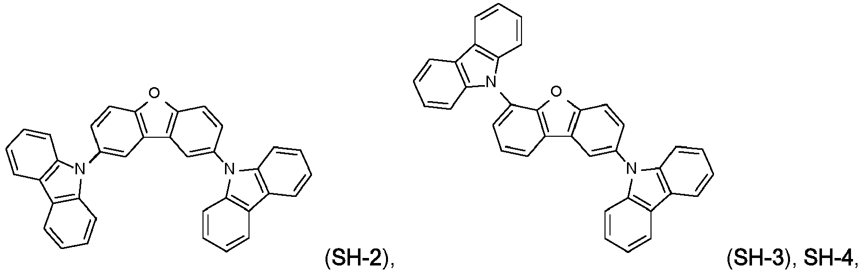 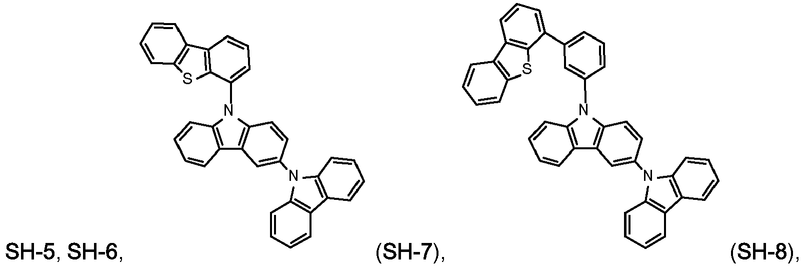 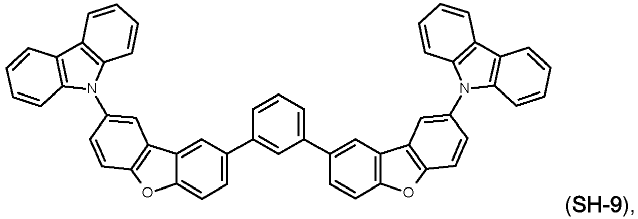 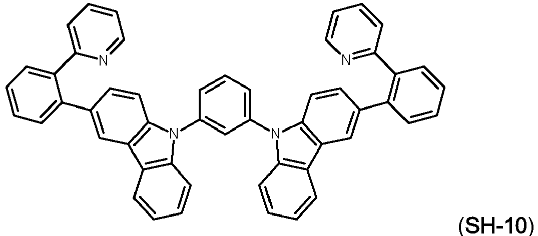 and 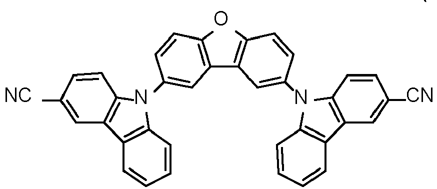 (**SH-11**) are most preferred.

### Hole/exciton blocking layer (f):

Blocking layers may be used to reduce the number of charge carriers (electrons or holes) and/or excitons that leave the emissive layer. The hole blocking layer may be disposed between the emitting layer (e) and electron transport layer (g), to block holes from leaving layer (e) in the direction of electron transport layer (g). Blocking layers may also be used to block excitons from diffusing out of the emissive layer.

Additional hole blocker materials typically used in OLEDs are 2,6-bis(N-carbazolyl)pyridine (mCPy), 2,9-dimethyl-4,7-diphenyl-1,10-phenanthroline (bathocuproin, (BCP)), bis(2-methyl-8-quinolinato)-4-phenylphenylato)aluminum(III) (BAlq), phenothiazine *S*,*S*-dioxide derivates and 1,3,5-tris(N-phenyl-2-benzylimidazolyl)benzene) (TPBI), TPBI also being suitable as electron-transport material. Further suitable hole blockers and/or electron conductor materials are 2,2',2"-(1,3,5-benzenetriyl)tris(1-phenyl-1-H-benzimidazole), 2-(4-biphenylyl)-5-(4-tert-butylphenyl)-1,3,4-oxadiazole, 8-hydroxyquinolinolatolithium, 4-(naphthalen-1-yl)-3,5-diphenyl-4H-1,2,4-triazole, 1,3-bis[2-(2,2'-bipyridin-6-yl)-1,3,4-oxadiazo-5-yl]benzene, 4,7-diphenyl-1,10-phenanthroline, 3-(4-biphenylyl)-4-phenyl-5-tert-butylphenyl-1,2,4-triazole, 6,6'-bis[5-(biphenyl-4-yl)-1,3,4-oxadiazo-2-yl]-2,2'-bipyridyl, 2-phenyl-9,10-di(naphthalene-2-yl)anthracene, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, 2-(naphthalene-2-yl)-4,7-diphenyl-1,10-phenanthroline, tris(2,4,6-trimethyl-3-(pyridin-3-yl)phenyl)borane, 2,9-bis(naphthalene-2-yl)-4,7-diphenyl-1,10-phenanthroline, 1-methyl-2-(4-(naphthalene-2-yl)phenyl)-1H-imidazo[4,5-f][1,10]phenanthroline. In a further embodiment, it is possible to use compounds which comprise aromatic or heteroaromatic rings joined via groups comprising carbonyl groups, as disclosed in WO2006/100298, disilyl compounds selected from the group consisting of disilylcarbazoles, disilylbenzofurans, disilylbenzothiophenes, disilylbenzophospholes, disilylbenzothiophene S-oxides and disilylbenzothiophene S,S-dioxides, as specified, for example, in PCT applications WO2009/003919 and WO2009003898 and disilyl compounds as disclosed in WO2008/034758, as a blocking layer for holes/excitons (f).

In another preferred embodiment compounds (**SH-1**), (**SH-2**), (**SH-3**), **SH-4**, **SH-5**, **SH-6**, (**SH-7**), (**SH-8**), (**SH-9**), (**SH-10**) and (**SH-11**) may be used as hole/exciton blocking materials.

### Electron transport layer (g):

Electron transport layer may include a material capable of transporting electrons. Electron transport layer may be intrinsic (undoped), or doped. Doping may be used to enhance conductivity. Suitable electron-transporting materials for layer (g) of the inventive OLEDs comprise metals chelated with oxinoid compounds, such as tris(8-hydroxyquinolato)aluminum (Alq₃), compounds based on phenanthroline such as 2,9-dimethyl-4,7-diphenyl-1,10-phenanthroline (DDPA = BCP), 4,7-diphenyl-1,10-phenanthroline (Bphen), 2,4,7,9-tetraphenyl-1,10-phenanthroline, 4,7-diphenyl-1,10-phenanthroline (DPA) or phenanthroline derivatives disclosed in EP1786050, in EP1970371, or in EP1097981, and azole compounds such as 2-(4-biphenylyl)-5-(4-t-butylphenyl)-1,3,4-oxadiazole (PBD) and 3-(4-biphenylyl)-4phenyl-5-(4-t-butylphenyl)-1,2,4-triazole (TAZ).

It is likewise possible to use mixtures of at least two materials in the electron-transporting layer, in which case at least one material is electron-conducting. Preferably, in such mixed electron-transport layers, at least one phenanthroline compound is used, preferably BCP, or at least one pyridine compound according to the formula (**VIII**) below, preferably a compound of the formula (**VIIIaa**) below. More preferably, in mixed electron-transport layers, in addition to at least one phenanthroline compound, alkaline earth metal or alkali metal hydroxyquinolate complexes, for example Liq, are used. Suitable alkaline earth metal or alkali metal hydroxyquinolate complexes are specified below (**formula VII**). Reference is made to WO2011/157779.

The electron-transport layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo, Appl. Phys. Lett., Vol. 82, No. 25, 23 June 2003 and Pfeiffer et al., Organic Electronics 2003, 4, 89 - 103 and K. Walzer, B. Maennig, M. Pfeiffer, K. Leo, Chem. Soc. Rev. 2007, 107, 1233. For example, it is possible to use mixtures which lead to electrical n-doping of the electron-transport layer. n-Doping is achieved by the addition of reducing materials. These mixtures may, for example, be mixtures of the abovementioned electron transport materials with alkali/alkaline earth metals or alkali/alkaline earth metal salts, for example Li, Cs, Ca, Sr, CS₂CO₃, with alkali metal complexes, for example 8-hydroxyquinolatolithium (Liq), and with Y, Ce, Sm, Gd, Tb, Er, Tm, Yb, Li₃N, Rb₂CO₃, dipotassium phthalate, W(hpp)₄ from EP1786050, or with compounds described in EP1837926B1, EP1837927, EP2246862 and WO2010132236.

In a preferred embodiment, the electron-transport layer comprises at least one compound of the general formula (**VII**) 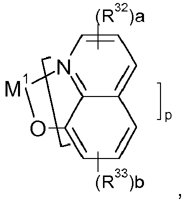 in which
R³² and R³³ are each independently F, C₁-C₈-alkyl, or C₆-C₁₄-aryl, which is optionally substituted by one or more C₁-C₈-alkyl groups, or
two R³² and/or R³³ substituents together form a fused benzene ring which is optionally substituted by one or more C₁-C₈-alkyl groups;
a and b are each independently 0, or 1, 2 or 3,
M¹ is an alkaline metal atom or alkaline earth metal atom,
p is 1 when M¹ is an alkali metal atom, p is 2 when M¹ is an earth alkali metal atom.

A very particularly preferred compound of the formula (**VII**) is 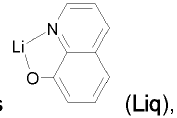 which may be present as a single species, or in other forms such as Li_{g}Q_{g} in which g is an integer, for example Li₆Q₆. Q is an 8-hydroxyquinolate ligand or an 8-hydroxyquinolate derivative.

In a further preferred embodiment, the electron-transport layer comprises at least one compound of the formula (**VIII**), 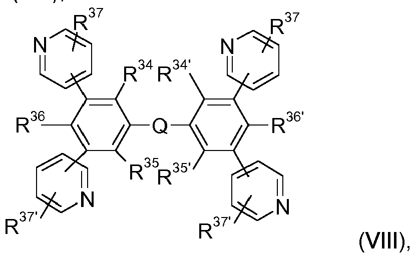 in which
R³⁴, R³⁵, R³⁶, R³⁷, R^{34'}, R^{35'}, R^{36'} and R^{37'}are each independently H, C₁-C₁₈-alkyl, C₁-C₁₈-alkyl which is substituted by E and/or interrupted by D, C₆-C₂₄-aryl, C₆-C₂₄-aryl which is substituted by G, C₂-C₂₀-heteroaryl or C₂-C₂₀-heteroaryl which is substituted by G,
Q is an arylene or heteroarylene group, each of which is optionally substituted by G;
D is -CO-; -COO-; -S-; -SO-; -SO₂-; -O-; -NR⁴⁰-; -SiR⁴⁵R⁴⁶-; -POR⁴⁷-; -CR³⁸=CR³⁹-; or-C≡C-;
E is -OR⁴⁴; -SR⁴⁴; -NR⁴⁰R⁴¹; -COR⁴³; -COOR⁴²; -CONR⁴⁰R⁴¹; -CN; or F;
G is E, C₁-C₁₈-alkyl, C₁-C₁₈-alkyl which is interrupted by D , C₁-C₁₈-perfluoroalkyl, C₁-C₁₈-alkoxy, or C₁-C₁₈-alkoxy which is substituted by E and/or interrupted by D,
   in which
R³⁸ and R³⁹ are each independently H, C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-;
R⁴⁰ and R⁴¹ are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-; or R⁴⁰ and R⁴¹ together form a 6-membered ring;
R⁴² and R⁴³ are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R⁴⁴ is C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R⁴⁵ and R⁴⁶ are each independently C₁-C₁₈-alkyl, C₆-C₁₈-aryl or C₆-C₁₈-aryl which is substituted by C₁-C₁₈-alkyl,
R⁴⁷ is C₁-C₁₈-alkyl, C₆-C₁₈-aryl or C₆-C₁₈-aryl which is substituted by C₁-C₁₈-alkyl.

Preferred compounds of the formula (**VIII**) are compounds of the formula (**VIIIa**) 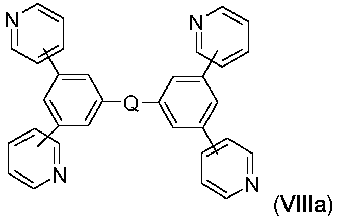 in which Q is: 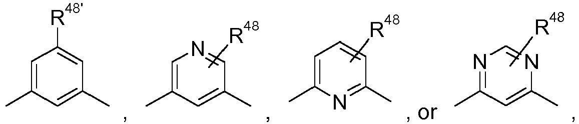 R⁴⁸ is H or C₁-C₁₈-alkyl and R^{48'} is H, C₁-C₁₈-alkyl or

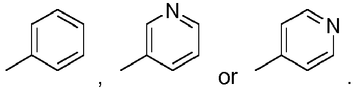

Particular preference is given to a compound of the formula

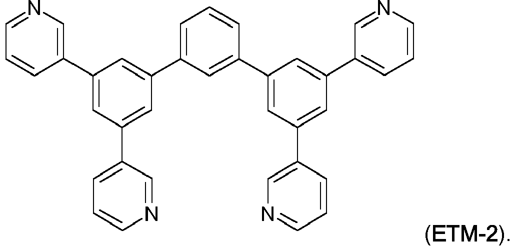

In a further, very particularly preferred embodiment, the electron-transport layer comprises a compound **Liq** and a compound **ETM-2**.

In a preferred embodiment, the electron-transport layer comprises the compound of the formula (**VII**) in an amount of 99 to 1% by weight, preferably 75 to 25% by weight, more preferably about 50% by weight, where the amount of the compounds of the formulae (**VII**) and the amount of the compounds of the formulae (**VIII**) adds up to a total of 100% by weight.

The preparation of the compounds of the formula (**VIII**) is described in J. Kido et al., Chem. Commun. (2008) 5821-5823, J. Kido et al., Chem. Mater. 20 (2008) 5951-5953 and JP2008/127326, or the compounds can be prepared analogously to the processes disclosed in the aforementioned documents.

It is likewise possible to use mixtures of alkali metal hydroxyquinolate complexes, preferably Liq, and dibenzofuran compounds in the electron-transport layer. Reference is made to WO2011/157790. Dibenzofuran compounds **A-1** to **A-36** and **B-1** to **B-22** described in WO2011/157790 are preferred, wherein dibenzofuran compound 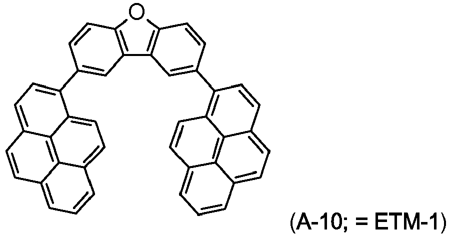 is most preferred.

In a preferred embodiment, the electron-transport layer comprises **Liq** in an amount of 99 to 1% by weight, preferably 75 to 25% by weight, more preferably about 50% by weight, where the amount of **Liq** and the amount of the dibenzofuran compound(s), especially **ETM-1**, adds up to a total of 100% by weight.

In a preferred embodiment, the electron-transport layer comprises at least one phenanthroline derivative and/or pyridine derivative.

In a further preferred embodiment, the electron-transport layer comprises at least one phenanthroline derivative and/or pyridine derivative and at least one alkali metal hydroxyquinolate complex.

In a further preferred embodiment, the electron-transport layer comprises at least one of the dibenzofuran compounds **A-1** to **A-36** and **B-1** to **B-22** described in WO2011/157790, especially **ETM-1**.

In a further preferred embodiment, the electron-transport layer comprises a compound described in WO2012/111462, WO2012/147397, WO2012014621, such as, for example, a compound of formula 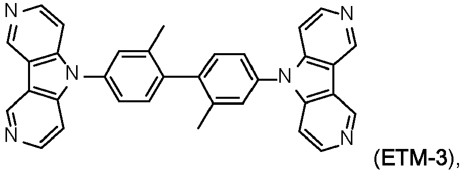 US2012/0261654 , such as, for example, a compound of formula 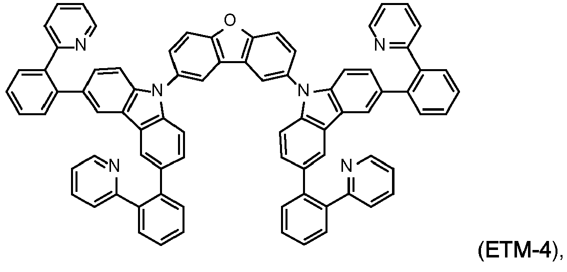 and WO2012/115034, such as for example, such as, for example, a compound of formula

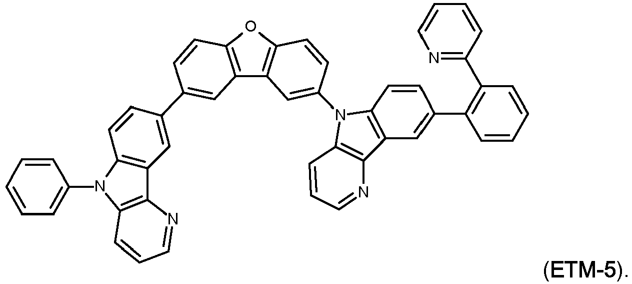

### Electron injection layer (h):

The electron injection layer may be any layer that improves the injection of electrons into an adjacent organic layer. Lithium-comprising organometallic compounds such as 8-hydroxyquinolatolithium (Liq), CsF, NaF, KF, Cs₂CO₃ or LiF may be applied between the electron transport layer (g) and the cathode (i) as an electron injection layer (h) in order to reduce the operating voltage.

### Cathode (i):

The cathode (i) is an electrode which serves to introduce electrons or negative charge carriers. The cathode may be any metal or nonmetal which has a lower work function than the anode. Suitable materials for the cathode are selected from the group consisting of alkali metals of group 1, for example Li, Cs, alkaline earth metals of group 2, metals of group 12 of the Periodic Table of the Elements, comprising the rare earth metals and the lanthanides and actinides. In addition, metals such as aluminum, indium, calcium, barium, samarium and magnesium, and combinations thereof, may be used.

In general, the different layers, if present, have the following thicknesses:
anode (a): 500 to 5000 Å (ångström), preferably 1000 to 2000 Å;
hole injection layer (b): 50 to 1000 Å, preferably 200 to 800 Å,
hole-transport layer (c): 50 to 1000 Å, preferably 100 to 800 Å,
exciton blocking layer (d): 10 to 500 Å, preferably 50 to 100 Å,
light-emitting layer (e): 10 to 1000 Å, preferably 50 to 600 Å,
hole/ exciton blocking layer (f): 10 to 500 Å, preferably 50 to 100 Å,
electron-transport layer (g): 50 to 1000 Å, preferably 200 to 800 Å,
electron injection layer (h): 10 to 500 Å, preferably 20 to 100 Å,
cathode (i): 200 to 10 000 Å, preferably 300 to 5000 Å.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive OLED can be produced by methods known to those skilled in the art. In general, the inventive OLED is produced by successive vapor deposition of the individual layers onto a suitable substrate. Suitable substrates are, for example, glass, inorganic semiconductors or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

Use of the compounds of the formula I in at least one layer of the OLED, preferably in the light-emitting layer (preferably as a matrix material), charge transport layer and/or in the charge/exciton blocking layer makes it possible to obtain OLEDs with high efficiency and with low use and operating voltage. Frequently, the OLEDs obtained by the use of the compounds of the formula I additionally have high lifetimes. The efficiency of the OLEDs can additionally be improved by optimizing the other layers of the OLEDs. For example, high-efficiency cathodes such as Ca or Ba, if appropriate in combination with an intermediate layer of LiF, can be used. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes only and do not limit the scope of the claims. Unless otherwise stated, all parts and percentages are by weight.

### Examples

### Example 1

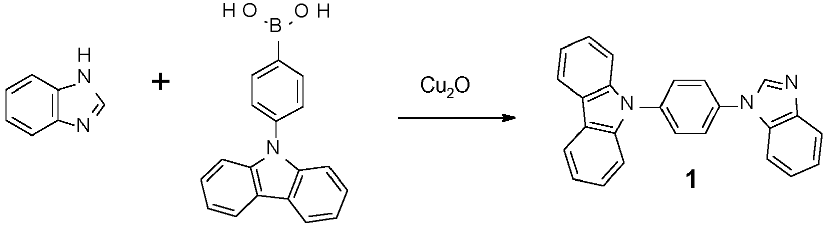

a) 10 g of benzimidazole (84.6 mmol) are combined with (4-carbazol-9-ylphenyl)boronic acid (20.3 g, 70.7 mmol), Cu₂O (1.10 g, 7.69 mmol), in MeOH (300 mL) and toluene (60 mL). The resulting mixture is stirred under an atmosphere of air at 35 °C for 30 hours. The reaction is allowed to cool to room temperature and concentrated under reduced pressure. The product is then purified by chromatography on silica using 10% MeOH in CH₂Cl₂ as eluent. The desired product **1** is isolated in quantitative yield and used without further purification. ¹H NMR (400 MHz, Chloroform-d) δ 8.15 (d, *J =* 7.8 Hz, 1H), 7.85-7.78 (m, 2H), 7.65 (s, 1H), 7.61 - 7.38 (m, 2H), 7.37 - 7.18 (m, 4H). Product was confirmed by GC-MS, m/z 359(M⁺). 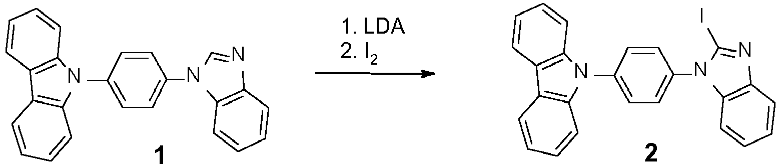
b) 30 ml of Lithium diisopropylamide (1 M in THF/hexane) are added to **1** (8.4 g, 23.4 mmol) in anhydrous THF (120 mL) at -78 °C under N₂. The reaction mixture is stirred for 1 hour at -78 °C. Then, iodine (7.14 g, 28.1 mmol) is added to the reaction mixture at -78 °C. The reaction mixture is stirred at -78 °C for 1 hour and is then warmed to 0 °C and stirred for 3 hours. The reaction mixture is then washed with saturated aqueous Na₂S₂O₃ and extracted with ethyl acetate (3X100 ml). The organic phase is then dried over anhydrous Na₂SO₄ and solvent is evaporated to give the crude product **2** as a yellow oil. The product is then purified by chromatography on silica using hexane/ethyl acetate (3/1 to 1/1) as eluent. The desired product **2** is isolated (5.14 g, 29 % yield). Product is confirmed by GC-MS, m/z 485(M⁺). 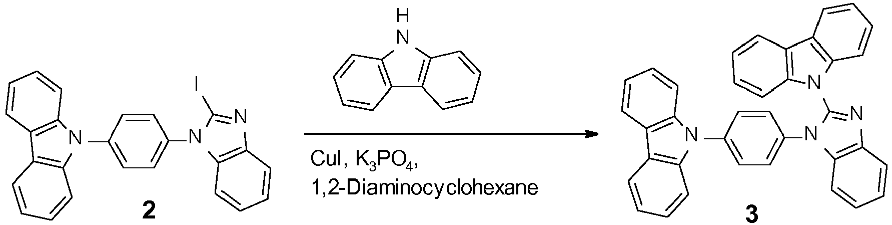
c) **2** (5.1 g, 10.5 mmol) are combined with carbazole (1.76 g, 10.5 mmol), CuI (0.020 g, 0.105 mmol), K₃PO₄ (4.68 g, 22.1 mmol) and 1,2-diaminocyclohexane (0.126 ml, 1.05 mmol) in 1,4-dioxane (60 mL) and the reaction mixture is heated at 115 °C for 30 hours. The reaction is allowed to cool to room temperature and is then filtered through a pad of celite washing well with ethyl acetate. The resulting solution is evaporated to give a crude material as a black oil. The crude material is purified by chromatography on NH-silica gel using 10 % MeOH in CH₂Cl₂ as eluent and is then recrystallized by 50 % CH₂Cl₂ in hexane to give **3** (= C-5) as a white solid (1.70 g, 31 % yield) in 99.3 % purity by HPLC. ¹H NMR (400 MHz, Chloroform-d) δ 8.16 - 7.96 (m, 5H), 7.70 - 7.63 (m, 1H), 7.55 - 7.40 (m, 4H), 7.40 - 7.22 (m, 12H), 7.04 (d, *J =* 8.2 Hz, 1H). Product confirmed by LCMS m/z = 525 (M+1).

### Example 2

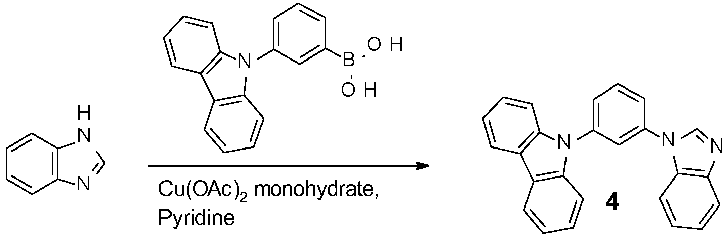

a) 6.6 g of benzimidazole (55.9 mmol) are combined with (3-carbazol-9-ylphenyl)boronic acid (32.0 g, 111 mmol), Cu(OAc)₂ monohydrate (1.02 g, 5.59 mmol), pyridine (9 ml), DMF (53 mL) and H₂O (1 ml). The resulting mixture is stirred under an atmosphere of air at 35 °C for 35 hours. The reaction is allowed to cool to room temperature, poured into 28 % aqueous NH₃ (100 ml) and then extracted with toluene (200ml x 2). The organic phase is then dried over anhydrous Na₂SO₄ and solvent is evaporated to give the crude product as a greenish-yellow oil. The product is then purified by chromatography on silica using hexane/ethyl acetate (2/1) as eluant. The desired product **4** is isolated (8.3 g, 41 % yield). ¹H NMR (400 MHz, Chloroform-d) δ 8.20 (s, 1H), 8.15 (d, *J* = 7.8 Hz, 2H), 7.96 - 7.87 (m, 1H), 7.84 - 7.74 (m, 2H), 7.69 (dd, *J* = 7.4, 2.0 Hz, 1H), 7.62 (dt, *J =* 6.2, 2.5 Hz, 2H), 7.46 (dt, *J* = 15.2, 8.1 Hz, 4H), 7.40 - 7.28 (m, 4H). 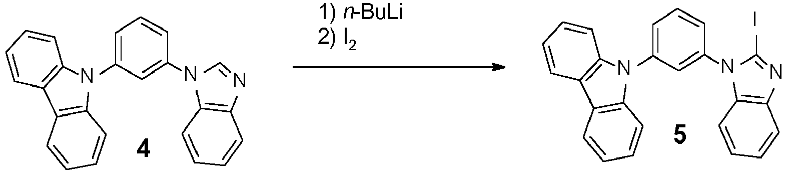
b) 17.3 ml of *n*-butyllithium (1.6 M in heptane) are added to **4** (7.4 g, 20.6 mmol) in 300 ml anhydrous THF at -78 °C under N₂. The reaction mixture is stirred for 30 min at -78 °C. Then, iodine (6.28 g, 24.7 mmol) is added to the reaction mixture at -78 °C. The reaction mixture is then stirred at -78 °C for 1 hour and is then warmed to 0 °C and stirred for 6 hours. The mixture is washed with saturated aqueous Na₂S₂O₃ and extracted with CH₂Cl₂ (3 x100 ml). The organic phase is then dried over anhydrous Na₂SO₄ and solvent is evaporated to give the crude product as a yellow oil. The product is then purified by chromatography on silica using hexane/ethyl acetate (4/1) as eluent. The desired product **5** is isolated (6 g, 60 % yield). ¹H NMR (400 MHz, Chloroform-d) δ 8.15 (d, *J* = 7.8 Hz, 2H), 7.82 (dd, *J* = 13.6, 5.4 Hz, 3H), 7.65 (s, 1H), 7.60 - 7.39 (m, 4H), 7.37 - 7.21 (m, 6H). Product was confirmed by GC-MS, m/z 485(M⁺). 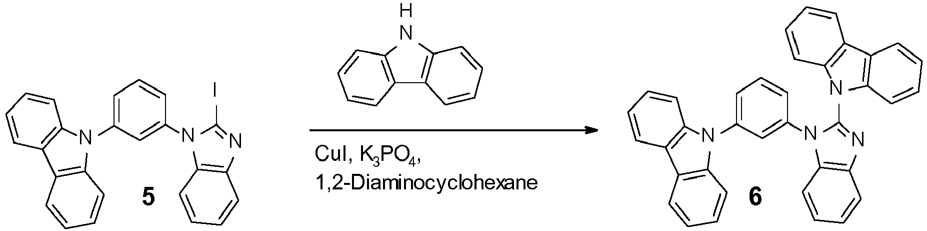
c) **5** (6 g, 12.4 mmol) is combined with carbazole (2.07 g, 12.4 mmol), CuI (0.118 g, 0.619 mmol), K₃PO₄ (5.25 g, 24.7 mmol) and 1,2-diaminocyclohexane (0.150 ml, 1.24 mmol) in 1,4-dioxane (150 mL) and the reaction mixture is heated at 115 °C for 16 hours. The reaction is allowed to cool to room temperature and is then filtered through a pad of celite washing well with CH₂Cl₂. The resulting solution is evaporated to give a crude material as a black oil. The crude material is purified by chromatography on silica gel using hexane/CH₂Cl₂ (1/1 to 2/3) as eluent to give 6 (= C-1) as a white solid (3.10 g, 48 % yield) in 99.8 % purity by HPLC. ¹H NMR (400 MHz, Chloroform-d) δ 8.17 - 8.09 (m, 2H), 8.07 - 8.01 (m, 2H), 7.99 - 7.94 (m, 1H), 7.59 (dd, *J =* 7.5, 1.6 Hz, 1H), 7.54 (t, *J =* 7.9 Hz, 1H), 7.50 - 7.17 (m, 15H), 6.81 (d, *J =* 7.7 Hz, 2H). Product confirmed by LCMS m/z = 525 (M+1).

### Example 3

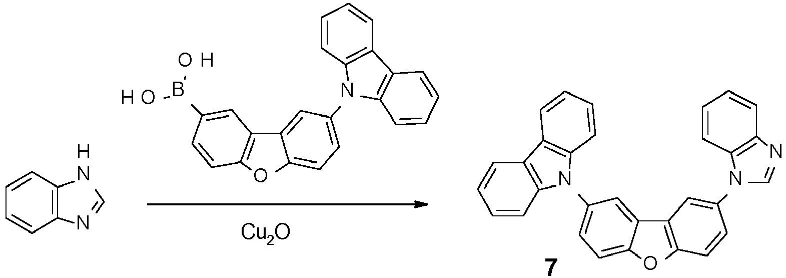

a) 10g of benzimidazole (84.6 mmol) are combined with (8-carbazol-9-yldibenzofuran-2-yl)boronic acid (20.3 g, 70.7 mmol), Cu₂O (1.33 g, 9.30 mmol), in MeOH (300 ml) and toluene (60 mL). The resulting mixture is stirred under an atmosphere of air at 35 °C for 30 hours. The reaction is allowed to cool to room temperature and concentrated under reduced pressure. The product is then purified by chromatography on neutral silica gel using 5 % MeOH in toluene as eluent. The desired product **7** is isolated (18.6 g, 73 % yield). ¹H NMR (400 MHz, Chloroform-d) δ 8.21 - 8.12 (m, 4H), 8.06 (d, *J* = 2.1 Hz, 1H), 7.87 (m, 3H), 7.68 (ddd, *J* = 21.8, 8.6, 2.2 Hz, 2H), 7.56 - 7.50 (m, 1 H), 7.45 - 7.28 (m, 8H). Product was confirmed by GC-MS 449 (M⁺). 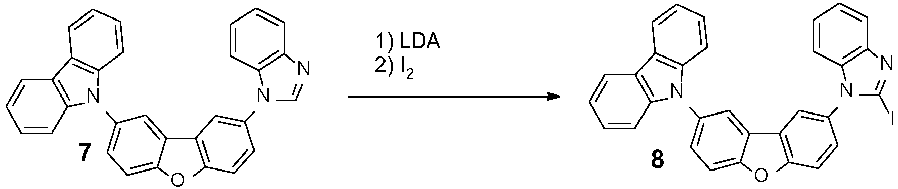
b) 20 ml of Lithium diisopropylamide 1.0 M in THF/hexane are added to **7** (7.00 g, 15.6 mmol) in 120 ml water free THF at -78 °C under N₂. The reaction mixture is stirred for 1 hour at -78 °C. Then, iodine (4.75 g, 18.7 mmol) is added to the reaction mixture at -78 °C. The reaction mixture is stirred at -78 °C for 1 hour and is then warmed to 0 °C and stirred for 3 hours. The mixture is washed by saturated aqueous Na₂S₂O₃ and extracted with ethyl acetate (3X100 ml). The organic phase is then dried over anhydrous Na₂SO₄ and solvent is evaporated to give the crude product as an orange powder. The product is then purified by chromatography on neutral silica gel using hexane/ethyl acetate (9/1 to 4/1) as eluent. The product 8 is isolated (4.60 g, 51 % yield). ¹H NMR (400 MHz, Chloroform-d) δ 8.20 - 8.13 (m, 3H), 7.98 (m, 1H), 7.91 - 7.78 (m, 3H), 7.72 (m, 1H), 7.53 (m, 1H), 7.46 - 7.34 (m, 4H), 7.34 - 7.24 (m, 4H), 7.21 (m, 1H). 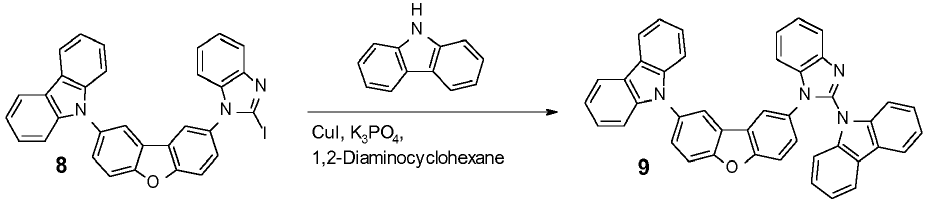
c) **8** (8.70 g, 15.1 mmol) are combined with carbazole (2.52 g, 15.1 mmol), CuI (0.028 g, 0.151 mmol), K₃PO₄ (6.73 g, 31.7 mmol) and 1,2-diaminocyclohexane (0.182 ml, 1.51 mmol) in 1,4-dioxane (80 mL) and the reaction mixture is heated at 115 °C for 30 hours. The reaction is allowed to cool to room temperature and is then filtered through a pad of celite washing well with ethyl acetate. The resulting solution is evaporated to give a crude material as a black oil. The crude material is purified by chromatography on neutral silica gel using hexane/ethyl acetate (9/1) as eluent and is then recrystallized by dichloromethane to give **9** (= **C-8**) as a slightly-yellow solid (3.50 g, 38 % yield). ¹H NMR (400 MHz, Chloroform-d) δ 8.17 (d, *J* = 7.7 Hz, 2H), 7.98 (d, *J* = 7.8 Hz, 3H), 7.88 (m, 1H), 7.80 (m, 1H), 7.72 80 (m, 1H), 7.61 80 (m, 1H), 7.55 - 7.34 (m, 9H), 7.34 - 7.17 (m, 8H). Product confirmed by LCMS m/z = 615 (M+1).

### Comparative Application Example 1

**A** glass substrate with 120 nm-thick indium-tin-oxide (ITO) transparent electrode used as an anode is first cleaned with isopropanol in an ultrasonic bath for 10 min. To eliminate any possible organic residues, the substrate is exposed to an ultraviolet light and ozone for further 30 min. This treatment also improves the hole injection properties of the ITO. The cleaned substrate is mounted on a substrate holder and loaded into a vacuum chamber.

Thereafter, the organic materials specified below are applied by vapor deposition to the ITO substrate at a rate of approx. 0.2-1 Å/sec at about 10⁻⁶ -10⁻⁸ mbar. As a hole injection layer, compound 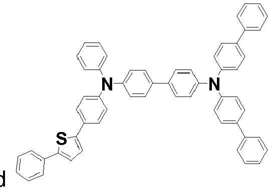 with 30 nm thickness is applied. Then compound 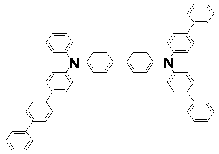 with 60 nm thickness is applied as a hole transporting layer. As an exciton and electron blocker, compound 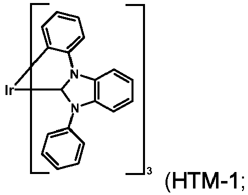 for preparation, see Ir complex (7) in the application WO2005/019373) is then applied with a thickness of 10 nm.

Subsequently, a mixture of 20% by weight of emitter compound, 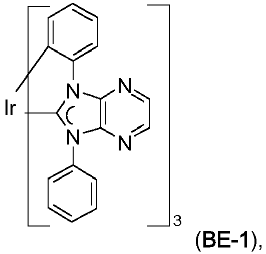 15% by weight of compound (**HTM-1**) and 65% by weight of host 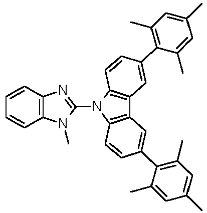 (**CH-1**) are applied to form a 40 nm-thick emitting layer. On the emitting layer, 5 nm-thick material 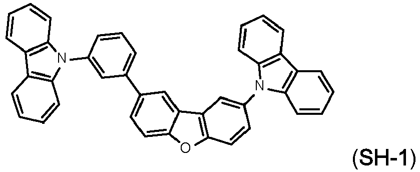 is applied as an exciton blocker.

Thereafter, compound 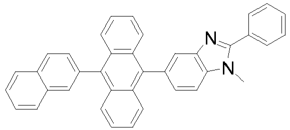 with 20 nm thickness is deposited as an electron transport layer. Finally, 1 nm-thick LiF is deposited as an electron injection layer and 80 nm-thick Al is then deposited as a cathode to complete the device. The device is sealed with a glass lid and a getter in an inert nitrogen atmosphere with less than 1 ppm of water and oxygen.

### OLED characterization

To characterize the OLED, electroluminescence spectra are recorded at various currents and voltages. In addition, the current-voltage characteristic is measured in combination with the luminance to determine luminous efficiency and external quantum efficiency (EQE). Driving voltage U and EQE are given at luminance (L) = 1000 cd/m² and Commission Internationale de l'Éclairage (CIE) coordinate are given at 5mA/cm² except otherwise stated. Furthermore, 70% lifetime (LT70), the time spent until the initial luminance of 4'000 cd/m² is reduced to 70% (2'800 cd/m²), is recorded. The LT70 of the Comparative Application Examples are set to 100 and the data of the Application Examples are specified in relation to those of the respective Comparative Application Examples.

### Application Example 1 and 2

Comparative Application Example 1 is repeated except that the host (CH-1) is replaced by compound 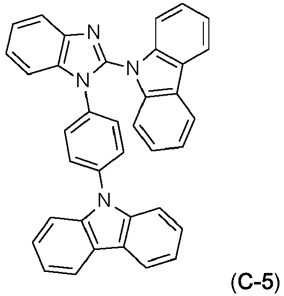 or compound

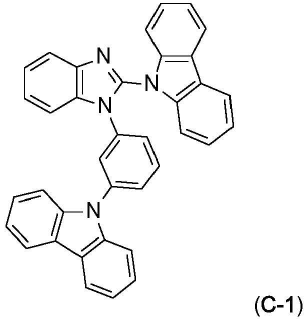

The device results are shown in Table 1.

**Table 1**

| Appl. Ex. | Host | U [V] | EQE [%] | CIEx | CIEy | LT70 |
|---|---|---|---|---|---|---|
| 1 | (C-5) | 5.22 | 15.76 | 0.152 | 0.266 | 295 |
| 2 | (C-1) | 5.45 | 15.99 | 0.150 | 0.267 | 597 |
| Comp. Appl. Ex. 1 | (CH-1) | 6.02 | 15.33 | 0.150 | 0.256 | 100 |

The results shown in Table 1 demonstrate that the EQE and lifetime are improved when compounds (**C-5**) and (**C-1**) are used as host instead of reference compound (**CH-1**).

### Comparative Application Example 2

Comparative Application Example 1 is repeated except that the exciton and electron blocker (**HTM-1**) is replaced by compound (**CH-1**), and host (**CH-1**) is replaced by compound (**SH-1**).

### Application Example 3

Comparative Application Example 2 is repeated except that the exciton and electron blocker (**CH-1**) is replaced by compound (**C-1**). The device results are shown in Table 2.

**Table 2**

| Appl. Ex. | Exciton and electron blocker | U [V] | EQE [%] | CIEx | CIEy | LT70 |
|---|---|---|---|---|---|---|
| 3 | (C-1) | 7.37 | 13.93 | 0.152 | 0.273 | 1066 |
| Comp. Appl. Ex. 2 | (CH-1) | 7.71 | 14.11 | 0.152 | 0.268 | 100 |

The results shown in Table 2 demonstrate that the driving voltage and lifetime are improved when compound (**C-1**) is used as exciton and electron blocker instead of reference compound (**CH-1**).

### Comparative Application Example 3

A glass substrate with 120 nm-thick ITO is cleaned and treated in the same manner as comparative application example 1. As a hole injection layer, compound 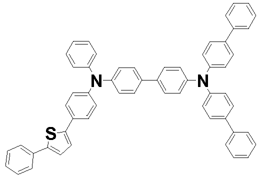 with 30 nm thickness is applied by vapor deposition. Then 60 nm of compound (**SH-1**) doped with MoOx (∼10%) is deposited as hole transporting layer. MoOx is used to improve the hole conductivity of **SH-1.** As an exciton and electron blocker, compound (**CH-1**) is applied with a thickness of 10 nm. Subsequently, a mixture of 20% by weight of emitter compound (**BE-1**) and 80% by weight of host 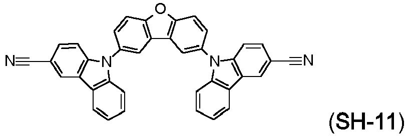 are applied to form a 40 nm of emitting layer. On the emitting layer, 5 nm of material (**SH-11**) is applied as an exciton blocker. Thereafter, compound 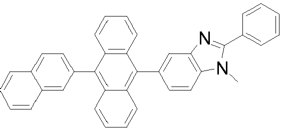 with 20 nm thickness is deposited as an electron transport layer. Finally, 1 nm of LiF is deposited as an electron injection layer and 80 nm of Al is then deposited as a cathode to complete the device. The device is sealed with a glass lid and a getter in an inert nitrogen atmosphere with less than 1 ppm of water and oxygen.

### Application Examples 4 and 5

Comparative Application Example 3 is repeated except that the exciton and electron blocker (**CH-1**) is replaced by compound (**C-5**) or compound (**C-1**) for application examples 5 or 6, respectively. The device results are shown in Table 3.

**Table 3**

| Appl. Ex. | Exciton and electron blocker | U [V] | EQE [%] | CIEx | CIEy | LT70 |
|---|---|---|---|---|---|---|
| 4 | (C-5) | 4.39 | 18.08 | 0.156 | 0.322 | 545 |
| 5 | (C-1) | 4.09 | 17.67 | 0.155 | 0.306 | 308 |
| Comp. Appl. Ex. 3 | (CH-1) | 4.50 | 17.86 | 0.155 | 0.302 | 100 |

The results shown in Table 3 demonstrate that the driving voltage, EQE and lifetime are improved when compounds (**C-5**) or (**C-1**) are used as exciton and electron blocker instead of reference compound (**CH-1**).

### Synthesis of compound (SH-11)

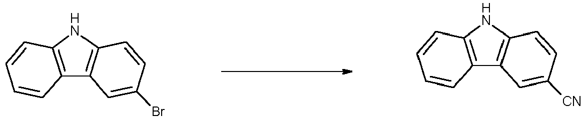

a) 100 g (0.406 mol) 3-Bromo-9H-carbazole and 58.2 g (0.650 mol) CuCN are added to 800 ml of DMF and the suspension is heated to reflux for 20 h under Nitrogen. The resulting solution is cooled to room temperature and then poured on 4 l of water. The suspension is filtered and the residue is washed three times with water (1 l each). The white solid is suspended in 1.5 l of 10% Ammonia in water, stirred for three hours, filtered, washed three times with water (1 l each) and dried at 80°C/125 mbar overnight. The resulting solid is dissolved in 1 l of boiling THF, 100 g of silica is added, stirred for 1 h and then filtered hot. The filtrate is evaporated to 400 ml, cooled to room temperature, filtered, washed three times with cold THF (50 ml each) and dried at 80°C/125 mbar overnight to yield 43.3 g (55.6% of theory) 9H-Carbazole-3-carbonitrile as a white solid.
   ¹H-NMR (400 MHz, CDCl₃): δ 8.39 (br s, 2H), 8.10 (d, J = 8.0 Hz, 1H), 7.67 (dxd, J₁ = 8.4 Hz, J₂= 1.6 Hz, 1H), 7.54 - 7.47 (m, 3H), 7.35 - 7.31 (m, 1H). 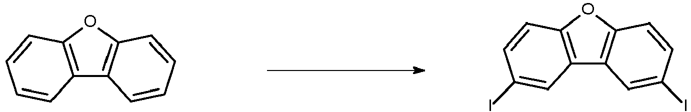
b) 100 g (0.594 mol) Dibenzofuran are dissolved in 1.2 l of AcOH. 116.1 g (0.457 mol) I₂, 44.2 g (0.252 mol) HIO₃, 40 ml of water, 12 ml of H₂SO₄ 97% and 60 ml of CCl₄ are added and the mixture is stirred at reflux for 22 h under Nitrogen. The resulting suspension is cooled to room temperature, filtered, washed four times with water (1 l each) and dried at 60°C/125 mbar overnight. The crude product is crystallized twice from Toluene to yield 113.5 g (59.1% of theory) 2,8-Diiododibenzofuran as a white solid.
   ¹H-NMR (300 MHz, CDCl₃): δ 8.22 (s, 2H), 7.76 (d, J = 6.9 Hz, 2H), 7.35 (d, J = 6.9 Hz, 2H). 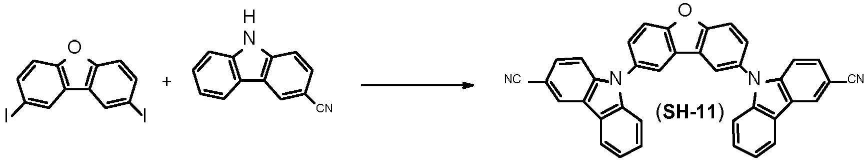
c) 20 g (47.4 mmol) 2,8-Diiododibenzofuran, 20.19 g (104.4 mmol) 9H-Carbazole-3-carbonitrile,1.36 g (7.1 mmol) CuI, 2.84 g (14.2 mmol) 1,10-Phenanthroline, 32.2 g (151.9 mmol) K₃PO₄ and 500 ml Mesitylene are mixed and heated to reflux for 72 h under Nitrogen. The brown suspension is cooled to 0°C, filtered and the residue is washed three times with Heptane (100 ml each) and twice with MeOH (100 ml each). The filter cake is suspended in 1 l of water, stirred for 30 min, filtered, washed three times with water (200ml each) and dried at 80°C/125 mbar overnight. The crude product is suspended in 1.2 l of THF and heated to reflux. 60 g of silica are added, stirred for 15 min, filtered hot and the residue is washed three times with hot THF (250 ml each). The filtrate is evaporated and the residue is decocted with 200 ml of MeOH for 1 h, filtered hot and washed three times with MeOH (50 ml each). The product is purified by Soxhlet extraction with Toluene, followed by Soxhlet extraction with MEK then by decocting with 150 ml of 1,4-Dioxane, filtered hot and washed three times with 1,4-Dioxane (20 ml each) to yield 12.3 g (47.1% of theory) 9-[8-(3-Cyanocarbazol-9-yl)dibenzofuran-2-yl]carbazole-3-carbonitrile as a white solid. ¹H-NMR (400 MHz, DMSO): δ 8.89 (d, J = 1.2 Hz, 2H), 8.60 (d, J = 2.0 Hz, 2H), 8.41 (d, J = 7.6 Hz., 2H), 8.14 (d, J = 8.8 Hz, 2H), 7.86 (dxd, J₁ = 8.8 Hz, J₂ = 2.0 Hz, 2H), 7.82 (dxd, J₁ = 8.8 Hz, J₂ = 1.2 Hz, 2H), 758 - 7.50 (m, 4H), 7.45 - 7.41 (m, 4H).

## Claims

1. A compound of the formula 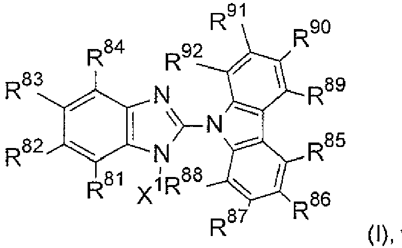 wherein
R⁸¹, R⁸², R⁸³ and R⁸⁴ are independently of each other H, a C₁-C₂₅alkyl group, which can optionally be substituted by E and or interrupted by D; a C₆-C₂₄aryl group, which can optionally be substituted by G, a C₂-C₃₀heteroaryl group, which can optionally be substituted by G;
R⁸⁵, R⁸⁶, R⁸⁷, R⁸⁸, R⁸⁹, R⁹⁰, R⁹¹ and R⁹² are independently of each other H, CN, a C₁-C₂₅alkyl group, which can optionally be substituted by E and or interrupted by D; a C₆-C₂₄aryl group, which can optionally be substituted by G, a C₂-C₃₀heteroaryl group, which can optionally be substituted by G;
with the proviso that R⁸⁵, R⁸⁶, R⁸⁷, R⁸⁸, R⁸⁹, R⁹⁰, R⁹¹ and R⁹² are different from a carbazolyl group,
X¹ is a group of formula -A¹-X², which is selected from 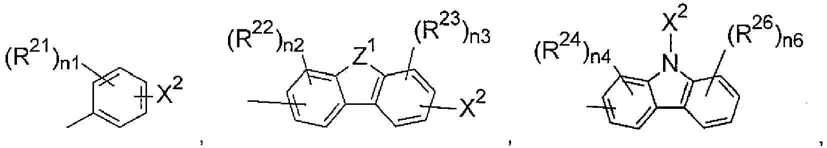 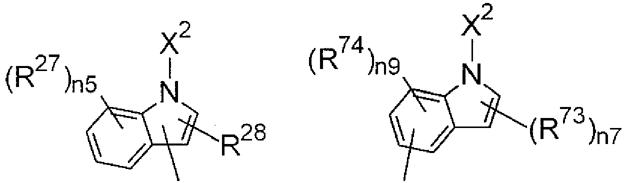 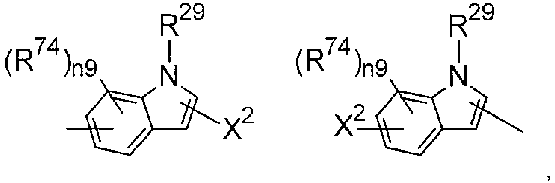 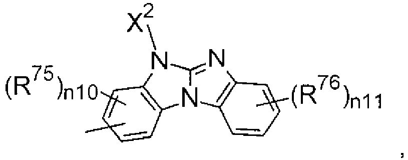 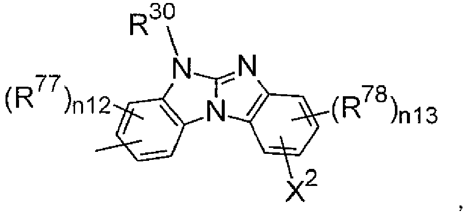 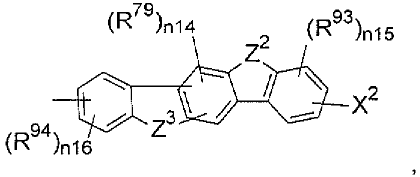 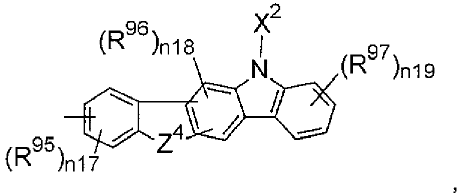 or 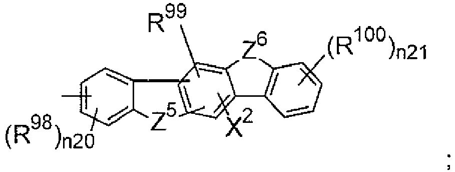
X² is a group of formula -(A²)ₚ-(A³)_{q}-(A⁴)ᵣ-R¹⁶,
p is 0, or 1, q is 0, or 1, r is 0, or 1;
R¹⁶ is a group of formula 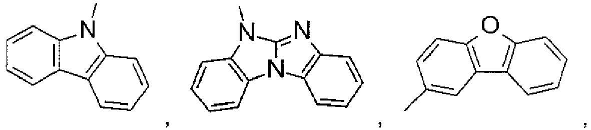 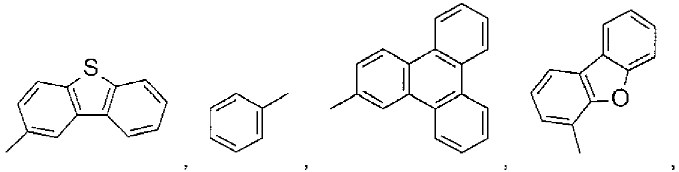 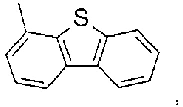 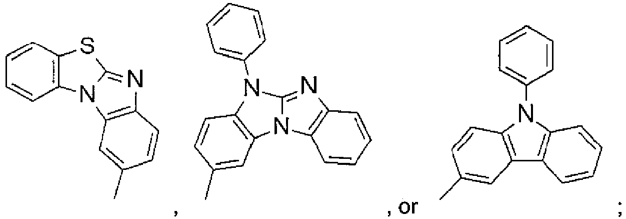
R¹⁰ and R¹¹ are independently of each other a C₆-C₂₄aryl group, which can optionally be substituted by G; or a C₂-C₃₀heteroaryl group, which can optionally be substituted by G;
R¹², R¹³ and R¹⁴ are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G;
n7, n14 and n18 are independently of each other 0, 1, or 2;
n2, n3, n4, n9, n10, n12, n13, n15, n16, n17 and n20 are independently of each other 0, 1, 2, or 3,
n1, n5, n6, n11, n19 and n21 are independently of each other 0, 1, 2, 3, or 4;
R²¹, R²², R²³, R²⁴, R²⁶, R²⁷, R²⁸, R⁷³, R⁷⁴, R⁷⁵, R⁷⁶, R⁷⁷, R⁷⁸, R⁷⁹, R⁹³, R⁹⁴, R⁹⁵, R⁹⁶, R⁹⁷, R⁹⁸, R⁹⁹ and R¹⁰⁰ are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G;
Z¹, Z², Z³, Z⁴, Z⁵ and Z⁶ are independently of each other O, S, or NR²⁹;
R²⁹ and R³⁰ are independently of each other a C₁-C₂₅alkyl group, which can optionally be substituted by E and or interupted by D; a C₆-C₂₄aryl group, which can optionally be substituted by G; or a C₂-C₃₀heteroaryl group, which can optionally be substituted by G;
A², A³ and A⁴ are independently of each other a C₆-C₂₄arylen group, which can optionally be substituted by G, or a C₂-C₃₀heteroarylen group, which can optionally be substituted by G;
D is -CO-, -COO-, -S-, -O-, -NR⁶⁵-, -SiR⁷⁰R⁷¹-, -POR⁷²-, -CR⁶³=CR⁶⁴-, or -C≡C-,
E is -OR⁶⁹, -SR69, -NR⁶⁵R⁶⁶, -COR68, -COOR⁶⁷, -CONR⁶⁵R⁶⁶, -CN, or F,
G is E, or a C₁-C₁₈alkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈arkyl group; or a C₁-C₁₈alkyl group, which is interrupted by -O-; or
R⁶⁵ and R⁶⁶ together form a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-, R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by - O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl, with the proviso that
X¹ is bonded to the nitrogen atom by a carbon atom.

2. The compound according to claim 1, wherein R⁸¹, R⁸², R⁸³ and R⁸⁴ are H.

3. The compound according to claim 1 or 2, which is a compound of formula 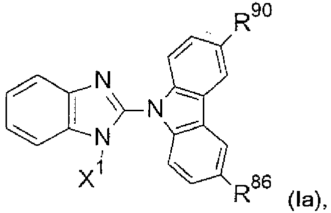 wherein X¹, R⁸⁶ and R⁹⁰ are defined in claim 1.

4. The compound according to claim 3, wherein R⁹⁰ is H.

5. The compound according to claim 3, wherein R⁸⁶ and R⁹⁰ are H.

6. The compound according to any of claims 1 to 5, wherein X¹ is a group of formula - A¹-X², which is selected from 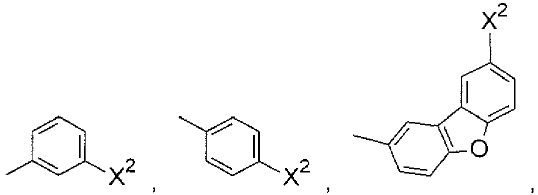 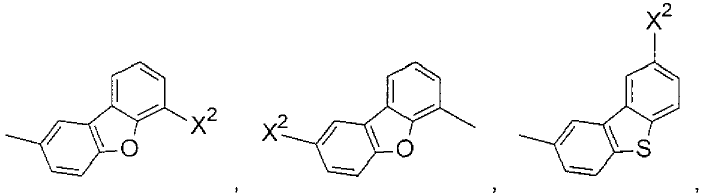 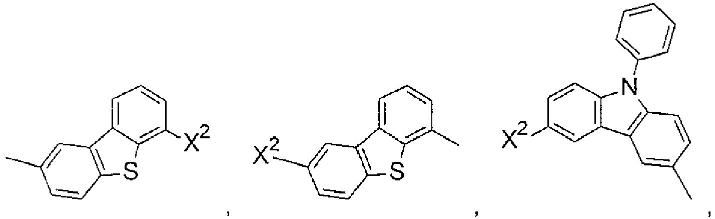 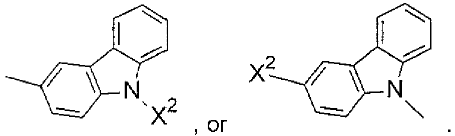 X² is a group of formula -(A²)ₚ-(A³)_{q}-(A⁴)ᵣ-R¹⁶, wherein p, q, r, R¹⁶, A², A³ and A⁴ are defined in claim 1 .

7. The compound according to any of claims 1 or 6, wherein A², A³ and A⁴ are independently of each other a group of formula 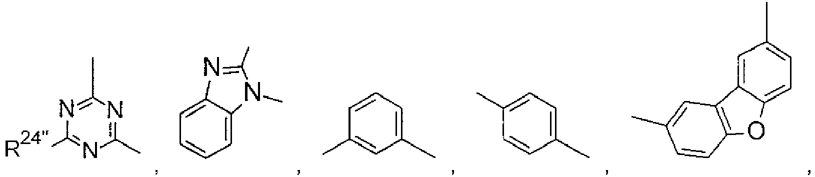 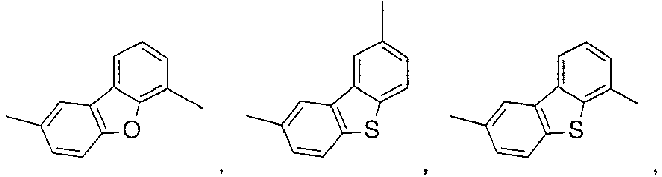 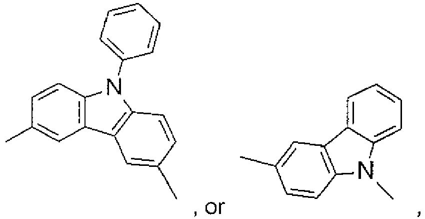 wherein R^{24"} is 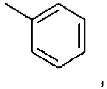 or

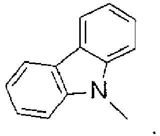

8. The compound according to any of claims 1 to 7, wherein the group -A¹-(A²)ₚ-(A³)_{q}-(A⁴)ᵣ- is a group of formula,

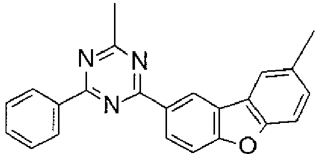

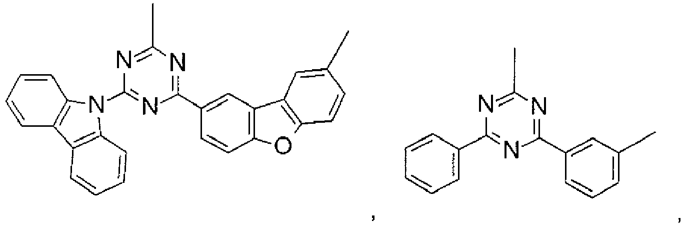

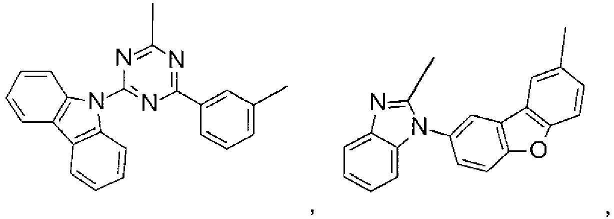

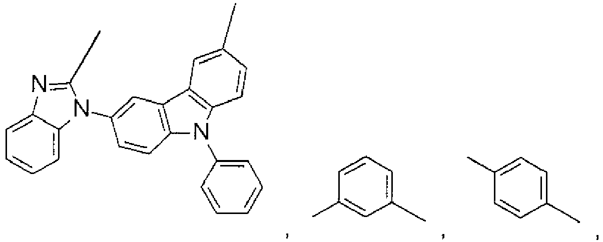

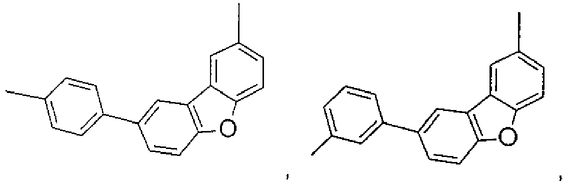

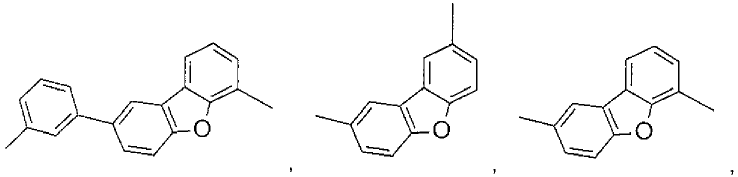

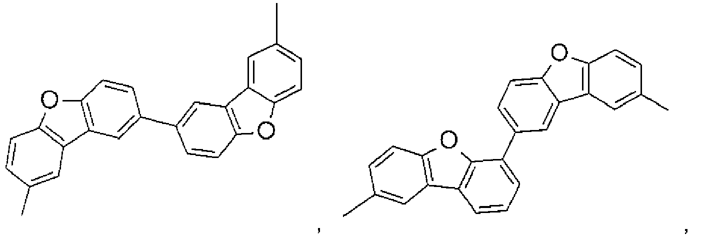

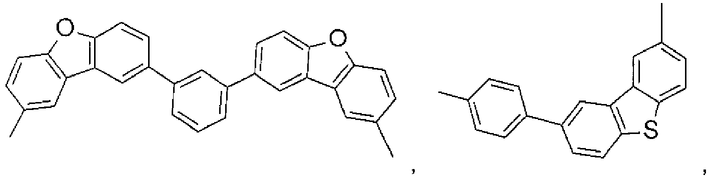

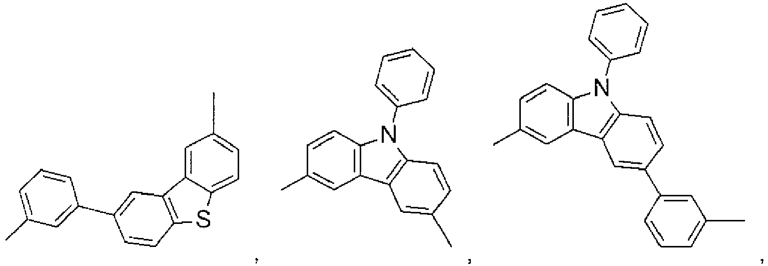

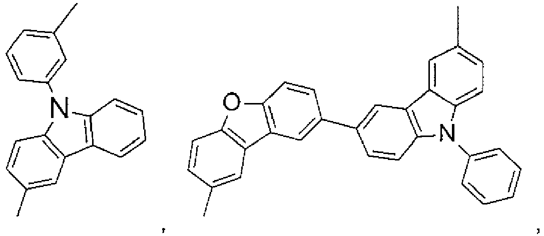

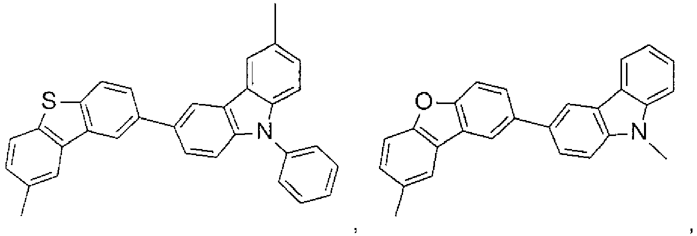

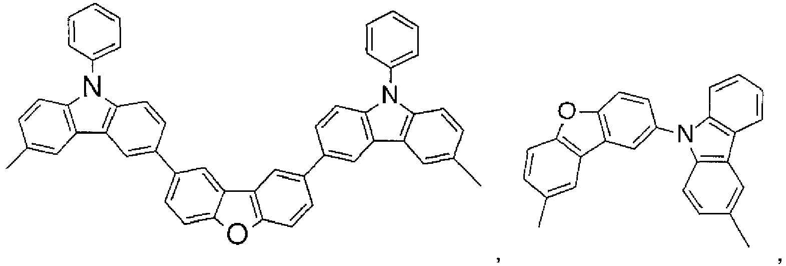

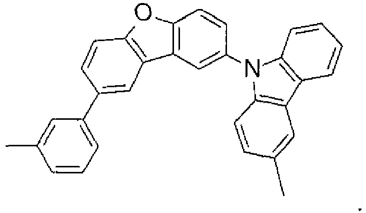

9. The compound according to any of claims 1 to 8, which is a compound of formula 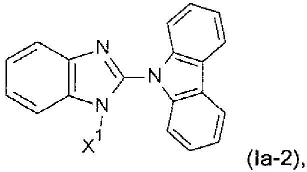 wherein
X¹ is a group of formula -A¹-(A²)ₚ-(A³)_{q}-(A⁴)ᵣ-R¹⁶, wherein
-A¹-(A²)ₚ-(A³)_{q}-(A⁴)ᵣ- is a group of formula 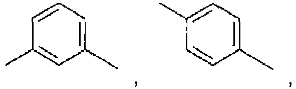 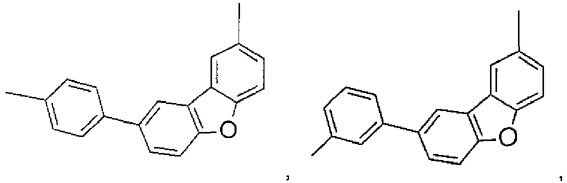 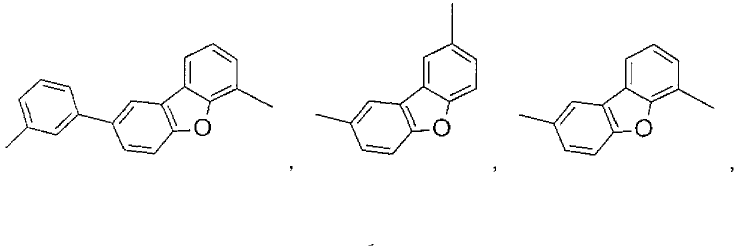 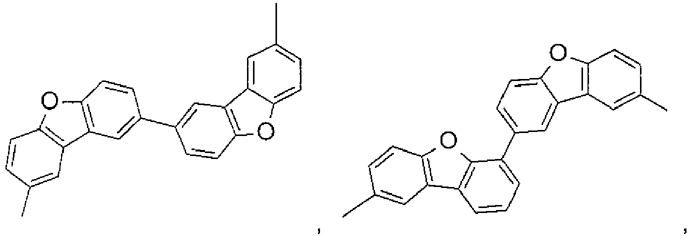 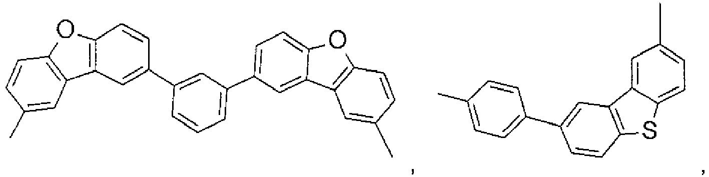 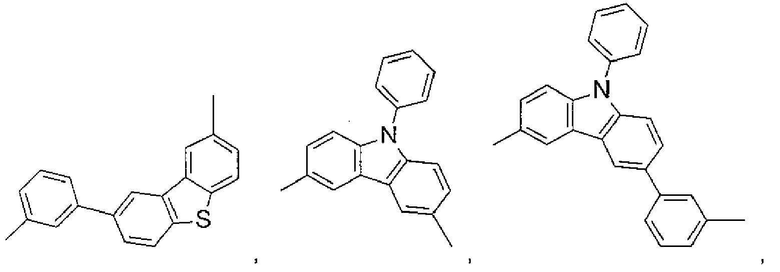 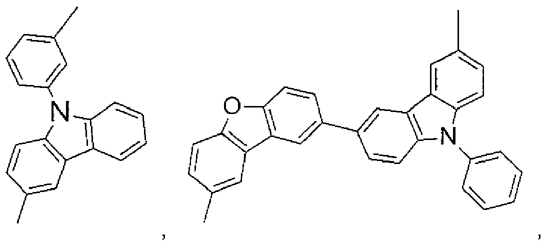 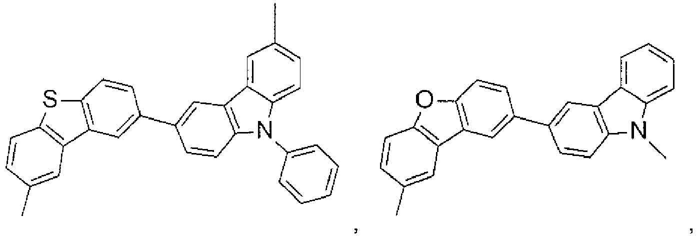 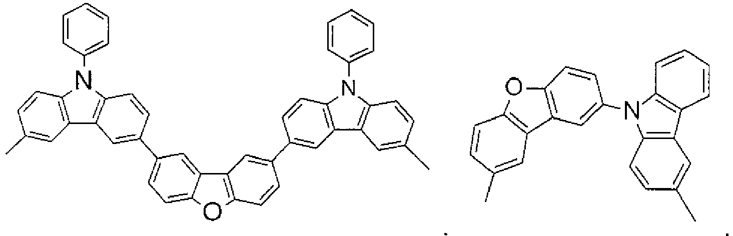 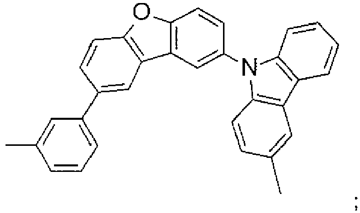 and
R¹⁶ is a group of formula

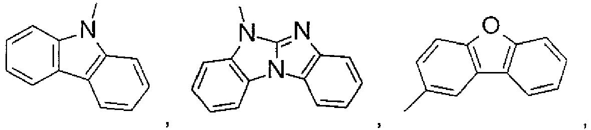

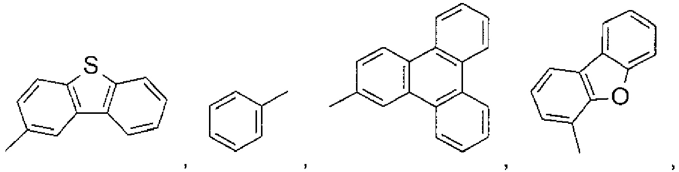

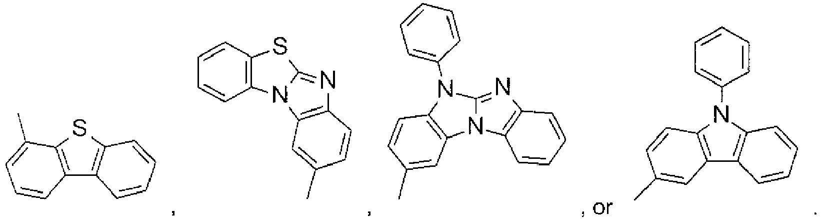

10. An electronic device, comprising a compound according to any of claims 1 to 9.

11. The electronic device according to claim 10, which is an electroluminescent device.

12. A charge transport layer, a charge/exciton blocker layer, or an emitting layer comprising a compound according to any of claims 1 to 9.

13. The emitting layer according to claim 12, comprising a compound according to any of claims 1 to 11 as host material in combination with a phosphorescent emitter.

14. An apparatus selected from the group consisting of stationary visual display units; mobile visual display units; illumination units; keyboards; items of clothing; furniture; wallpaper, comprising the organic electronic device according to claim 10, or 11, or the charge transport layer, the charge/exciton blocker layer, or the emitting layer according to claim 12.

15. Use of the compounds of formula I according to any of claims 1 to 9 for electrophotographic photoreceptors, photoelectric converters, organic solar cells, switching elements, organic light emitting field effect transistors, image sensors, dye lasers and electroluminescent devices.

## Patentansprüche

1. Verbindung der Formel 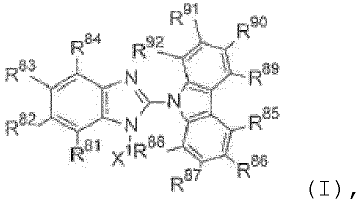 wobei
R⁸¹, R⁸², R⁸³ und R⁸⁴ unabhängig voneinander für H, eine C₁-C₂₅-Alkylgruppe, die gegebenenfalls durch E substituiert und oder durch D unterbrochen sein kann, eine C₆-C₂₄-Arylgruppe, die gegebenenfalls durch G substituiert sein kann, oder eine C₂-C₃₀-Heteroarylgruppe, die gegebenenfalls durch G substituiert sein kann, stehen,
R⁸⁵, R⁸⁶, R⁸⁷, R⁸⁸, R⁸⁹, R⁹⁰, R⁹¹ und R⁹² unabhängig voneinander für H, CN, eine C₁-C₂₅-Alkylgruppe, die gegebenenfalls durch E substituiert und oder durch D unterbrochen sein kann, eine C₆-C₂₄-Arylgruppe, die gegebenenfalls durch G substituiert sein kann, oder eine C₂-C₃₀-Heteroarylgruppe, die gegebenenfalls durch G substituiert sein kann, stehen,
mit der Maßgabe, dass R⁸⁵, R⁸⁶, R⁸⁷, R⁸⁸, R⁸⁹, R⁹⁰, R⁹¹ und R⁹² von einer Carbazolylgruppe verschieden sind,
X¹ für eine Gruppe der Formel -A¹-X² steht, die aus 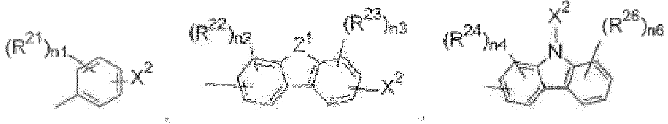 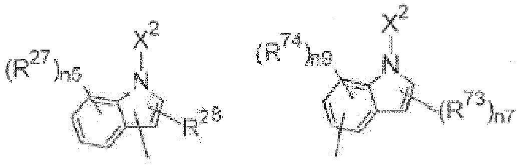 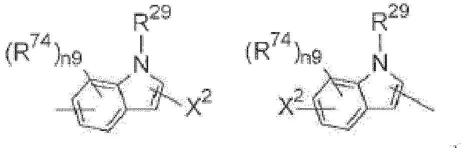 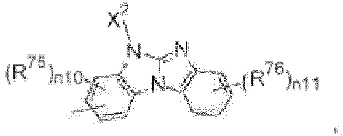 oder 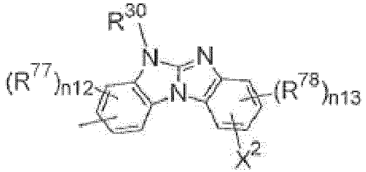 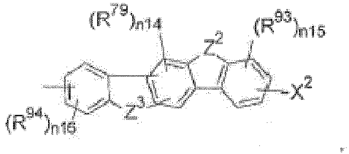 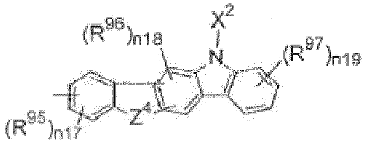 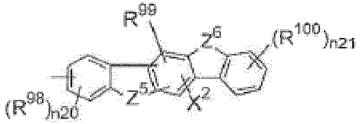 ausgewählt ist,
X² für eine Gruppe der Formel - (A²)ₚ-(A³)_{q}-(A⁴)ᵣ-R¹⁶ steht,
p für 0 oder 1 steht, q für 0 oder 1 steht, r für 0 oder 1 steht,
R¹⁶ für eine Gruppe der Formel 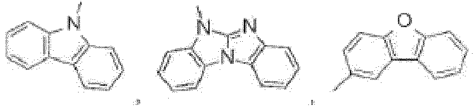 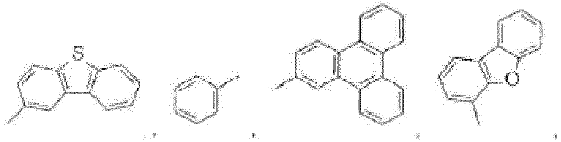 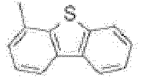 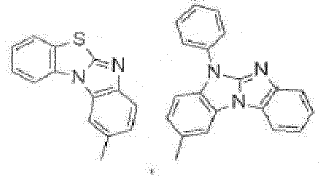 oder 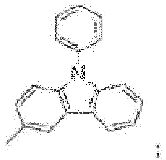 steht,
R¹⁰ und R¹¹ unabhängig voneinander für eine C₆-C₂₄-Arylgruppe, die gegebenenfalls durch G substituiert sein kann, oder eine C₂-C₃₀-Heteroarylgruppe, die gegebenenfalls durch G substituiert sein kann, stehen,
R¹², R¹³ und R¹⁴ unabhängig voneinander für eine C₁-C₂₅-Alkylgruppe, die gegebenenfalls durch E substituiert und oder durch D unterbrochen sein kann, eine C₆-C₂₄-Arylgruppe, die gegebenenfalls durch G substituiert sein kann, oder eine C₂-C₃₀-Heteroarylgruppe, die gegebenenfalls durch G substituiert sein kann, stehen,
n7, n14 und n18 unabhängig voneinander für 0, 1 oder 2 stehen,
n2, n3, n4, n9, n10, n12, n13, n15, n16, n17 und n20 unabhängig voneinander für 0, 1, 2 oder 3 stehen,
n1, n5, n6, n11, n19 und n21 unabhängig voneinander für 0, 1, 2, 3 oder 4 stehen,
R²¹, R²², R²³, R²⁴, R²⁶, R²⁷, R²⁸, R⁷³, R⁷⁴, R⁷⁵, R⁷⁶, R⁷⁷, R⁷⁸, R⁷⁹, R⁹³, R⁹⁴, R⁹⁵, R⁹⁶, R⁹⁷, R⁹⁸, R⁹⁹ und R¹⁰⁰ unabhängig voneinander für eine C₁-C₂₅-Alkylgruppe, die gegebenenfalls durch E substituiert und oder durch D unterbrochen sein kann, eine C₆-C₂₄-Arylgruppe, die gegebenenfalls durch G substituiert sein kann, oder eine C₂-C₃₀-Heteroaryl-gruppe, die gegebenenfalls durch G substituiert sein kann, stehen,
Z¹, Z², Z³, Z⁴, Z⁵ und Z⁶ unabhängig voneinander für O, S oder NR²⁹ stehen,
R²⁹ und R³⁰ unabhängig voneinander für eine C₁-C₂₅-Alkylgruppe, die gegebenenfalls durch E substituiert und oder durch D unterbrochen sein kann, eine C₆-C₂₄-Arylgruppe, die gegebenenfalls durch G substituiert sein kann, oder eine C₂-C₃₀-Heteroarylgruppe, die gegebenenfalls durch G substituiert sein kann, stehen,
A², A³ und A⁴ unabhängig voneinander für eine C₆-C₂₄-Arylengruppe, die gegebenenfalls durch G substituiert sein kann, oder eine C₂-C₃₀-Heteroarylengruppe, die gegebenenfalls durch G substituiert sein kann, stehen,
D für -CO-, -COO-, -S-, -O-, -NR⁶⁵-, -SiR⁷⁰R⁷¹-, -POR⁷²-, -CR⁶³=CR⁶⁴- oder -C≡C- steht,
E für -OR⁶⁹, -SR⁶⁹, -NR⁶⁵R⁶⁶, -COR⁶⁸, -COOR⁶⁷, -CONR⁶⁵R⁶⁶, -CN oder F steht,
G für E oder eine C₁-C₁₈-Alkylgruppe, eine C₆-C₂₄-Arylgruppe, eine C₆-C₂₄-Arylgruppe, die durch F, C₁-C₁₈-Alkyl oder C₁-C₁₈-Alkyl, das durch O unterbrochen ist, substituiert ist, eine C₂-C₃₀-Heteroarylgruppe oder eine C₂-C₃₀-Heteroarylgruppe, die durch F, C₁-C₁₈-Alkyl oder C₁-C₁₈-Alkyl, das durch O unterbrochen ist, substituiert ist, steht, R⁶³ und R⁶⁴ unabhängig voneinander für H, C₆-C₁₈-Aryl, C₆-C₁₈-Aryl, das durch C₁-C₁₈-Alkyl oder C₁-C₁₈-Alkoxy substituiert ist, C₁-C₁₈-Alkyl oder C₁-C₁₈-Alkyl, das durch -O- unterbrochen ist, stehen, R⁶⁵ und R⁶⁶ unabhängig voneinander für eine C₆-C₁₈-Arylgruppe, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, stehen oder
R⁶⁵ und R⁶⁶ zusammen einen fünf- oder sechsgliedrigen Ring bilden,
R⁶⁷ für eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht,
R⁶⁸ für H, eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht,
R⁶⁹ für ein C₆-C₁₈-Aryl, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht,
R⁷⁰ und R⁷¹ unabhängig voneinander für eine C₁-C₁₈-Alkylgruppe, eine C₆-C₁₈-Arylgruppe oder eine C₆-C₁₈-Arylgruppe, die durch C₁-C₁₈-Alkyl substituiert ist, stehen und
R⁷² für eine C₁-C₁₈-Alkylgruppe, eine C₆-C₁₈-Arylgruppe oder eine C₆-C₁₈-Arylgruppe, die durch C₁-C₁₈-Alkyl substituiert ist, steht, mit der Maßgabe, dass
X¹ über ein Kohlenstoffatom an das Stickstoffatom gebunden ist.

2. Verbindung nach Anspruch 1, wobei R⁸¹, R⁸², R⁸³ und R⁸⁴ für H stehen.

3. Verbindung nach Anspruch 1 oder 2, bei der es sich um eine Verbindung der Formel 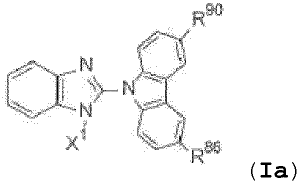 handelt, wobei X¹, R⁸⁶ und R⁹⁰ wie in Anspruch 1 definiert sind.

4. Verbindung nach Anspruch 3, wobei R⁹⁰ für H steht.

5. Verbindung nach Anspruch 3, wobei R⁸⁶ und R⁹⁰ für H stehen.

6. Verbindung nach einem der Ansprüche 1 bis 5, wobei X¹ für eine Gruppe der Formel -A¹-X² steht, die aus 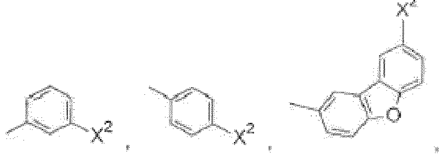 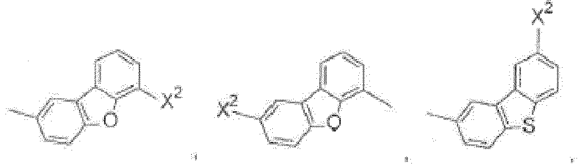 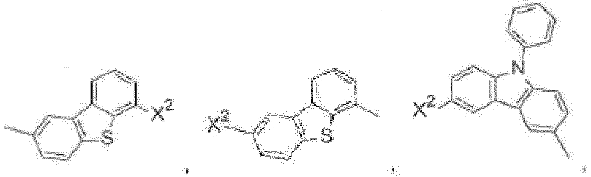 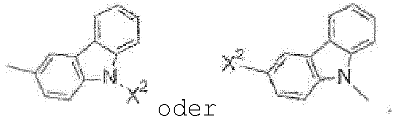 ausgewählt ist,
X² für eine Gruppe der Formel - (A²)ₚ-(A³)_{q}-(A⁴)ᵣ-R¹⁶ steht, wobei p, q, r, R¹⁶, A², A³ und A⁴ wie in Anspruch 1 definiert sind.

7. Verbindung nach einem der Ansprüche 1 bis 6, wobei A², A³ und A⁴ unabhängig voneinander für eine Gruppe der Formel 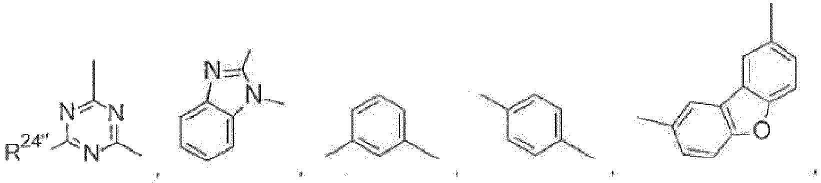 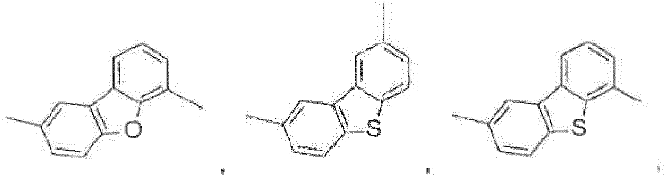 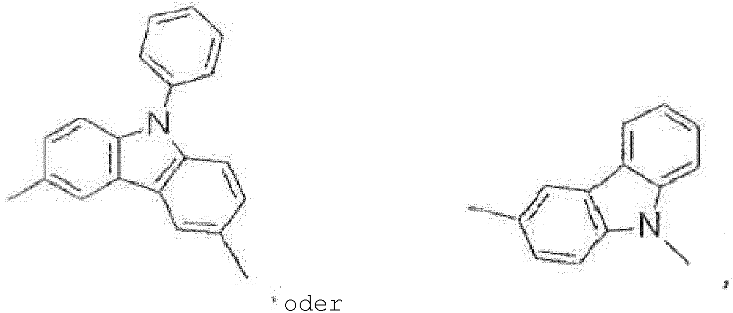 stehen, wobei R^{24"} für 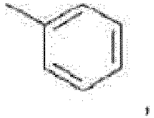 oder 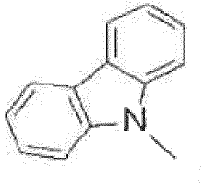 steht.

8. Verbindung nach einem der Ansprüche 1 bis 7, bei dem die Gruppe -A¹-(A²)ₚ-(A³)_{q}-(A⁴)ᵣ für eine Gruppe der Formel 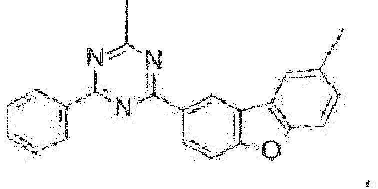 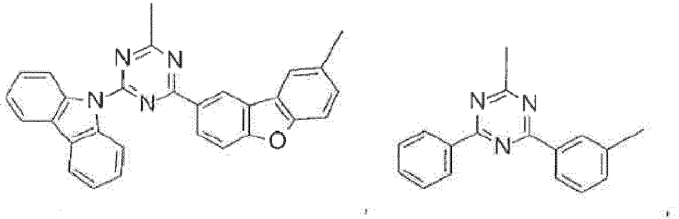 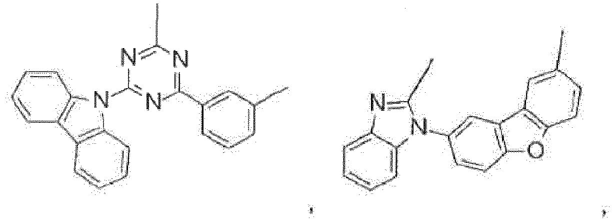 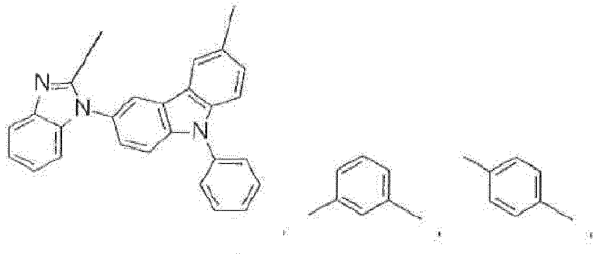 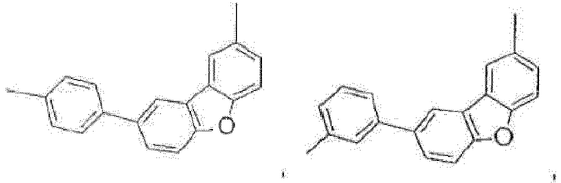 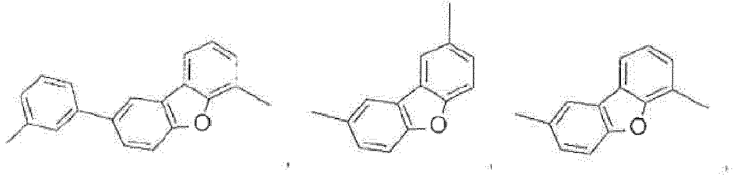 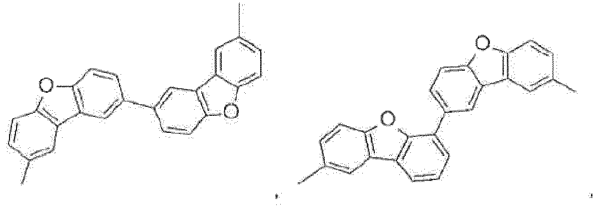 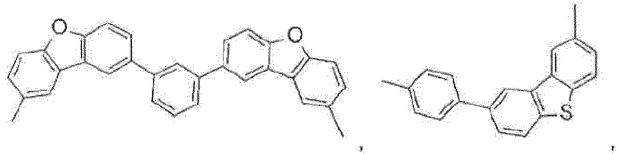 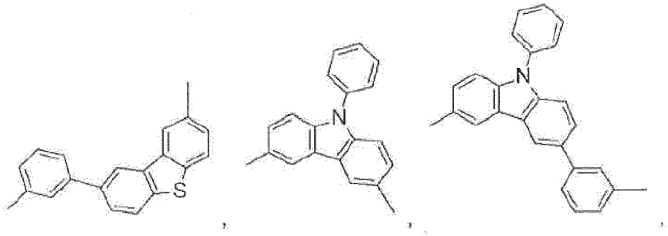 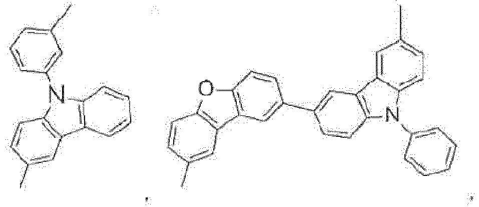 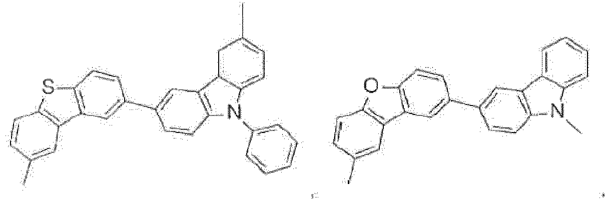 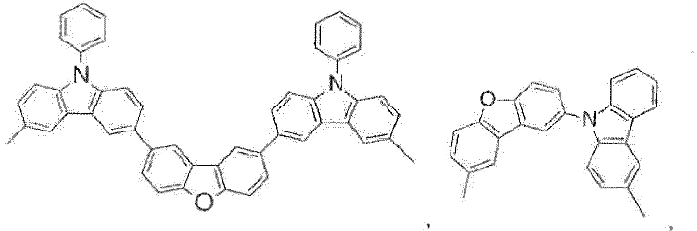 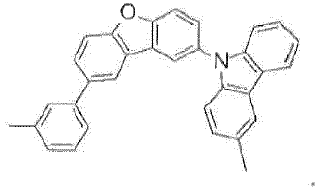 steht.

9. Verbindung nach einem der Ansprüche 1 bis 8, bei der es sich um eine Verbindung der Formel 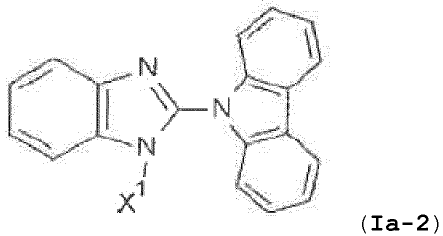 handelt, wobei
X¹ für eine Gruppe der Formel -A¹-(A²)ₚ-(A³)_{q}-(A⁴)ᵣ-R¹⁶ steht, wobei -A¹-(A²)ₚ-(A³)_{q}-(A⁴)ᵣ für eine Gruppe der Formel 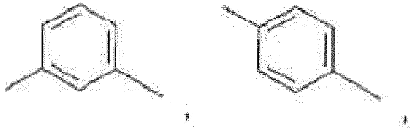 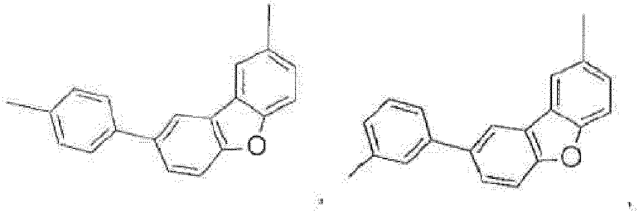 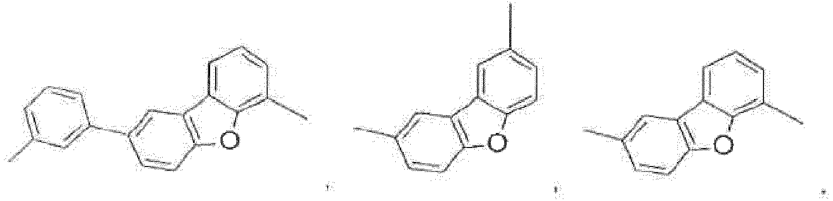 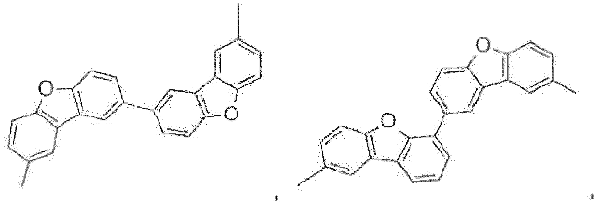 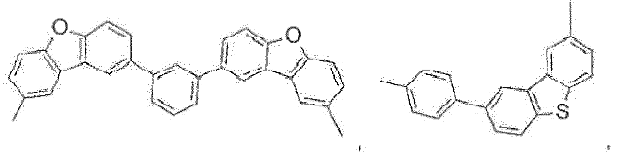 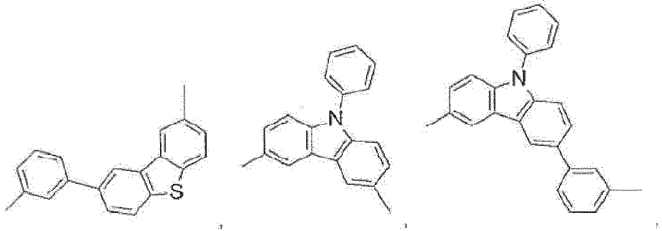 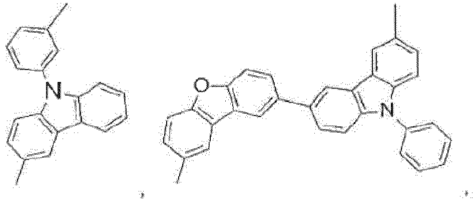 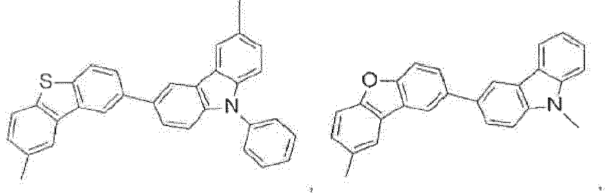 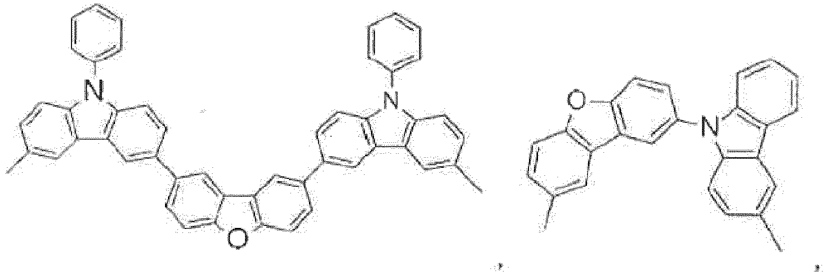 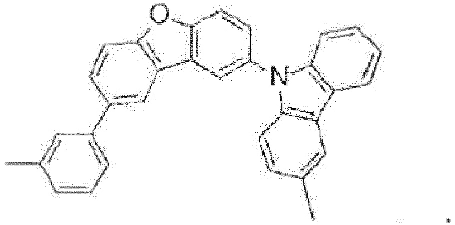 steht, und
R¹⁶ für eine Gruppe der Formel 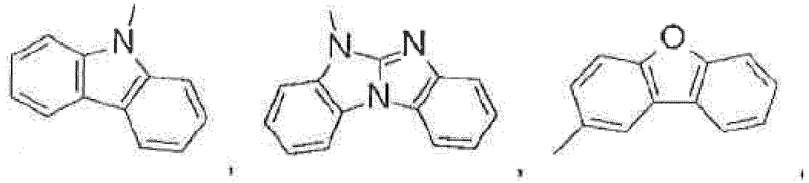 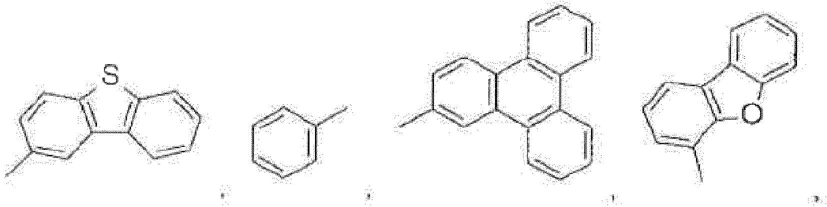 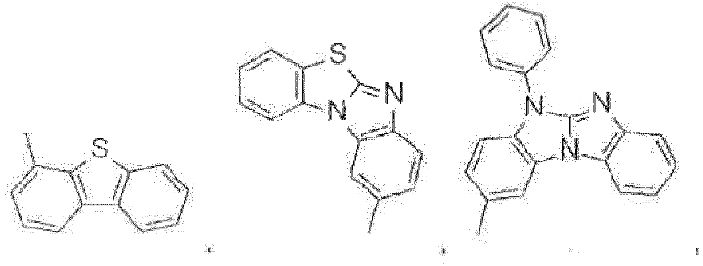 oder 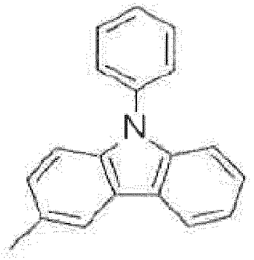 steht.

10. Elektronische Vorrichtung, umfassend eine Verbindung nach einem der Ansprüche 1 bis 9.

11. Elektronische Vorrichtung nach Anspruch 10, bei der es sich um eine Elektrolumineszenzvorrichtung handelt.

12. Ladungstransportschicht, Ladungs-/Exzitonen-Blockerschicht oder emittierende Schicht, umfassend eine Verbindung nach einem der Ansprüche 1 bis 9.

13. Emittierende Schicht nach Anspruch 12, umfassend eine Verbindung nach einem der Ansprüche 1 bis 11 als Wirtsmaterial in Kombination mit einem phosphoreszierenden Emitter.

14. Apparatur, ausgewählt aus der Gruppe bestehend aus stationären Bildschirmen; mobilen Bildschirmen; Beleuchtungseinheiten; Tastaturen; Kleidungsstücken; Möbeln; Tapeten, umfassend die organische elektronische Vorrichtung nach Anspruch 10 oder 11 oder die Ladungstransportschicht, die Ladungs-/Exzitonen-Blockerschicht oder die emittierende Schicht nach Anspruch 12.

15. Verwendung der Verbindungen der Formel I nach einem der Ansprüche 1 bis 9 für elektrophotographische Photorezeptoren, photoelektrische Umwandler, organische Solarzellen, Schaltelemente, organische lichtemittierende Feldeffekttransistoren, Bildsensoren, Farbstofflaser und Elektrolumineszenzvorrichtungen.

## Revendications

1. Composé de formule 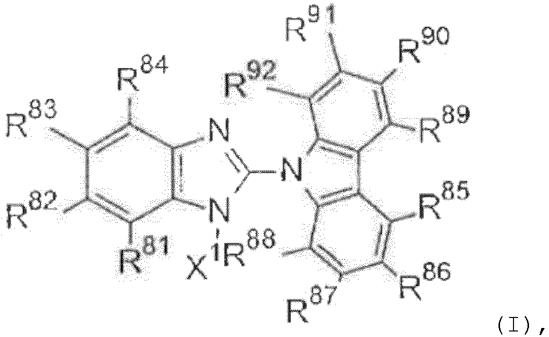 dans lequel
R⁸¹, R⁸², R⁸³ et R⁸⁴ sont, indépendamment les uns des autres, H, un groupe alkyle en C₁-C₂₅, qui peut facultativement être substitué par E et/ou interrompu par D ; un groupe aryle en C₆-C₂₄, qui peut facultativement être substitué par G, un groupe hétéroaryle en C₂-C₃₀, qui peut facultativement être substitué par G ;
R⁸⁵, R⁸⁶, R⁸⁷, R⁸⁸, R⁸⁹, R⁹⁰, R⁹¹ et R⁹² sont, indépendamment les uns des autres, H, CN, un groupe alkyle en C₁-C₂₅, qui peut facultativement être substitué par E et/ou interrompu par D ; un groupe aryle en C₆-C₂₄, qui peut facultativement être substitué par G, un groupe hétéroaryle en C₂-C₃₀, qui peut facultativement être substitué par G ;
à condition que R⁸⁵, R⁸⁶, R⁸⁷, R⁸⁸, R⁸⁹, R⁹⁰, R⁹¹ et R⁹² soient différents d'un groupe carbazolyle,
X¹ est un groupe de formule -A¹-X², qui est choisi parmi 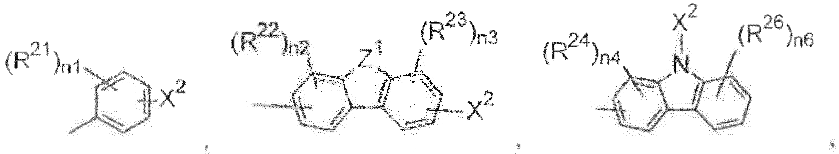 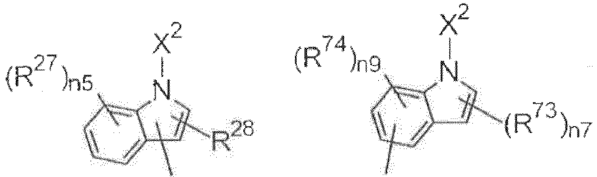 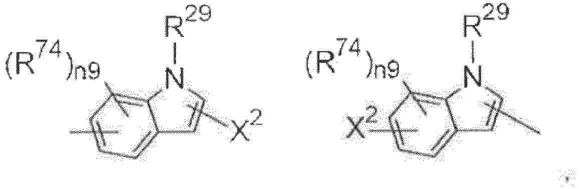 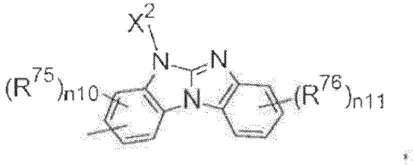 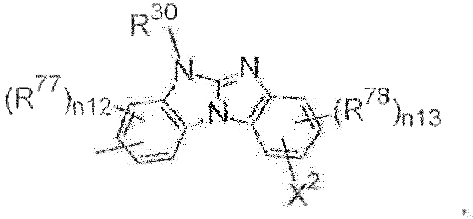 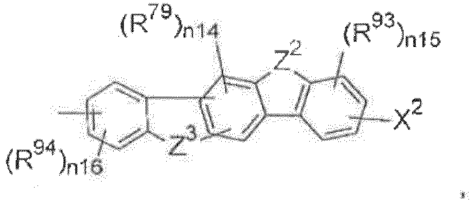 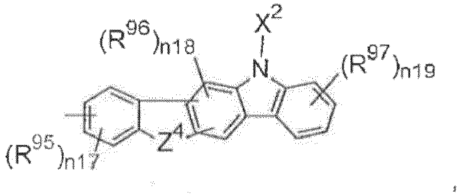 ou 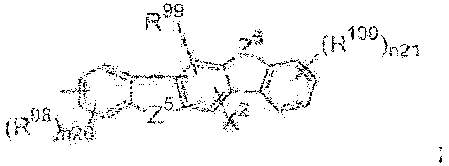
X² est un groupe de formule - (A²)ₚ-(A³)_{q}-(A⁴)ᵣ-R¹⁶, p est 0 ou 1, q est 0 ou 1, r est 0 ou 1 ;
R¹⁶ est un groupe de formule 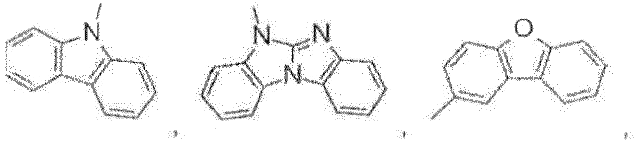 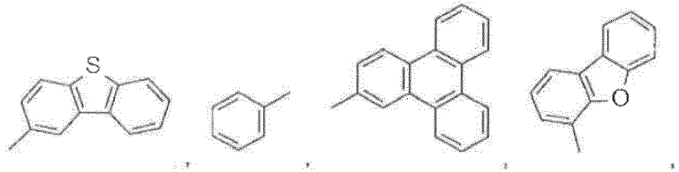 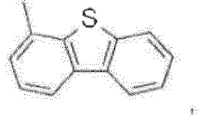 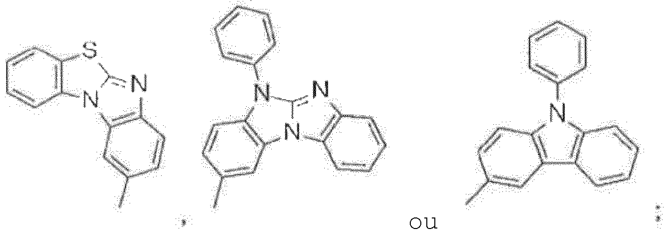
R¹⁰ et R¹¹ sont, indépendamment l'un de l'autre, un groupe aryle en C₆-C₂₄, qui peut facultativement être substitué par G ; ou un groupe hétéroaryle en C₂-C₃₀, qui peut facultativement être substitué par G ;
R¹², R¹³ et R¹⁴ sont, indépendamment les uns des autres, un groupe alkyle en C₁-C₂₅, qui peut facultativement être substitué par E et/ou interrompu par D ; un groupe aryle en C₆-C₂₄, qui peut facultativement être substitué par G ; ou un groupe hétéroaryle en C₂-C₃₀, qui peut facultativement être substitué par G ;
n7, n14 et n18 sont, indépendamment les uns des autres, 0, 1 ou 2 ;
n2, n3, n4, n9, n10, n12, n13, n15, n16, n17 et n20 sont, indépendamment les uns des autres, 0, 1, 2 ou 3, n1, n5, n6, n11, n19 et n21 sont, indépendamment les uns des autres, 0, 1, 2, 3 ou 4 ;
R²¹, R²², R²³, R²⁴, R²⁶, R²⁷, R²⁸, R⁷³, R⁷⁴, R⁷⁵, R⁷⁶, R⁷⁷, R⁷⁸, R⁷⁹, R⁹³, R⁹⁴, R⁹⁵, R⁹⁶, R⁹⁷, R⁹⁸, R⁹⁹ et R¹⁰⁰ sont, indépendamment les uns des autres, un groupe alkyle en C₁-C₂₅, qui peut facultativement être substitué par E et/ou interrompu par D ; un groupe aryle en C₆-C₂₄, qui peut facultativement être substitué par G ; ou un groupe hétéroaryle en C₂-C₃₀, qui peut facultativement être substitué par G ;
Z¹, Z², Z³, Z⁴, Z⁵ et Z⁶ sont, indépendamment les uns des autres, O, S ou NR²⁹ ;
R²⁹ et R³⁰ sont, indépendamment les uns des autres, un groupe alkyle en C₁-C₂₅, qui peut facultativement être substitué par E et/ou interrompu par D ; un groupe aryle en C₆-C₂₄, qui peut facultativement être substitué par G ; ou un groupe hétéroaryle en C₂-C₃₀, qui peut facultativement être substitué par G ;
A², A³ et A⁴ sont, indépendamment les uns des autres, un groupe arylène en C₆-C₂₄, qui peut facultativement être substitué par G, ou un groupe hétéroarylène en C₂-C₃₀, qui peut facultativement être substitué par G ;
D est -CO-, -COO-, -S-, -O-, -NR⁶⁵-, -SiR⁷⁰R⁷¹-, -POR⁷²-, -CR63=CR64- ou -C≡C-,
E
est -OR⁶⁹, -SR⁶⁹, -NR⁶⁵R⁶⁶, -COR⁶⁸, -COOR⁶⁷, -CONR⁶⁵R⁶⁶, -CN ou F,
G est E, ou un groupe alkyle en C₁-C₁₈, un groupe aryle en C₆-C₂₄, un groupe aryle en C₆-C₂₄, qui est substitué par F, alkyle en C₁-C₁₈, ou alkyle en C₁-C₁₈ qui est interrompu par O ; un groupe hétéroaryle en C₂-C₃₀, ou un groupe hétéroaryle en C₂-C₃₀, qui est substitué par F, alkyle en C₁-C₁₈, ou alkyle en C₁-C₁₈ qui est interrompu par O ;
R⁶³ et R⁶⁴ sont, indépendamment l'un de l'autre, H, aryle en C₆-C₁₈ ; aryle en C₆-C₁₈ qui est substitué par alkyle en C₁-C₁₈, ou alcoxy en C₁-C₁₈ ; alkyle en C₁-C₁₈ ; ou alkyle en C₁-C₁₈ qui est interrompu par -O- ;
R⁶⁵ et R⁶⁶ sont, indépendamment l'un de l'autre, un groupe aryle en C₆-C₁₈ ; un aryle en C₆-C₁₈ qui est substitué par alkyle en C₁-C₁₈ ou alcoxy en C₁-C₁₈ ; un groupe alkyle en C₁-C₁₈ ; ou un groupe alkyle en C₁-C₁₈, qui est interrompu par -O- ; ou
R⁶⁵ et R⁶⁶ forment conjointement un cycle de cinq ou six chaînons,
R⁶⁷ est un groupe aryle en C₆-C₁₈ ; un groupe aryle en C₆-C₁₈, qui est substitué par alkyle en C₁-C₁₈, ou alcoxy en C₁-C₁₈ ; un groupe alkyle en C₁-C₁₈ ; ou un groupe alkyle en C₁-C₁₈, qui est interrompu par -O-, R⁶⁸ est H ; un groupe aryle en C₆-C₁₈ ; un groupe aryle en C₆-C₁₈, qui est substitué par alkyle en C₁-C₁₈, ou alcoxy en C₁-C₁₈ ; un groupe alkyle en C₁-C₁₈ ; ou un groupe alkyle en C₁-C₁₈, qui est interrompu par -O-,
R⁶⁹ est un aryle en C₆-C₁₈ ; un aryle en C₆-C₁₈, qui est substitué par alkyle en C₁-C₁₈, ou alcoxy en C₁-C₁₈ ; un groupe alkyle en C₁-C₁₈ ; ou un groupe alkyle en C₁-C₁₈, qui est interrompu par -O-,
R⁷⁰ et R⁷¹ sont, indépendamment l'un de l'autre, un groupe alkyle en C₁-C₁₈, un groupe aryle en C₆-C₁₈, ou un groupe aryle en C₆-C₁₈, qui est substitué par alkyle en C₁-C₁₈, et
R⁷² est un groupe alkyle en C₁-C₁₈, un groupe aryle en C₆-C₁₈, ou un groupe aryle en C₆-C₁₈, qui est substitué par alkyle en C₁-C₁₈, à condition que
X¹ soit lié à l'atome d'azote par un atome de carbone.

2. Composé selon la revendication 1, dans lequel R⁸¹, R⁸², R⁸³ et R⁸⁴ sont H.

3. Composé selon la revendication 1 ou 2, qui est un composé de formule 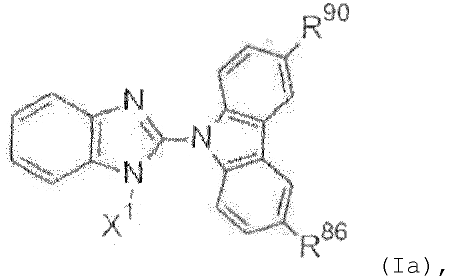 dans lequel X¹, R⁸⁶ et R⁹⁰ sont définis dans la revendication 1.

4. Composé selon la revendication 3, dans lequel R⁹⁰ est H.

5. Composé selon la revendication 3, dans lequel R⁸⁶ et R⁹⁰ sont H.

6. Composé selon l'une quelconque des revendications 1 à 5, dans lequel X¹ est un groupe de formule -A¹-X², qui est choisi parmi 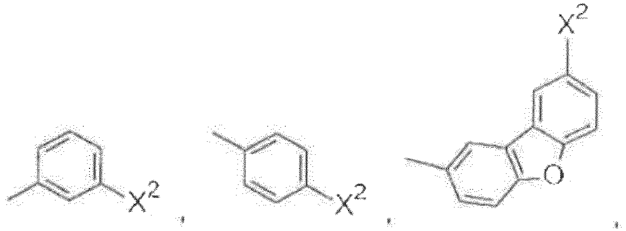 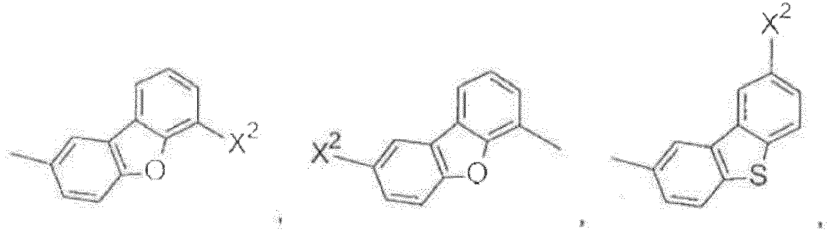 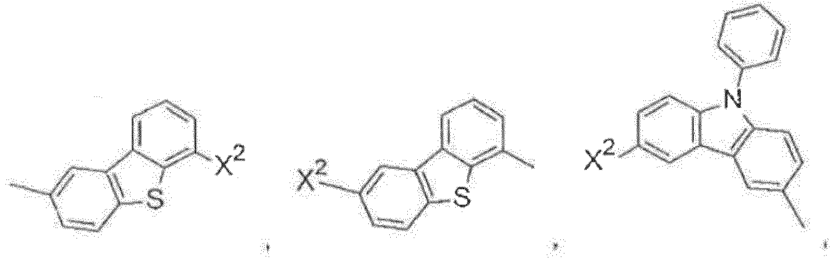 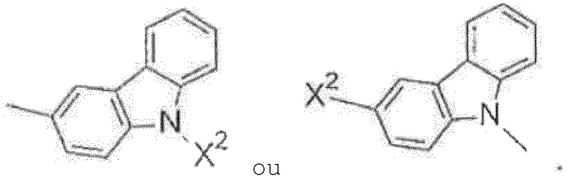 X² est un groupe de formule - (A²)ₚ-(A³)_{q}-(A⁴)ᵣ-R¹⁶, où p, q, r, R¹⁶, A², A³ et A⁴ sont définis dans la revendication 1.

7. Composé selon l'une quelconque des revendications 1, ou 6, dans lequel A², A³ et A⁴ sont, indépendamment les uns des autres, un groupe de formule 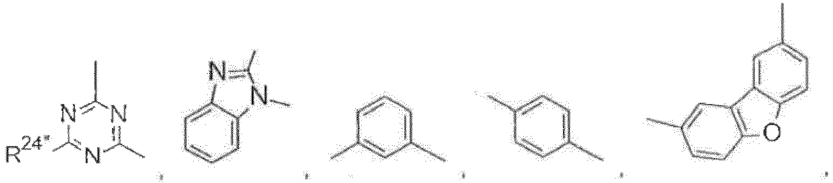 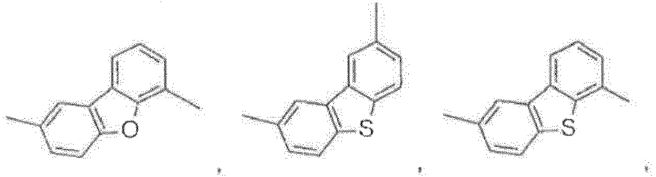 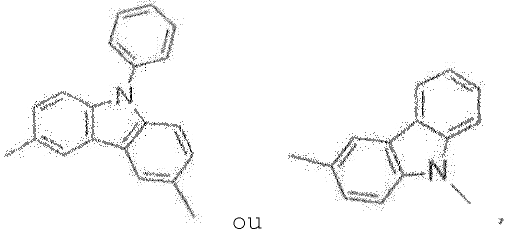 dans lequel R^{24"} est

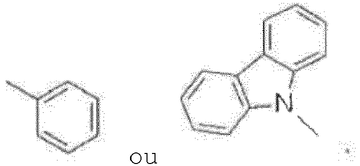

8. Composé selon l'une quelconque des revendications 1 à 7, dans lequel le groupe -A¹-(A²)ₚ-(A³)_{q}-(A⁴)ᵣ- est un groupe de formule

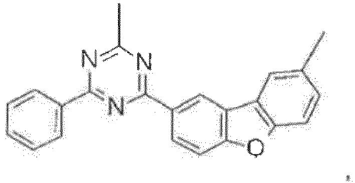

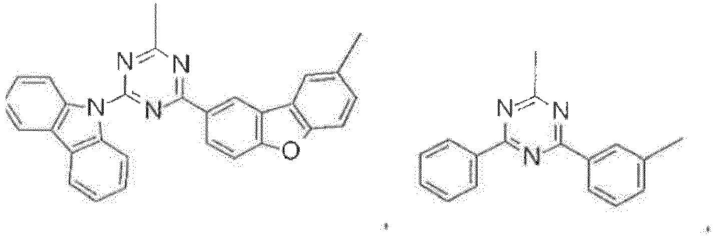

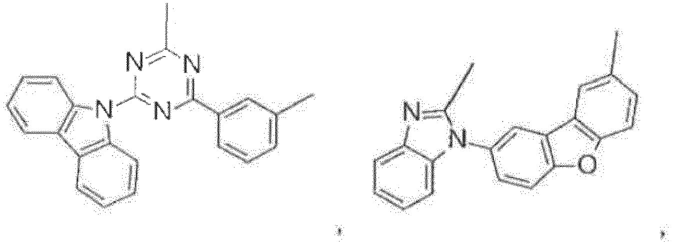

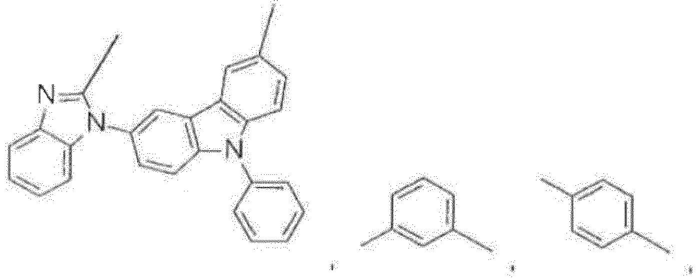

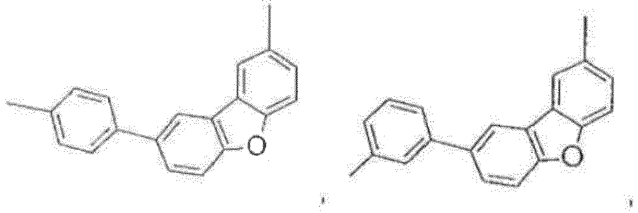

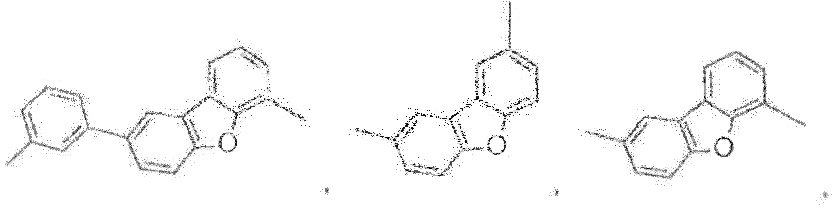

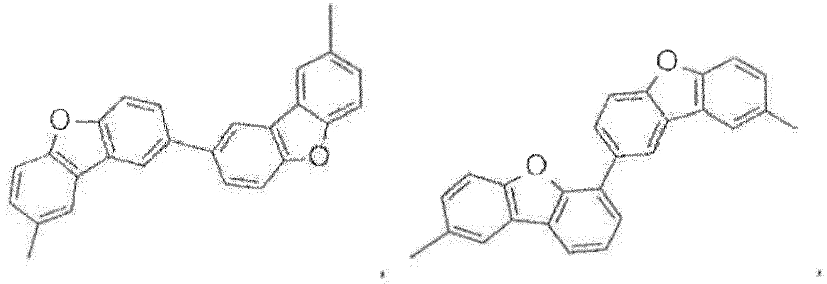

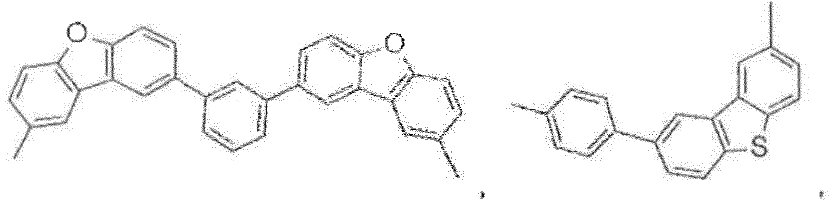

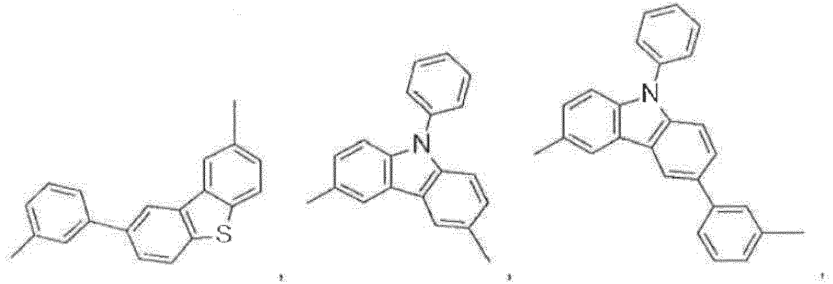

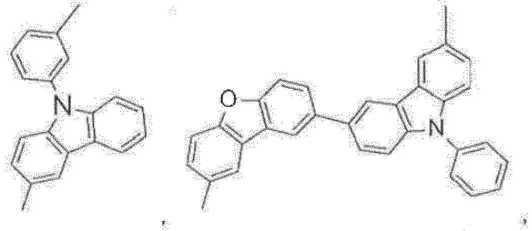

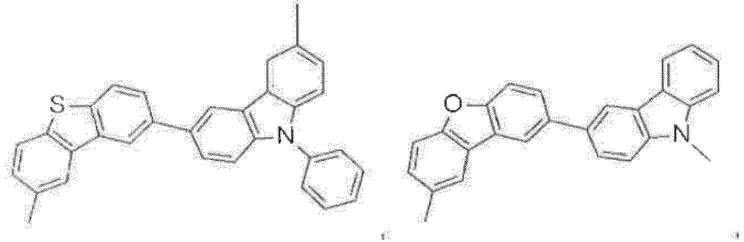

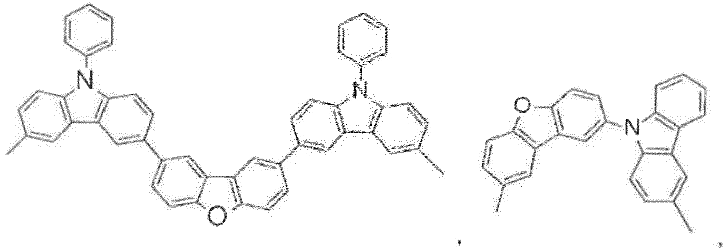

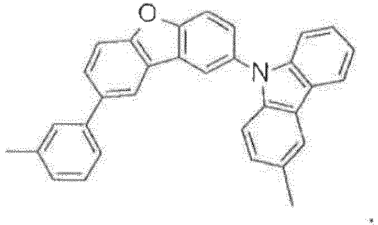

9. Composé selon l'une quelconque des revendications 1 à 8, qui est un composé de formule 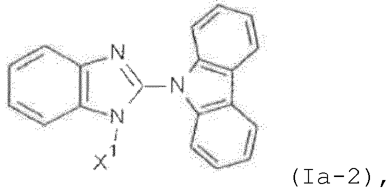 dans laquelle
X¹ est un groupe de formule -A¹-(A²)ₚ-(A³)_{q}-(A⁴)ᵣ-R¹⁶, dans laquelle
-A¹-(A²)p-(A³)_{q}-(A⁴)ᵣ est un groupe de formule 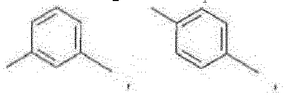 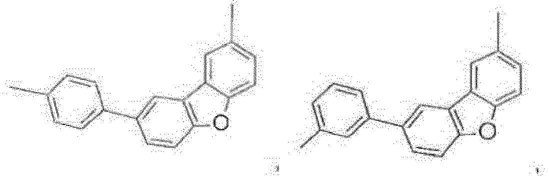 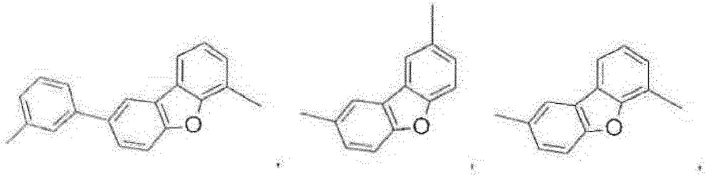 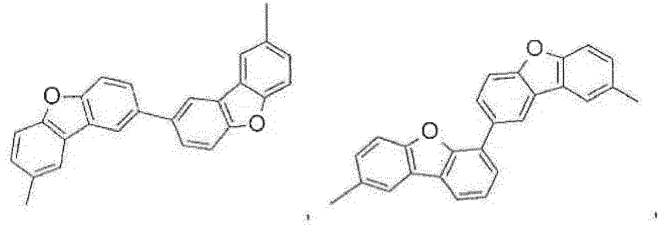 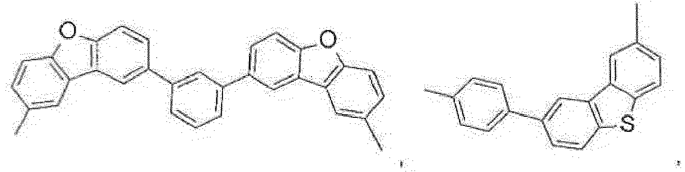 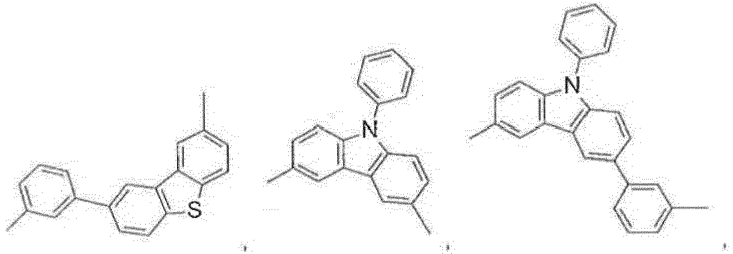 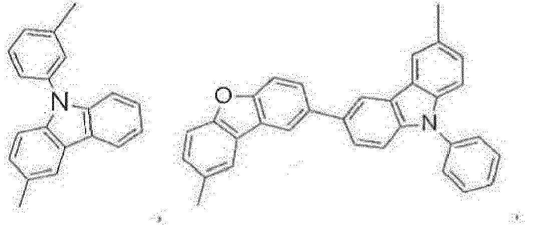 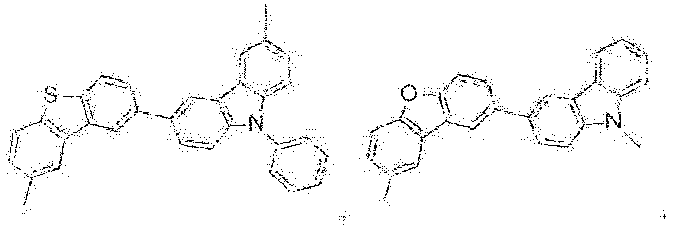 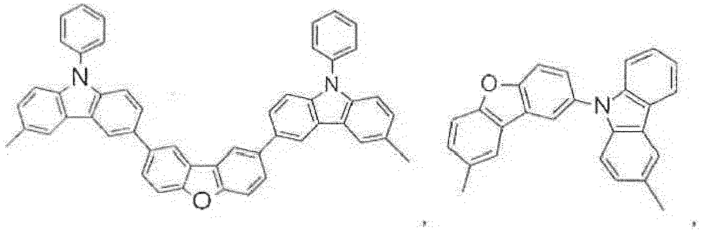 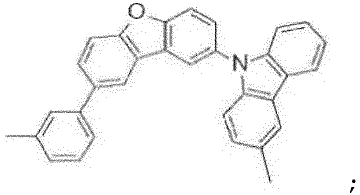 et
R¹⁶ est un groupe de formule

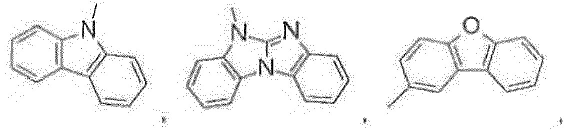

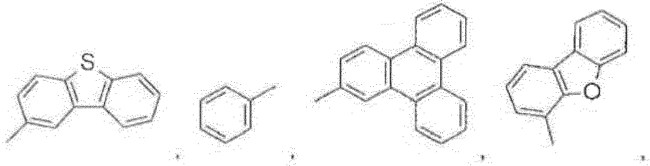

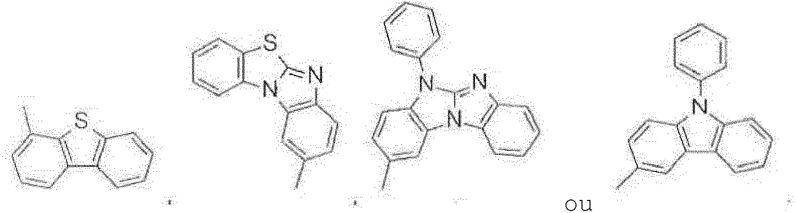

10. Dispositif électronique, comprenant un composé selon l'une quelconque des revendications 1 à 9.

11. Dispositif électronique selon la revendication 10, qui est un dispositif électroluminescent.

12. Couche de transport de charges, couche de bloqueur de charge/exciton ou couche émettrice comprenant un composé selon l'une quelconque des revendications 1 à 9.

13. Couche émettrice selon la revendication 12, comprenant un composé selon l'une quelconque des revendications 1 à 11 en tant que matériau hôte en combinaison avec un émetteur phosphorescent.

14. Appareil choisi dans le groupe constitué d'unités d'affichage visuel stationnaires ; d'unités d'affichage visuel mobiles ; unités d'éclairage ; de claviers ; d'articles d'habillement ; de mobilier ; de papier peint, comprenant le dispositif électronique selon la revendication 10 ou 11, ou la couche de transport de charge, la couche de charge/exciton, ou la couche émettrice selon la revendication 12.

15. Utilisation des composés de formule I selon l'une quelconque des revendications 1 à 9 pour des photorécepteurs électrophotographiques, des convertisseurs photoélectriques, des cellules solaires organiques, des éléments de commutation, des transistors à effet de champ émetteurs de lumière organiques, des capteurs d'image, des lasers à colorant et des dispositifs électroluminescents.
